(19) Europäisches Patentamt
European Patent Office
Office européen des brevets

(11) EP 2 196 469 A1

(12) EUROPEAN PATENT APPLICATION

(43) Date of publication:
**16.06.2010 Bulletin 2010/24**

(21) Application number: **09075144.7**

(22) Date of filing: **27.10.2005**

(51) Int Cl.:
***C07J 43/00*** (2006.01) ***C07D 413/12*** (2006.01)
***C07D 267/00*** (2006.01) ***A61K 31/58*** (2006.01)
***A61P 29/00*** (2006.01)

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**HR**

(30) Priority: **27.10.2004 US 623154 P**

(62) Document number(s) of the earlier application(s) in accordance with Art. 76 EPC:
**05824103.5 / 1 805 202**

(71) Applicant: **GlaxoSmithKline istrazivacki centar Zagreb d.o.o.**
**10000 Zagreb (HR)**

(72) Inventors:
- **Mercep, Mladen**
  **10000 Zagreb (HR)**
- **Mesic, Milan**
  **10000 Zagreb (HR)**
- **Tomaskovic, Linda**
  **10000 Zagreb (HR)**
- **Markovic, Stribor**
  **10000 Zagreb (HR)**
- **Poljak, Visnja**
  **23000 Zadar (HR)**
- **Sijan, Gordana**
  **48260 Krizevci (HR)**
- **Selmani, Selvira**
  **10000 Zagreb (HR)**

(74) Representative: **Crawley, Karen Anne et al**
**GlaxoSmithKline**
**Corporate Intellectual Property CN925.1**
**980 Great West Road**
**Brentford, Middlesex TW8 9GS (GB)**

Remarks:
- Claims 16-50 are deemed to be abandoned due to non-payment of the claims fees (Rule 45(3) EPC).
- This application was filed on 30-03-2009 as a divisional application to the application mentioned under INID code 62.

(54) **Conjugates with anti-inflammatory activity**

(57) The present invention relates to new compounds represented by Formula I:

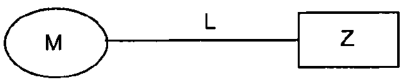

I

wherein M represents a macrolide subunit of the substructure VIII:

EP 2 196 469 A1

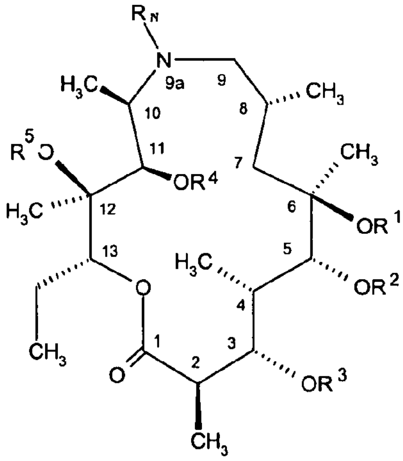

VIII

L represents the chain of the substructure IX or XIII:

$-X^1-(CH_2)_m-Q-(CH_2)_n-X^2-$ IX

$-X^1-(CH_2)_m-V-(CH_2)_p-Q-(CH_2)_n-X^1-$ XIII

Z represents the steroid or nonsteroidal subunit derived from steroid or nonsteroidal (NSAID) drugs with anti-inflammatory activity;

The present invention relates also to pharmaceutically acceptable salts and solvates of such prepared compounds, to processe and intermediates for their preparation, as well as to the improved therapeutic action and the use in the treatment of inflammatory diseases and conditions in humans and animals.

## Description

### Background of the Invention

Anti-inflammatory medicaments can be classified into those of steroid and of nonsteroidal type. Steroid anti-inflammatory compounds are still the most effective ones in the treatment of inflammatory diseases and conditions such as: asthma, inflammatory nasal diseases such as allergic rhinitis, nasal polyps, intestinal diseases such as Crohn's disease, colitis, ulcerative colitis, dermatological inflammations such as eczema, psoriasis, allergic dermatitis, neurodermatitis, pruritis, conjunctivitis and rheumatoid arthritis. In addition to excellent potency and effectiveness, medicaments of this type also possess numerous unfavourable side-effects, (e.g. disturbance of carbohydrate metabolism, decreased calcium resorption, decreased excretion of endogenous corticosteroids and disturbance of physiological functions of the pituitary gland, adrenal cortex and thymus. Steroids present on the market are highly effective against inflammatory conditions and processes whereas their systemic side-effects are diminished. Patent applications WO 94/13690; 94/14834; 92/13872 and 92/13873 describe the so-called "soft" steroids or hydrolysable corticosteroids designed for topical application at the inflammation site, whereas their systemic side-effects are diminished due to the hydrolysis in the serum, wherein the active steroid very rapidly hydrolyses into the inactive form. An ideal steroid, however, without unfavourable effects in a long-term and continuous treatment as required for the control of diseases such as asthma or Crohn's disease has yet to be found, so that there are intense efforts on the discovery and development of steroids with improved therapeutic profile.

Macrolide antibiotics accumulate preferentially within different cells of subjects, especially within phagocyte cells such as mononuclear peripheral blood cells, and peritoneal and alveolar macrophages. (Gladue, R. P. et al, Antimicrob. Agents Chemother. 1989, 33, 277-282; Olsen, K. M. et al, Antimicrob. Agents Chemother. 1996, 40, 2582-2585*).* Inflammatory effects of some macrolides have been described in the literature, although their effects are relatively weak. For example, the anti-inflammatory effect of erythromycin derivatives (J. Antimicrob. Chemother. 1998, 41, 37-46; WO Patent Application No. 00/42055) and azithromycin derivatives has been described (EP Pat. Br. 0283055). Anti-inflammatory effects of some macrolides are also known from *in vitro* and *in vivo* studies in experimental animal models such as in zymosan-induced peritonitis in mice (J. Antimicrob. Chemother. 1992, 30, 339-348) and endotoxin-induced neutrophil accumulation in rat trachea (J. Immunol. 1997, 159, 3395-4005). The modulating effect of macrolides upon cytokines such as interleukin 8 (IL-8) *(*Am. J. Respir. Crit. Care. Med. 1997, 156, 266-271) and interleukin 5 (IL-5) (EP Pat. No. 0775489 and EP Pat. No. 771564) is known as well.
International Publication No. WO 02/055531 A1, herein incorporated by reference in its entirety, discloses conjugate compounds represented by the Formula **II**:

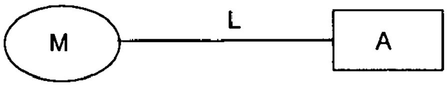


**II**

wherein **M** represents a macrolide subunit possessing the property of accumulation in inflammatory cells, **A** represents an anti-inflammatory subunit that can be steroid or nonsteroid, and **L** represents a linker molecule linking **M** and **A**, (b) their pharmacologically acceptable salts, prodrugs and solvates, (c) processes and intermediates for their preparation, and (d) their use in the treatment of inflammatory diseases and conditions in humans and animals. In WO 02/05531, the conjugate steroid-macrolide compounds are mostly linked with the steroid subunit at the N/9a-position of macrolide ring.

U.S. Published Application 2004 0014685 and International Publication No. WO 04/005310 A2, herein incorporated by reference in their entirety, relate to compounds represented by

Formula **III.**

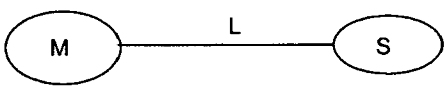


**III**

wherein M represents a macrolide subunit (macrolide moiety) derived from macrolide possessing the property of accumulation in inflammatory cells, S represents a steroid subunit derived from a steroid drug with anti-inflammatory activity and L represents a linker molecule linking M and S to their pharmaceutically acceptable salts and solvates processes and intermediates for their preparation and to their use in the treatment of inflammatory diseases and conditions in humans and animals.

US Published Application 20040077612 herein incorporated by reference in its entirety relates to new compounds represented by Formula **IV**.

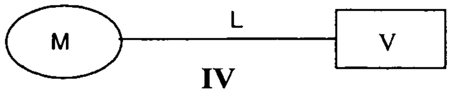

**IV**

wherein M represents a macrolide subunit (macrolide moiety) derived from macrolide possessing the property of accumulation in inflammatory cells, V represents an anti-inflammatory steroid or non steroid subunit or an anti neoplastic or antiviral subunit and L represents a linking group covalently linking M and V to their pharmaceutically acceptable salts and solvates processes and intermediates for their preparation and to their use in the treatment of inflammatory diseases and conditions in humans and animals.

US Published Application 2004 0097434 and International Publication No. WO 04/005309, each of which are herein incorporated by reference in their entirety relates to new compounds represented by formula **V.**

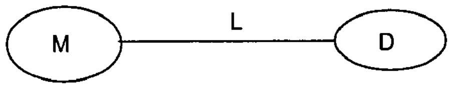

**V**

wherein M represents a macrolide subunit (macrolide moiety) derived from macrolide possessing the property of accumulation in inflammatory cells, D represents a nonsteroidal subunit (nonsteroidal moiety) derived from a nonsteroid drug with anti-inflammatory, analgesic and/or antipyretic activity (NSAID) and L represents a linking group covalent linking M and D to their pharmaceutically acceptable salts and solvates processes and intermediates for their preparation and to their use in the treatment of inflammatory diseases and conditions in humans and animals.

US Published Application 20050080003, herein incorporated by reference in its entirety, describes yet further conjugate compounds having a steroid or non-steroidal anti-inflammatory subunit **D** linked via the chain **L** to position N/9a of an aglycone type macrolide subunit.

US Published Application 20040087517 and International Publication WO2003/070174 disclose a conjugate of (i) a "transportophore" and (ii) a "non-antibiotic therapeutic agent" covalently linked by a bond or a linker incorporating the transportophore. The transportophore and conjugate must have an immune selectivity ratio of at least 2. "Transportophore" is broadly defined as a compound, a portion of which resembles and is recognized as a substrate for transport protein(s).

However, there is still need for novel anti-inflammatory conjugates of macrolides and steroids having therapeutic action.

## Summary of the Invention

The present invention relates to: a) new compounds represented by the structure **I:**

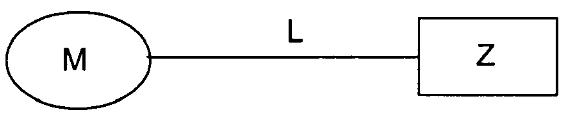

**I**

wherein **M** represents a macrolide subunit derived from macrolides, possessing the property of accumulation in inflammatory cells, **Z** represents either a steroid subunit or nonsteroidal subunit derived from nonsteroidal anti-inflammatory drugs (NSAID), and **L** represents a chain linking **M** and **Z**; b) their pharmacologically acceptable salts and solvates; c) processes and intermediates for their preparation and d) their activity and use in the treatment of inflammatory diseases and conditions in humans and animals. Specifically the macrolide subunit is an 9-deoxo-9-dihydro-9a-aza-9a-homoerithronolide A or an azithromycin aglycone subunit and the linkage to **Z** is effected via the linker **L** through the hydroxy group at position C/11 or through the nitrogen at position 9a of the aglycone ring. Also, specifically the macrolide subunit is an 9-deoxo-9-dihydro-9a-aza-9a-homoerithronolide **A** or an azithromycin aglycone subunit and **Z** is steroid subunit and the linkage to M is effected via the linker L through the 17α-hydroxy group

**Detailed Description of the Invention**

A characteristic of compounds represented by Formula **I** is selective accumulation in target organs and cells in the above mentioned inflammatory diseases and conditions. These pharmacokinetic properties enable the compounds represented by Formula I to act at the inflammation site in inflammation cells by inhibiting the production of inflammation mediators. In such a manner, the unfavourable systemic side-effects of corticosteroids or non-steroidal anti-inflammatory molecules are avoided and the therapeutic action of either the steroid or the NSAID moiety is targeted to the area where it is most needed. Following local or systemic application molecules rapidly accumulate in inflammation cells wherein they act by inhibiting the production of cytokines and chemokines and/or other inflammatory mediators thus suppressing the inflammation.

According to the known and established state of the art, compounds represented by Formula **I**, which are the object of the present invention, their pharmacologically acceptable salts, pharmaceutical compositions comprising them, and processes for making them have hitherto not been described. None of the compounds which are the object of the present invention has been described either as anti-inflammatory substance or as an inhibitor of eosinophilic accumulation in inflammation tissues.

In one aspect, the present invention relates to:

a) compounds represented by Formula **I**:

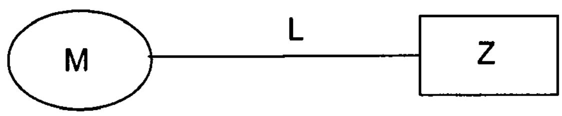


**I**

wherein **M** represents a macrolide subunit with substructure **VIII:**

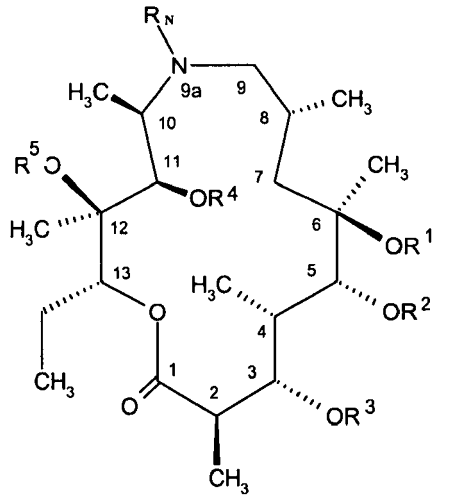

**VIII**

wherein

$R^1$, $R^2$, $R^3$, $R^4$ and $R^5$ are, independently of each other, hydrogen or groups such as $C_1$-$C_4$ alkyl (preferably methyl), alkanoyl (preferably acetyl), alkoxycarbonyl (preferably methoxycarbonyl or *tert*-butoxycarbonyl), arylmethoxycarbonyl (preferably benzyloxycarbonyl), aroyl (preferably benzoyl), arylalkyl (preferably benzyl), alkylsilyl (preferably trimethylsilyl), alkylsilylalkoxyalkyl (preferably trimethylsilylethoxymethyl) or a covalent bond with $X^1$ of chain **L** of formula **IX**;

In another aspect $R^1$, $R^2$, $R^3$, $R^4$ and $R^5$ are independantly chosen from the group consisting of $C_1$-$C_4$ alkyl and hydrogen.

In another aspect $R^1$, $R^2$, $R^3$, $R^4$ and $R^5$ are independantly chosen from the group consisting of methyl and hydrogen.

In another aspect $R^1$, $R^2$, $R^3$, $R^4$ and $R^5$ are hydrogen.

In another aspect $R^4$ is group that can combine with $R^5$ to form a "bridge" (e.g. a cyclic carbonate or carbamate) or $R^4$ is a group that can combine with $>NR_N$ to form a "bridge" (e.g. a cyclic carbamate).

In another aspect $R^4$ represents a covalent bond with $X^1$ of chain **L** of formula **IX;**

$R_N$ represents hydrogen, $C_1$-$C_4$ alkyl group or the covalent bond with $X^1$ of chain L of formula IX;

L represents a linker chain

Preferably **L** represents a linker chain with substructure **IX** or **XIII:**

$$-X^1-(CH_2)_m-Q-(CH_2)_n-X^2- \qquad \textbf{IX}$$

$$-X^1-(CH_2)_m-V-(CH_2)_p-Q-(CH_2)_n-X^2- \qquad \textbf{XIII}$$

wherein

$X^1$ is selected from: $-CH_2-$, $-CH_2-NH-$, -C(O)-, -OC(O)-, =N-O-, -C(O)NH- or -OC(O)NH-;

$X^2$ is selected from: -NH-, $-CH_2-$, -NHC(O)-, -C(=O), -OC(O)-, -C(=O)O-, or -C(O)NH-;

Q is -NH- or $-CH_2-$;

wherein each $-CH_2-$ or -NH- group are optionally substituted by $C_1$-$C_7$-alkyl, $C_2$-$C_7$-alkenyl, $C_2$-$C_7$-alkynyl, $C(O)R^x$, $C(O)OR^x$, $C(O)NHR^x$, $CH_2C(O)OR^x$, wherein $R^x$ may be $C_1$-$C_7$-alkyl, aryl or heteroaryl;

V is -NH- or -NH-C(O)-;

the symbols m, n and p are independently zero or a whole number from 1 to 12 with the proviso that if Q=NH; n cannot be zero.

This definition of the linking group is preferred not only for conjugates of nonsteroids and macrolides of Formula **VIII** but for any conjugate within Formula **I**. Other linking groups can be used as long as they provide the necessary spacer and can serve to link one subunit of the Formula **I** with the other, as is well-known in the art. For example at U.S. Patent 6,297,260, which is incorporated by reference in its entirety, at claim 1 and the specific list of NSAIDs contained therein.

**Z** represents a nonsteroidal subunit derived from nonsteroidal anti-inflammatory drugs (NSAID) or a steroid subunit preferably a steroid of substructure X:

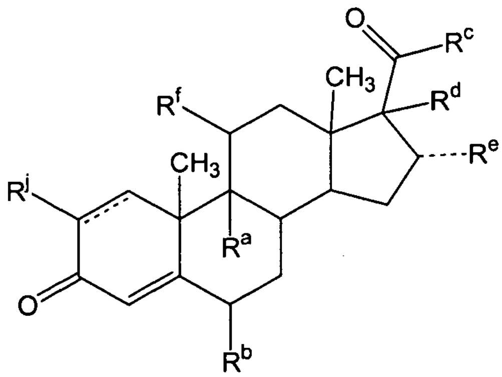

**X**

wherein

$R^a$, $R^b$, independently, are hydrogen, methyl or halogen;

$R^f$ is hydrogen, hydroxyl group or halogen (preferably chlorine) or forms a C=O (carbonyl) group with the carbon atom to which it is linked;

$R^c$ is hydroxy; $C_1$-$C_4$ alkyl (preferably methyl); $C_1$-$C_4$ alkoxy (preferably methoxy); $C_1$-$C_4$alkylhydroxy (preferably $CH_2OH$); NH-$C_1$-$C_4$ alkyl (preferably $NHCH_3$); $CH_2OC(O)C_1$-$C_4$alkyl (preferably $CH_2OC(O)CH_3$); $XC(O)N(R^1R^2)$ wherein X is S or O, $R^1$ and $R^2$ are independently $C_1$-$C_6$ alkyl or $R^1$ and $R^2$ together are $C_1$-$C_6$ alkylene; or $R^c$ is $SCH_2Y$ or $CH_2Y$ wherein Y is halogen (preferably chlorine or fluorine) or $R^c$ is the covalent link with $X^2$ of chain **L** provided that chain L is linked to $R^4$ of macrolide subunit of formula **VIII**;

$R^d$ is the covalent link with $X^2$ of chain **L**, hydrogen, hydroxy, methyl or $C_1$-$C_4$ alkoxy (preferably methoxy or n-propoxy) or together with $R^e$ and the pertaining C-atoms represent 1,3-dioxolane ring which can be additionally alkyl or alkenyl mono or di-substituted (preferably 2,2-dimethyl or 2-monopropyl or trans-propenyl ring);

$R^e$ is hydrogen, hydroxy, methyl or $C_1$-$C_4$ alkoxy (preferably methoxy or n-propoxy) or together with the $R^d$ and pertaining C-atoms represent 1,3-dioxolane ring which can be additionally alkyl or alkenyl mono or di-substituted (preferably 2,2-dimethyl or 2-monopropyl or trans-propenyl ring);

In another aspect $R^d$ is preferably the covalent link with $X^2$ of chain **L**;

$R^j$ is hydrogen or halogen (preferably chlorine).

In another aspect the present invention relates to compounds of Formula X chosen from the group consisting of

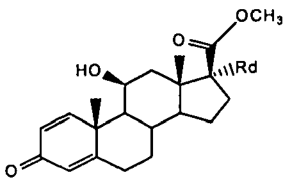
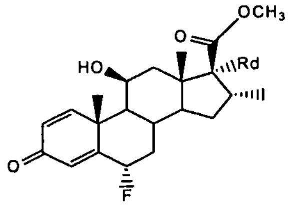
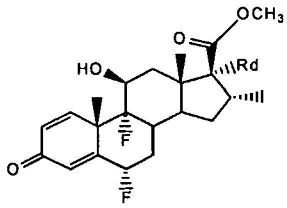
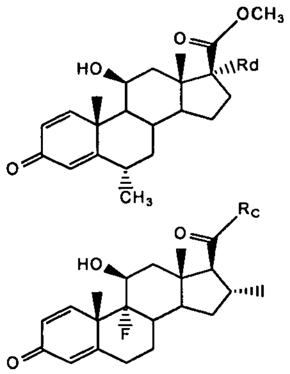
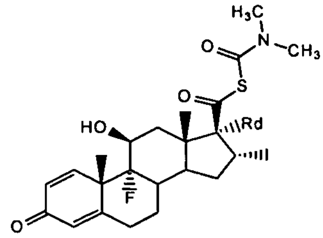
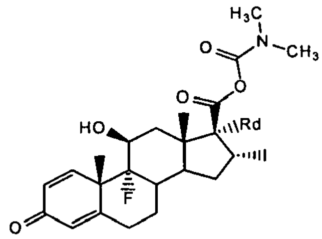
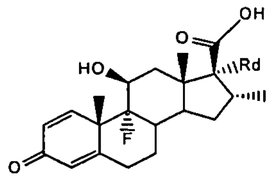
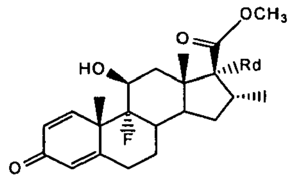
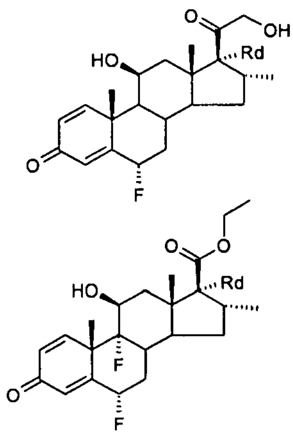
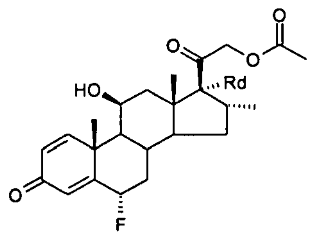

In another aspect, the present invention relates to processes for preparation of the foregoing compounds and to intermediates which may be used in such preparation.

In a third aspect, the present invention relates to combinations of one or more of the foregoing compounds in quantities sufficient for suppression of inflammatory processes; (e.g. two or more NSAID conjugates of the invention, two or more steroid conjugates of the invention, two or more compounds of the invention with at least one being an NSAID conjugate of the invention and at least one being a steroid conjugate of the invention.) These combinations offer more pronounced antiinflammatory activity if needed to treat inflammatory disease and conditions.

In yet an additional aspect, the present invention directed to methods for the use of the foregoing compounds in the treatment of disorders and conditions caused by inflammatory processes or to uses of the present compound in

the treatment of the foregoing disorders or in the manufacture of medicaments for such treatment.

In yet another aspect of the invention pharmaceutical compositions comprising a compound of the invention and pharmaceutically acceptable salts or solvates thereof including pharmaceutically acceptable diluent or carrier are contemplated. Examples include but are not limited to carboxymethylcellulose and salts thereof, polyacrylic acid and salts thereof, carboxyvinyl polymers and salts thereof, alginic acid and salts thereof, propylene glycol alginate, chitosan, hydroxypropylcellulose, hydroxypropylmethycellulose, hydroxyethylcellulose, ethylcellulose, methycellulose, polyvinyl alcohol, polyvinyl pyrolidone, N-vinylacetamide polymer, polyvinyl methacrylate, polyethylene glycol, pluronic, gelatin, methyl vinyl ether-maleic anhydride copolymer, starch, soluble starch croscaremlose, pullulan and a copolymer of methyl acrylate and 2-ethylhexyl acrylate lecithin, lecithin derivative, propylene glycol fatty acid esters, glycerin fatty acid esters, sorbitan fatty acid esters, polyoxyethylene sorbitan fatty acid esters, polyethylene glycol fatty acid esters polyoxyethylene hydrated caster oil, polyoxyethylene alkyl ethers, and pluronic. Appropriate buffer system if diluent is used is in pH range of 4 to 8, together with low molecular weight alcohols like thanol and isopropanol. The use of preservatives and masking agents is suitable.

In yet another aspect of the invention is a method of treatment of inflamatory diseases, disorders, and conditions characterized by or associated with an undesirable inflammatory immune response and all diseases and conditions induced by or associated with an excessive secretion of TNF-$\alpha$ and IL-1 which comprises administering to a subject a therapeutically effective amount of a compound of the invention.

In yet another aspect of the invention is a method of treating inflammatory conditions and immune or anaphylactic disorders associated with infiltration of leukocytes into inflamed tissues in a subject in need thereof which comprises administering to said subject a therapeutically effective amount of a compound of the invention.

In yet another aspect of the invention inflammatory conditions and immune disorders to be treated by the compounds of the invention are chosen from the group consisting of asthma, adult respiratory distress syndrome, bronchitis, cystic fibrosis, rheumatoid arthritis, rheumatoid spondylitis, osteoarthritis, gouty arthritis, uveitis, conjunctivitis, inflammatory bowel conditions, Crohn's disease, ulcerative colitis, distal proctitis, psoriasis, eczema, dermatitis, coronary infarct damage, chronic inflammation, endotoxin shock, and smooth muscle proliferation disorders.

In yet another aspect of the invention inflammatory conditions and immune disorders to be treated by the compounds of the invention are chosen from the group consisting of asthma, adult respiratory distress syndrome, chronic obstructive pulmonary disorder (COPD) inflammatory bowel conditions, Crohn's disease, bronchitis, and cystic fibrosis.

In yet another aspect of the invention is a method of treatment of inflammatory diseases, disorders and conditions characterized by or associated by excessive unregulated production of cytokines or inflamatory mediators which comprises administering to a subject a therapeutically effective amount of a compound of the invention.

Symbols **M**, **L** and **Z** represent three different subunits of compounds of Formula **I**. The symbol **M** represents the macrolide subunit, and the symbol **Z** represents the steroid or nonsteroidal subunit linked through the chain **L** with the macrolide subunit **M**.

In Formula **I**, **Z** can represent a nonsteroidal anti-inflammatory subunit, i.e., a moiety of a nonsteroidal antiinflammatory drug (NSAID). Suitable NSAIDs include, but are not limited to, those which inhibit cyclooxygenase, the enzyme responsible for the biosyntheses of the prostaglandins and certain autocoid inhibitors, including inhibitors of the various isoenzymes of cyclooxygenase (including, but not limited to, cyclooxygenase-1 and -2), and as inhibitors of both cyclooxygenase and lipoxygenase relates to nonsteroidal anti-inflammatory drug (NSAID), such as the commercially available NSAIDs aceclofenac, acemetacin, acetaminophen, acetaminosalol, acetyl-salicylic acid, acetyl-salicylic-2-amino-4-picoline-acid, 5-aminoacetylsalicylic acid, alclofenac, aminoprofen, amfenac, ampyrone, ampiroxicam, anileridine, bendazac, benoxaprofen, bermoprofen, $\alpha$-bisabolol, bromfenac, 5-bromosalicylic acid acetate, bromosaligenin, bucloxic acid, butibufen, carprofen, celecoxib, chromoglycate, cinmetacin, clindanac, clopirac, sodium diclofenac, diflunisal, ditazol, droxicam, enfenamic acid, etodolac, etofenamate, felbinac, fenbufen, fenclozic acid, fendosal, fenoprofen, fentiazac, fepradinol, flufenac, flufenamic acid, flunixin, flunoxaprofen, flurbiprofen, glutametacin, glycol salicylate, ibufenac, ibuprofen, ibuproxam, indomethacin, indoprofen, isofezolac, isoxepac, isoxicam, ketoprofen, ketorolac, lornoxicam, loxoprofen, meclofenamic acid, mefenamic acid, meloxicam, mesalamine, metiazinic acid, mofezolac, montelukast, mycophenolic acid, nabumetone, naproxen, niflumic acid, nimesulide, olsalazine, oxaceprol, oxaprozin, oxyphenbutazone, paracetamol, parsalmide, perisoxal, phenyl-acethyl-salicylate, phenylbutazone, phenylsalicylate, pyrazolac, piroxicam, pirprofen, pranoprofen, protizinic acid, reserveratol, salacetamide, salicylamide, salicylamide-O-acetyl acid, salicylsulphuric acid, salicin, salicylamide, salsalate, sulindac, suprofen, suxibutazone, tamoxifen, tenoxicam, theophylline, tiaprofenic acid, tiaramide, ticlopridine, tinoridine, tolfenamic acid, tolmetin, tropesin, xenbucin, ximoprofen, zaltoprofen, zomepirac, tomoxiprol, zafirlukast and cyclosporine. Additional NSAID genera and particular NSAID compounds are disclosed in U.S. Patent 6,297,260, incorporated entirely by reference (especially in the generic formulas of its claim 1 and the recitation of specific list of NSAID's contained therein and in claim 3, and thiazulidene NSAIDs disclosed in International Patent Application WO 01/87890, incorporated herein by reference in its entirety. Preferred are flufenamic acid, flunixin and celecoxib. In certain embodiments, the NSAID subunit is neither acetyl salicylic acid nor mycophenolic acid.

In formula **I**, **Z** may also represent **a** steroid subunit including, but not limited to, corticosteroids (such as glucocorticoids and mineralocorticoids) and androgens. Non-limiting examples of corticosteroids include cortisol, cortisone, clobetasol, hydrocortisone, fludrocortisone, fludroxycortide, flumetasone, flunisolide, fluocinolone, fluocinonide, fluocortolone, fluorometholone, prednisone, prednisolone, 6-alpha-methylprednisolone, triamcinolone, alclometasone, beclometasone, betamethasone, budesonide, dexamethasone, amcinonide, cortivazol, desonide, desoximethasone diflucortolone, difluprednate, fluclorolone and dichlorisone, fluperinidene, fluticasone, halcinonide, meprednisone, methylprednisolone, paramethasone, prednazoline, prednylidene, tixocortol, triamcinolone, and acid derivatives thereof, e.g., acetate, propionate, dipropionate, valerate, phosphate, isonicotinate, metasulfobenzoate, tebutate, and hemisuccinate).

Unless stated otherwise, the following terms have the meanings ascribed to them below.

"Halogen" means a halogen atom which may preferably be: fluorine, chlorine or bromine (the most preferably fluorine or chlorine).

"Alkyl" means a linear or branched saturated monovalent hydrocarbon radical of one to ten carbon atoms, more preferably one to six carbon atoms The preferred straight-chain or branched-chain alkyls include methyl, ethyl, propyl, iso-propyl, butyl, sec-butyl and tert-butyl. $C_1$-$C_4$ alkyl is prefered. Methyl is most preferred. Alkyl groups may be substituted with one up to five substituents including halogen (preferably fluorine or chlorine), hydroxy, alkoxy (preferably methoxy or ethoxy), acyl, acylamino cyano, amino, N-($C_1$-$C_4$)alkylamino (preferably N-methylamino or N-ethylamino), N,N-di ($C_1$-$C_4$-alkyl)amino (preferably dimethylamino or diethylamino), aryl (preferably phenyl) or heteroaryl, thiocarbonylamino, acyloxy, amino, amidino, alkylamidino, thioamidino, aminoacyl, aminocarbonylamino, aminothiocarbonylamino, aminocarbonyloxy, aryl, heteroaryl, aryloxy, aryloxyaryl, nitro, carboxyl, carboxylalkyl, carboxyl-substituted alkyl, carboxylcycloalkyl, carboxyl-substituted cycloalkyl, carboxylaryl, carboxyl-substituted aryl, carboxylheteroaryl, carboxyl-substituted heteroaryl, carboxylheterocyclic, carboxyl-substituted heterocyclic, cycloalkyl, cycloalkoxy, heteroaryloxy, heterocyclyloxy, and oxycarbonylamino. Such substituted alkyl groups are within the present definition of "alkyl." The present definition of alkyl carries over to other groups having an alkyl moiety such as alkoxy or alkanoyl.

"Alkenyl" means a linear or branched monovalent hydrocarbon radical of two to ten and preferably two to six carbon atoms which has at least one double carbon-carbon bond. Alkenyl groups may be substituted with the same groups as alkyl and such optionally substituted alkenyl groups are encompassed within the term "alkenyl". Ethenyl, propenyl, butenyl and cyclohexenyl are preferred.

"Alkynyl" means a linear or branched monovalent hydrocarbon radical, having a straight-chain or a branched-chain of two to ten, and preferably two to six carbon atoms and containing at least one and preferably no more than three triple carbon-carbon bonds. Alkynyl groups can be substituted with the same groups as alkyl, and the substituted groups are within the present definition of alkynyl. Ethynyl, propynyl and butynyl groups are preferred.

"Cycloalkyl" means a cyclic group having 3-8 carbon atoms having a single ring optionally fused to an aryl or heteroaryl group. The cycloalkyl groups can be substituted as specified for "aryl" below, and the substituted cycloalkyl groups are within the present definition of "cycloalkyl". Preferred cycloalkyls are cyclopentyl and cyclohexyl.

"Aryl" means an unsaturated aromatic carbocyclic group having 6-14 carbon atoms having a single ring such as phenyl or multiple fused rings such as naphthyl. Aryl may optionally be further fused to an aliphatic or aryl group or can be substituted with one or more substituents such as halogen (fluorine, chlorine and/or bromine), hydroxy, $C_1$-$C_7$ alkyl, $C_1$-$C_7$ alkoxy or aryloxy, $C_1$-$C_7$ alkylthio or arylthio, alkylsulfonyl, cyano or primary or nonprimary amino.

"Heteroaryl" means a monocyclic or a bicyclic aromatic hydrocarbon ring having from 2 to 10 carbon atoms and from 1 to 4 heteroatoms, such as O, S or N. The heteroaryl ring may optionally be fused to another heteroaryl, aryl or aliphatic cyclic group. Examples of this type are furan, thiophene, imidazole, indole, pyridine, oxazole, thiazole, pyrrole, pyrazole, tetrazole, pyrimidine, pyrazine and triazine, with furan, pyrrole, pyridine and indole being preferred. The term includes groups that are substituted with the same substituents as specified for aryl above.

"Heterocyclic" means a saturated or unsaturated group having a single or multiple rings and from 1 to 10 carbon atoms and from 1-4 heteroatoms selected from nitrogen, sulfur or oxygen, wherein in a fused ring system the other ring or rings can be aryl or heteroaryl. Heterocyclic groups can be substituted as specified for alkyl groups and the thus substituted heterocyclic groups are within the present definition.

When $R^c$ represents a covalentbond, the nonsteroidal or steroid subunit **Z** is linked via $R^c$ with the chain **L** to the R4 of macrolide subunit **M**.

When $R^d$ represents a covalent bond, the nonsteroidal or steroid subunit **Z** is linked via $R^d$ with the chain **L** to the macrolide subunit **M**.

When $R_N$ represents a covalent bond, the macrolide subunit **M** is linked via $R_N$ with the chain **L** to the nonsteroidal or steroid subunit **Z**.

When $R_4$ represents a covalent bond, the macrolide subunit M is linked via $R_4$ with the chain L to the nonsteroidal or steroid subunit **Z**.

In the preparation of the compounds represented by Formula **I** of the specified pharmacological activity, in the present invention certain new compounds were prepared as intermediates in the preparation of pharmacologically active compounds. The present invention also relates to such intermediates.

The term "salts" can include acid addition salts or addition salts of free bases. Examples of acids which may be employed to form pharmaceutically acceptable acid addition salts include but are not limited to salts derived from nontoxic inorganic acids such as nitric, phosphoric, sulfuric, or hydrobromic, hydroiodic, hydrofluoric, phosphorous, as well as salts derived from nontoxic organic acids such as aliphatic mono- and dicarboxylic acids, phenyl-substituted alkanoic acids, hydroxyl alkanoic acids, alkanedioic acids, aromatic acids, aliphatic and aromatic sulfonic acids, and acetic, maleic, succinic, or citric acids. Non-limiting examples of such salts include napadisylate, besylate, sulfate, pyrosulfate, bisulfate, sulfite, bisulfite, nitrate, phosphate, monohydrogenphosphate, dihydrogenphosphate, metaphosphate, pyrophosphate, chloride, bromide, iodide, acetate, trifluoroacetate, propionate, caprylate, isobutyrate, oxalate, malonate, succinate, suberate, sebacate, fumarate, maleate, mandelate, benzoate, chlorobenzoate, methylbenzoate, dinitrobenzoate, phthalate, benzenesulfonate, toluenesulfonate, phenylacetate, citrate, lactate, maleate, tartrate, methanesulfonate, and the like. Also contemplated are salts of amino acids such as arginate and the like and gluconate, galacturonate (see, for example, Berge S. M. et al. "Pharmaceutical Salts," J. of Pharma. Sci., 1977; 66:1).

The acid addition salts of said basic compounds are prepared by contacting the free base form with a sufficient amount of the desired acid to produce the salt in the conventional manner. The free base form may be regenerated by contacting the salt form with a base and isolating the free base in the conventional manner. The free base forms differ from their respective salt forms somewhat in certain physical properties such as solubility in polar solvents, but otherwise the salts are equivalent to their respective free base for purposes of the present invention.

Pharmaceutically acceptable base addition salts are formed with metals or amines, such as alkali and alkaline earth metals or organic amines. Examples of metals used as cations are sodium, potassium, magnesium, calcium, and the like. Examples of suitable amines are N,N'-dibenzylethylenediamine, chloroprocaine, choline, diethanolamine, dicyclohexylamine, ethylenediamine, N-methylglucamine, and procaine.

The base addition salts of said acidic compounds are prepared by contacting the free acid form with a sufficient amount of the desired base to produce the salt in the conventional manner. The free acid form may be regenerated by contacting the salt form with an acid and isolating the free acid in the conventional manner.

The phrase "pharmaceutically acceptable", as used in connection with compositions of the invention, refers to molecular entities and other ingredients of such compositions that are physiologically tolerable and do not typically produce untoward reactions when administered to a mammal (e.g., human). Preferably, as used herein, the term "pharmaceutically acceptable" means approved by a regulatory agency of the Federal or a state government or listed in the U.S. Pharmacopeia or other generally recognized pharmacopeia for use in mammals, and more particularly in humans.

The term "carrier" applied to pharmaceutical compositions of the invention refers to a diluent, excipient, or vehicle with which an active compound is administered. Such pharmaceutical carriers can be sterile liquids, such as water, saline solutions, aqueous dextrose solutions, aqueous glycerol solutions, and oils, including those of petroleum, , animal, vegetable or synthetic origin, such as peanut oil, soybean oil, mineral oil, sesame oil and the like. However, since memantine is highly soluble, aqueous solutions are preferred. Suitable pharmaceutical carriers are described in "Remington's Pharmaceutical Sciences" by E.W. Martin, 18th Edition. Particularly preferred for the present invention are carriers suitable for immediate-release, i.e., release of most or all of the active ingredient over a short period of time, such as 60 minutes or less, and make rapid absorption of the drug possible.

The present invention also encompasses solvates (preferably hydrates) formed by the compounds represented by Formula **I** or their salts.

The present invention also relates to all possible tautomeric forms which can be formed by individual compounds of Formula **I**.

The present invention also encompasses prodrugs of Formula **I** compounds, i.e., compounds which release an active parent drug according to Formula (**I**) in vivo when administered to a mammalian subject. Prodrugs of a compound of Formula **I** are prepared by modifying functional groups present in the compound of Formula **I** in such a way that the modifications may be cleaved in vivo to release the parent compound. Prodrugs include compounds of Formula **I** wherein a hydroxy, amino, or carboxy group of a Formula **I** compound is bonded to any group that may be cleaved in vivo to regenerate the free hydroxyl, amino or carboxy group, respectively. Examples of prodrugs include, but are not limited to esters (e.g., acetate, formate, and benzoate derivatives) of compounds of Formula **I**. The compounds of Formula **I** have one or more chirality centers and, depending on the nature of individual substituents, they can also have geometrical isomers. Isomers that differ in the arrangement of their atoms in space are termed "stereoisomers". Stereoisomers that are not mirror images of one another are termed "diastereomers" and those that are non-superimposable mirror images of each other are termed "enantiomers". When a compound has a chiral center, a pair of enantiomers is possible. An enantiomer can be characterized by the absolute configuration of its asymmetric center and is described by the R- and S-sequencing rules of Cahn and Prelog, or by the manner in which the molecule rotates the plane of polarized light and designated as dextrorotatory or levorotatory (i.e., as (+) or (-)-isomer respectively). A chiral compound can exist as either an individual enantiomer or as a mixture of enantiomers. A mixture containing equal proportions of the enantiomers is called a "racemic mixture". The present invention encompasses all individual isomers of compounds of Formula I. The description or naming of a particular compound in the specification and claims is intended to include both individual

enantiomers and mixtures, racemic or otherwise, thereof. Methods for the determination of stereochemistry and the separation of stereoisomers are well-known in the art.

A "pharmaceutically acceptable excipient" means an excipient that is useful in preparing a pharmaceutical composition that is generally safe, non-toxic and neither biologically nor otherwise undesirable, and includes an excipient that is acceptable for veterinary use as well as human pharmaceutical use. A "pharmaceutically acceptable excipient" as used in the present application includes both one and more than one such excipient.

"Treating" or "treatment" of a state, disorder or condition includes:

(1) preventing or delaying the appearance of clinical symptoms of the state, disorder or condition developing in a mammal that may be afflicted with or predisposed to the state, disorder or condition but does not yet experience or display clinical or subclinical symptoms of the state, disorder or condition,
(2) inhibiting the state, disorder or condition, i.e., arresting or reducing the development of the disease or at least one clinical or subclinical symptom thereof, or
(3) relieving the disease, i.e., causing regression of the state, disorder or condition or at least one of its clinical or subclinical symptoms.

The benefit to a subject to be treated is either statically significant or at least perceptible to the patient or to the physician
A "therapeutically effective amount" means the amount of a compound that, when administered to a mammal for treating a state, disorder or condition, is sufficient to effect such treatment. The "therapeutically effective amount" will vary depending on the compound, the disease and its severity and the age, weight, physical condition and responsiveness of the mammal to be treated.

The four classic symptoms of acute inflammation are redness, elevated temperature.

Swelling, and pain in the affected area, and loss of function of the affected organ. Symptoms and signs of inflammation associated with specific conditions include:

- rheumatoid arthritis- pain, swelling, warmth and tenderness of the involved joints; generalized and morning stiffness;
- insulin-dependent diabetes mellitus- insulitis; this condition can lead to a variety of complications with an inflammatory component, including: retinopathy, neuropathy, nephropathy; coronary artery disease, peripheral vascular disease, and cerebrovascular disease;
- autoimmune thyroiditis- weakness, constipation, shortness of breath, puffiness of the face, hands and feet, peripheral edema, bradycardia;
- multiple sclerosis- spasticity, blurry vision, vertigo, limb weakness, paresthesias;
- uveoretinitis- decreased night vision, loss of peripheral vision;
- lupus erythematosus- joint pain, rash, photosensitivity, fever, muscle pain, puffiness of the hands and feet, abnormal urinalysis (hematuria, cylinduria, proteinuria), glomerulonephritis, cognitive dysfunction, vessel thrombosis, pericarditis;
- scleroderma- Raynaud's disease; swelling of the hands, arms, legs and face; skin thickening; pain, swelling and stiffness of the fingers and knees, gastrointestinal dysfunction, restrictive lung disease; pericarditis,; renal failure;
- other arthritic conditions having an inflammatory component such as rheumatoid spondylitis, osteoarthritis, septic arthritis and polyarthritis- fever, pain, swelling, tenderness;
- other inflammatory brain disorders, such as meningitis, Alzheimer's disease, AIDS dementia encephalitis- photophobia, cognitive dysfunction, memory loss;
- other inflammatory eye inflammations, such as retinitis- decreased visual acuity;
- inflammatory skin disorders, such as , eczema, other dermatites (e.g., atopic, contact), psoriasis, bums induced by UV radiation (sun rays and similar UV sources)-erythema, pain, scaling, swelling, tenderness;
- inflammatory bowel disease, such as Crohn's disease, ulcerative colitis- pain, diarrhea, constipation, rectal bleeding, fever, arthritis;
- asthma- shortness of breath, wheezing;
- other allergy disorders, such as allergic rhinitis- sneezing, itching, runny nose
- conditions associated with acute trauma such as cerebral injury following stroke-sensory loss, motor loss, cognitive loss;
- heart tissue injury due to myocardial ischemia- pain, shortness of breath;
- lung injury such as that which occurs in adult respiratory distress syndrome- shortness of breath, hyperventilation, decreased oxygenation, pulmonary infiltrates;
- inflammation accompanying infection, such as sepsis, septic shock, toxic shock syndrome- fever, respiratory failure, tachycardia, hypotension, leukocytosis;
- other inflammatory conditions associated with particular organs or tissues, such as nephritis (e.g., glomerulonephritis)-oliguria, abnormal urinalysis;

inflamed appendix- fever, pain, tenderness, leukocytosis;
gout- pain, tenderness, swelling and erythema of the involved joint, elevated serum and/or urinary uric acid;
inflamed gall bladder- abdominal pain and tenderness, fever, nausea, leukocytosis;
chronic obstructive pulmonary disorder (COPD), shortness of breath, wheezing;
congestive heart failure- shortness of breath, rales, peripheral edema;
Type II diabetes- end organ complications including cardiovascular, ocular, renal, and peripheral vascular disease
lung fibrosis- hyperventilation, shortness of breath, decreased oxygenation;
vascular disease, such as atherosclerosis and restenosis- pain, loss of sensation, diminished pulses, loss of function and alloimmunity leading to transplant rejection-pain, tenderness, fever.

Subclinical symptoms include without limitation diagnostic markers for inflammation the appearance of which may precede the manifestation of clinical symptoms. One class of subclinical symptoms is immunological symptoms, such as the invasion or accumulation in an organ or tissue of proinflammatory lymphoid cells or the presence locally or peripherally of activated pro-inflammatory lymphoid cells recognizing a pathogen or an antigen specific to the organ or tissue. Activation of lymphoid cells can be measured by techniques known in the art.
"Delivering" a therapeutically effective amount of an active ingredient to a particular location within a host means causing a therapeutically effective blood concentration of the active ingredient at the particular location. This can be accomplished, e.g., by local or by systemic administration of the active ingredient to the host.
The term host or subject in need thereof as used herein refers to a mammal preferably a human.
The term leaving group refers to a chemical group which is capable of being displaced by a nucleophile. Examples of such groups include but are not limited to halogen, mesylate, tosylate and ester groups.

**Methods of Preparation**

A further aspect of the present invention relates to a method for the preparation of compounds within Formula **I** comprising:

a) for the compounds of Formula **I,** wherein $X^2$ is -NH-
a reaction of the steroid or nonsteroidal subunit of the substructure **V**:

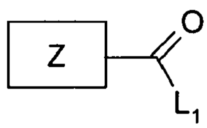

**XI**

(wherein $L_1$ represents a leaving group such as hydroxy)
and the amino group of the macrolide subunit of the substructure **VIa:**

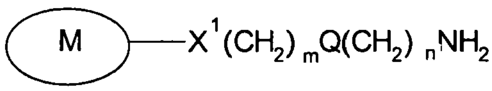

**XII**

Steroid or nonsteroidal subunits of the substructure **XI** are either commercially available products or have been obtained, like the starting macrolide subunits of the substructure **XII** by methods for preparation of analogous compounds described in our earlier patent applications (HR Patent Application No. 20010018; WO Patent Application No. 02/055531); WO 04/005309; WO 04/005310 herein incorporated by reference in their entireties.
The reaction is generally performed with acid derivatives which have the ability to activate the carboxylic acid group of steroidal anti-inflammatory subunit, such as halogenides, mixed anhydrides and especially carbodiimides (such as -(3-dimethylaminopropyl)-3-ethyl-carbodiimide (EDC)) and benzotriazoles. The reaction proceeds in the presence

of a base, such as an organic base (e.g., triethylamine), at room temperature under an inert atmosphere such as nitrogen or argon. The reaction may require several hours to several days to come to completion.

b) Compounds represented by Formula **I**, where $X^1$ is -C(O)NH-, Q is $-CH_2-$ or -NH-and $X^2$ is -NH- or NHC(O)-, can be prepared by reacting a macrolide subunit and a derivatized steroid or nonsteroidal subunit having a free amino group as shown below.

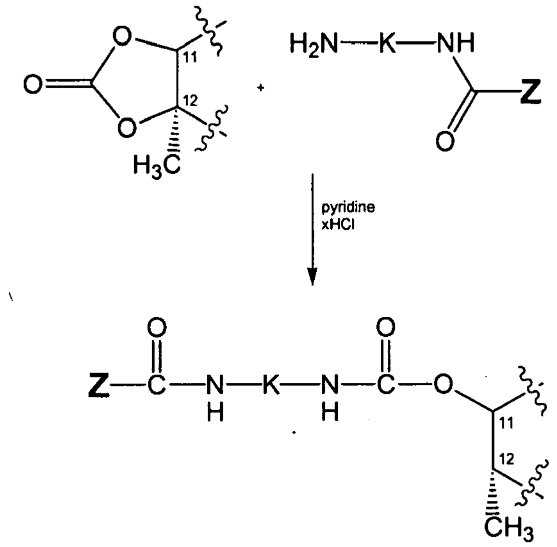


c) Compounds represented by Formula **I**, where $X^1$ is -C(O)NH-, Q is $-CH_2-$ or -NH- and $X^2$ is -NH- or -NHC(O)-, can be prepared by reacting a macrolide subunit and a steroid or nonsteroidal subunit having a free carboxylic acid group as shown below.

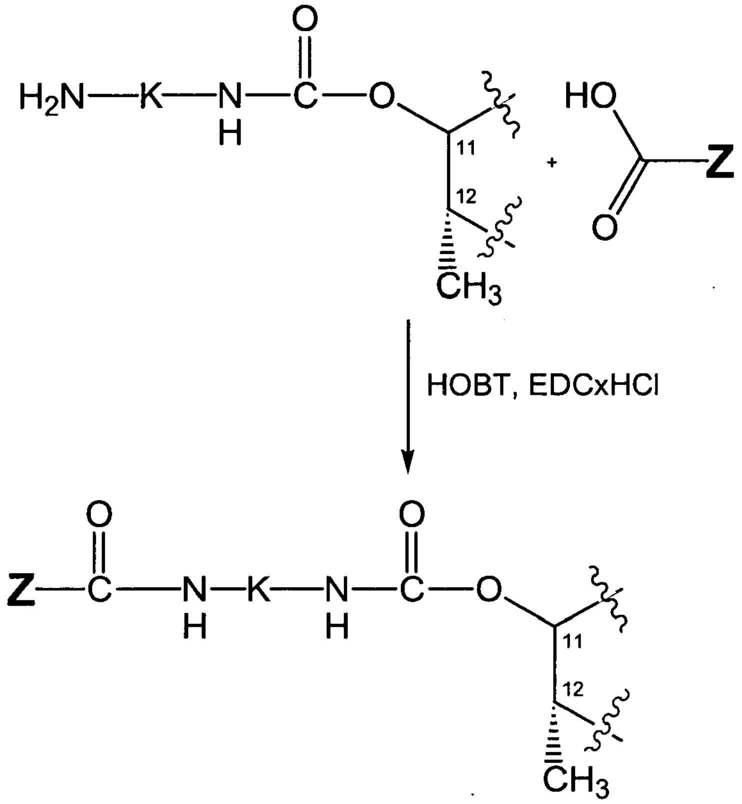


The steroid subunit may be linked to the macrolide through the 21 hydroxy group in steroids that have such a group. Begining with a 21-hydroxy steroid cyclic ketal is reacted with an appropriate carboxylic acid halide or an anhydride, preferably in a solvent such as methylene chloride in the presence of a tertiary amine base or pyridine at a reduced temperature (-50° C-100° C). The intermediate so produced is reacted with $H_2N$-L-M to form compounds of Formula **I**

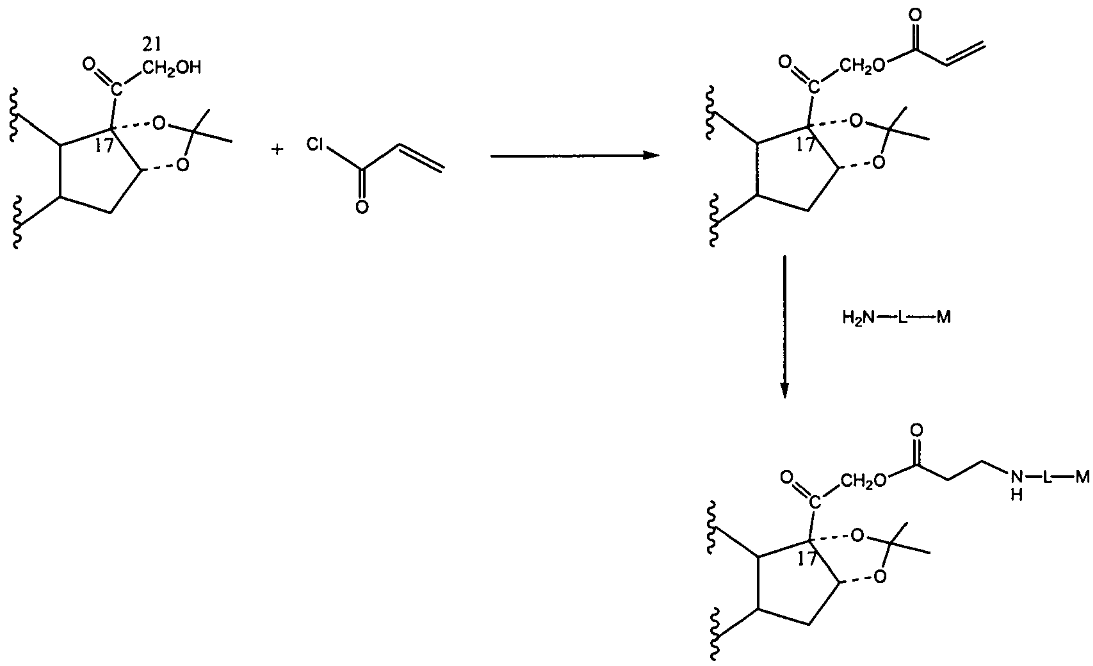

The steroid subunit may also be linked to the macrolide through the 17 position on the steroid subunit. One method for preparing such a compound is as follows:

As illustrated by the synthetic schemes above and below there are two synthetic pathway alternatives: one derivatives the carboxylic acid group at $C_{17}$ to form C(O)-$R^c$ prior to coupling with the linker-macrolide partion, and the other derivatives the same carboxylic group only subsequesnt to such coupling.

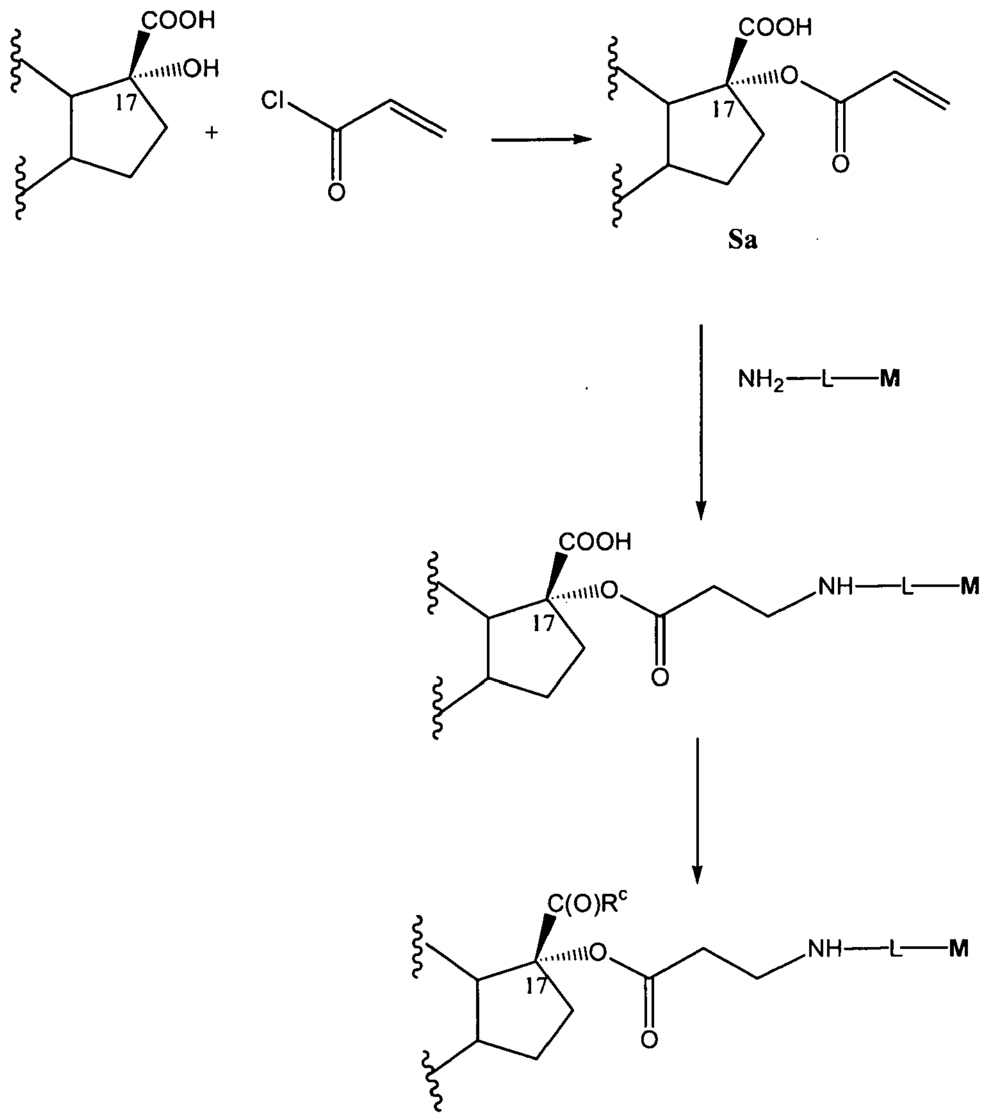


For example, when L is -K-NH- (wherein K is the portion of the L molecule attached to the macrolide) the compound of Formula **I** can be formed by derivatizing an NH group on the macrolide ring to a terminal -N-K-$NH_2$ group and reacting the derivatized macrolide with a steroid anti-inflammatory subunit represented by Formula **Sa**:

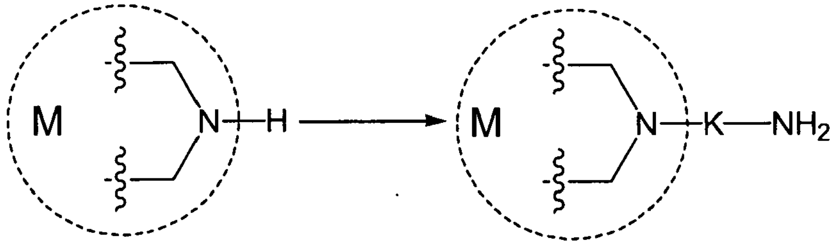

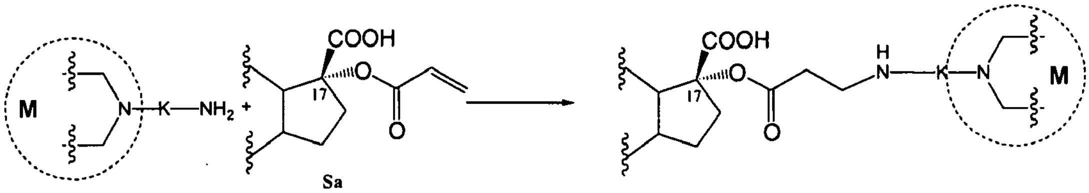

Sa

Compounds represented by Formula **I**, where $X^2$ is -NH-, can be prepared by reacting a macrolide subunit and a steroid subunit having a -C=C- bond as shown below.

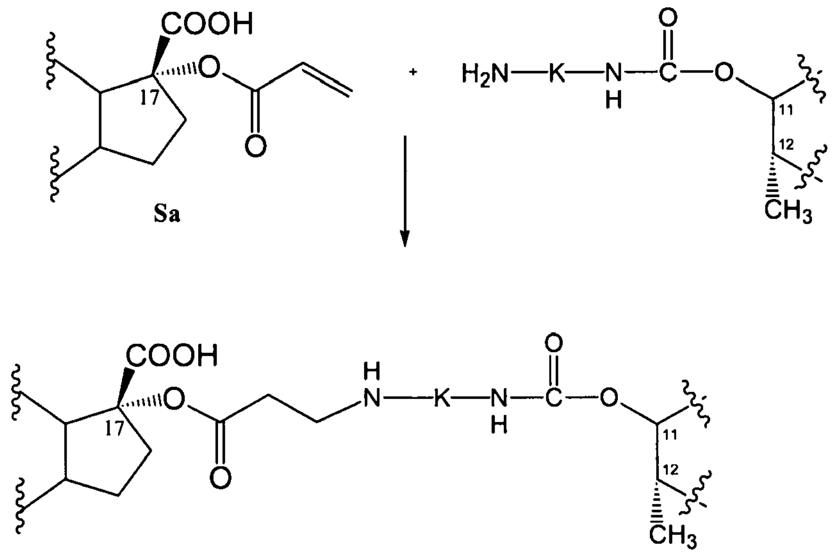

The carboxylic acid group at the 17 position of the starting steroid subunit may be modified prior to the reaction with $NH_2$-L-**M**.

The carboxylic acid group at the 17 position of the starting steroid subunit can also be protected prior to the reaction with $NH_2$-L-**M** and deprotected after the reaction with $NH_2$-L-**M** or the esterification step.

The non-steroidal anti-inflammatory subunit D may contain a -C(O)$L^1$ group (such as a free carboxylic acid group) or be derivatized by methods known in the art.

Scheme I

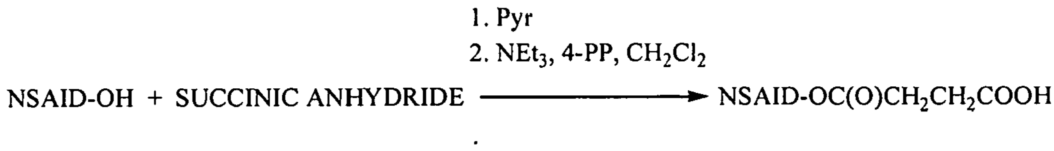


According to Scheme I, NSAID compounds having a hydroxyl group may alternatively be derivatized by the action of succinic anhydride in the presence of pyridine followed by reaction of the intermediate so produced with triethylamine, 4-pyrrolopyridine in methylene chloride to produce NSAID having free carboxylic acid group (Huang C.M. et al. Chem.&Biol. 2000,7,453-461, Hess S. et al. Bioorg.&Med. Chem. 2001, 9, 1279-1291) The NSAID derivatives so produced may be coupled to a linker macrolide compound such as formula VIa.

Scheme II

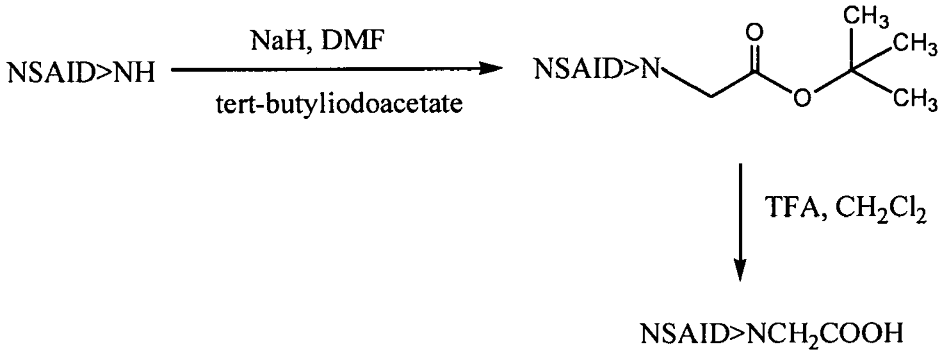


According to Scheme II, NSAID compounds having an amino group may alternatively be derivatized by the action of sodium hydride and tert-butyliodoacetate in N,N-dimethylformamide to produce a (butoxy carbonyl derivative of the NSAID which is then reacted with (trifluoracetic acid in methylene chloride to produce NSAID having free carboxylic acid group (Hess S. et al. Bioorg.&Med. Chem. 2001, 9, 1279-1291). The NSAID derivatives so produced may be coupled to a linker macrolide compound such as formula VIa.

Scheme III

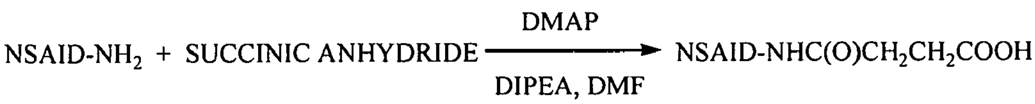


Alternatively by NSAID compounds having an amino group may be derivatized according to Scheme III by the action of succinic anhydride in the presence of dimethylaminopyridine, N,N'-diisopropylethylamine in dimethylformamide to produce NSAID having free carboxylic acid group (Pandori M. W. et al. Chem.&Biol. 2002, 9, 567-573). The NSAID derivatives so produced may be coupled to a linker macrolide compound such as formula VIa.

Compounds of Formula **I** can generally be obtained so that: one end of the chain **L** is first linked to the macrolide subunit M, and then the other end of the chain is linked to the nonsteroidal or steroid subunit **D**; or one a of the chain **L** is first linked to the nonsteroidal or steroid subunit **D** and then the other end of the chain is linked to the macrolide subunit **M**, and, finally, one end of the yet unformed chain is linked to the macrolide subunit **M**, and the other end of the also unformed chain is linked to the nonsteroidal or steroid subunit **D**, and subsequently the ends are chemically linked to form the chain **L**. To prevent undesirable side-reactions, it is frequently necessary to protect certain groups such as e.g. a hydroxy or

amino group. **A** comprehensive discussion of the ways in which such groups may be protected and methods for cleaving the resulting protected derivatives is given by for example T.W. Greene and P.G.M Wuts in Protective Groups in Organic Synthesis 2nd ed., John Wiley & Son, Inc 1991 and by P.J. Kocienski in Protecting Groups, Georg Thieme Verlag 1994 which are incorporated herein by reference. Examples of suitable amino protecting groups include acyl type protecting groups (e.g. formyl, trifluoroacetyl and acetyl), aromatic urethane type protecting groups (e.g. benzyloxycarbonyl (Cbz) and substituted Cbz, and 9-fluorenylmethoxycarbonyl (Fmoc)), aliphatic urethane protecting groups (e.g. t-butyloxycarbonyl (Boc), isopropyloxycarbonyl and cyclohexyloxycarbonyl) and alkyl type protecting groups (e.g. benzyl, trityl and chlorotrityl). Examples of suitable oxygen protecting groups may include for example alkyl silyl groups, such as trimethylsilyl or tertbutyldimethylsilyl; alkyl ethers such as tetrahydropyranyl or tert-butyl; or esters such as acetate. Hydroxy groups may be protected by reaction of for example acetic anhydride, benzoic anhydride or a trialkylsilyl chloride in an aprotic solvent. Examples of aprotic solvents are dichloromethane, N,N-dimethylformamide, dimethylsulfoxide, tetrahydrofuran and the like.
For example, one possibility for the protection of the amino group is t-butyloxycarbonyl (Boc). Deprotection using trifluoroacetic acid (TFA) is described in the examples.
Corresponding protection for amino and alkylamino groups are groups such as alkanoyl (acetyl), alkoxycarbonyl (methoxycarbonyl, etoxycarbonyl or *tert*-butoxycarbonyl), arylmethoxycarbonyl (benzyloxycarbonyl), aroyl (benzoyl) and alkylsilyl group (trimethylsilyl or trimethylsilyletoxymethyl). The conditions for elimination of the protective group depend on the selection and properties of that group. Thus, for example, acyl groups such as alkanoyl, alkoxycarbonyl and aroyl group can be removed by hydrolysis in the presence of a base (sodium or potassium hydroxide), tert-butoxycarbonyl or alkylsilyl (trimethylsilyl) group can be removed with a corresponding acid (for example, hydrochloric, sulphuric, phosphoric or trifluoroacetic acid), while arylmethoxycarbonyl group (benzyloxycarbonyl) can be removed by hydrogenolysis in the presence of a catalyst such as palladium-on-charcoal.
A further aspect of the present invention relates to the methods for using the compounds of Formula **I** as anti-inflammatory, anti-anaphylactic and immunomodulating agents which can be administered in different ways, depending on the inflammation site, e.g. percutaneously, orally, buccally, rectally, parenterally or by inhalation when application within the respiratory tract is intended.

A further aspect of the present invention relates to the methods for using the compounds of Formula **I** as anti-inflammatory, anti-anaphylactic and immunomodulating agents which can be administered in different ways, depending on the inflammation site. Further, the present invention relates to pharmaceutical compositions containing an effective dose of compounds of the present invention as well as pharmaceutically acceptable excipients, such as carriers or diluents.

The preparation of the pharmaceutical compositions of the invention can include mixing, granulating, tabletting and dissolving the ingredients. Chemical carriers can be in solid or liquid form. Solid carriers can be lactose, sucrose, talc, gelatine, agar, pectin, magnesium stearate, fatty acids without limitation. Liquid carriers can be syrups, oils such as olive, sunflower seed or soybean oils, water, or physiologic saline without limitation. Similarly, carriers may also contain a component for a sustained release of the active component such as glyceryl monostearate or glyceryl distearate. Several forms of pharmaceutical compositions can be prepared. If a solid carrier is used, these forms can include tablets, caplets, solid gelatinous capsules, powders or granules without limitation that can be administered orally. The amount of the solid carrier can vary but mainly it is in the range from 25 mg to 1 g. If a liquid carrier is used, the formulation can be in the form of a syrup, emulsion, soft gelatinous capsules, or sterile injectable liquids, or nonaqueous liquid suspensions topically or systemically, e.g., orally, parenterally, percutaneously, mucosally, e.g., buccally, intranasally, intrarectally and intravaginally. "Parenterally" means by intravenous, intramuscular or subcutaneous route.

The corresponding preparations of the compounds of the present invention can be used in the prophylaxis as well as in the therapeutic treatment (prevention, delay, inhibition or relief) of several disorders (diseases and other pathological inflammatory conditions) caused by or associated with an abnormal or undesirable (excessive, nonregulated, or dysregulated) inflammatory immune response involving the production of inflammatory cytokines or other inflammation mediators, including without limitation TNF-$\alpha$ and IL-1$\beta$. These disorders include autoimmune diseases such as rheumatoid arthritis, insulin-dependent diabetes mellitus, autoimmune thyroiditis, multiple sclerosis, uveoretinitis, lupus erythematosus, scleroderma; other arthritic conditions having an inflammatory component such as rheumatoid spondylitis, osteoarthritis, septic arthritis and polyarthritis; other inflammatory brain disorders, such as meningitis, Alzheimer's disease, AIDS dementia encephalitis, other inflammatory eye inflammations, such as retinitis; inflammatory skin disorders, such as , eczema, other dermatites (e.g., atopic, contact), psoriasis, burns induced by UV radiation (sun rays and similar UV sources); inflammatory bowel disease, such as Crohn's disease, ulcerative colitis; asthma; other allergy disorders, such as allergic rhinitis; conditions associated with acute trauma such as cerebral injury following stroke, heart tissue injury due to myocardial ischemia, lung injury such as that which occurs in adult respiratory distress syndrome; inflammation accompanying infection, such as sepsis, septic shock, toxic shock syndrome, other inflammatory conditions associated with particular organs or tissues , such as nephritis (e.g., glomerulonephritis), inflamed appendix, gout,

inflamed gall bladder, congestive heart failure, Type II diabetes, lung fibrosis, vascular disease, such as atherosclerosis and restenosis; and alloimmunity leading to transplant rejection. The compounds can also be administered by inhalation when application within the respiratory tract is intended. A further object of the present invention relates to the preparation of various pharmaceutical forms of the compounds to achieve the optimal bioavailability of the active compound of Formula **I**.

For percutaneous or mucosal external administration, the compound of Formula **I** can be prepared in a form of an ointment or cream, gel or lotion. Ointments, creams and gels can be formulated using a water or oil base with addition of an appropriate emulsifier or gelling agent Formulation of the present compounds is especially significant for respiratory inhalation, wherein the compound of Formula **I** is to be delivered in the form of an aerosol under pressure. It is preferred to micronize the compound of Formula **I** after it has been homogenised, e.g., in lactose, glucose, higher fatty acids, sodium salt of dioctylsulfosuccinic acid or, most preferably, in carboxymethyl cellulose, in order to achieve a microparticle size of 5 μm or less for the majority of particles. For the inhalation formulation, the aerosol can be mixed with a gas or a liquid propellant for dispensing the active substance. An inhaler or atomizer or nebulizer may be used. Such devices are known. See, e.g., Newman et al., Thorax, 1985, 40:61-676 Berenberg, M., J. Asthma USA, 1985, 22: 87-92. A Bird nebulizer can also be used. See also U.S. Patents 6,402,733; 6,273,086; and 6,228,346.

The compound of the structure **I** for inhalation is preferably formatted in the form of a dry powder with micronized particles, as described herein.

The compound can also be incorporated into a formulation for treating inflammation localized in an organ or tissue, e.g., Crohn's disease, where it can be administered orally or rectally. Formulations for oral administration can incorporate excipients enabling bioavailability of the compound at the site of inflammation. This can be achieved by different combinations of enteric and delayed release formulations. The compound of Formula **I** can also be used in the treatment of Crohn's disease and intestinal inflammation disease if the compound is applied in the form of a clyster, for which a suitable formulation can be used, as is well known in the field.

A therapeutically effective amount of the compound of the present invention can be determined by methods known in the art. Since the compound of the present invention is more efficiently delivered to the desired site than the corresponding anti-inflammatory steroid or NSAID drug alone, a lesser amount of the compound on a molar basis than of the steroid or NSAID anti-inflammatory drug can be administered while still achieving the same therapeutic effect. Furthermore, since administration of the compound results in fewer side effects than with the corresponding steroid or NSAID anti-inflammatory drug, the steroid or NSAID amount can be increased. Thus, the table below serves only as a guide. A threshold therapeutically effective amount of the compound, a pharmaceutically salt thereof, a solvate thereof, or a prodrug thereof is generally equal to or less than a therapeutically effective amount of the nonsteroidal anti-inflammatory drug on a molar basis. Broad and preferred effective amounts of the compound, a pharmaceutically salt thereof, a solvate thereof, or a prodrug thereof are shown in the table below.

| | Amount of Compound, Pharmaceutically Acceptable Salt Thereof, Solvate Thereof, or Prodrug Thereof | |
|---|---|---|
| | mg/kg body weight/day of the steroid or NSAID (had it been administered alone) | μmol/kg body weight/day of the hybrid or the steroid or NSAID |
| Broad | from about 0.001 to about 1000 | from about 0.004 to about 4000 |
| Preferred | from about 0.01 to about 100 | from about 0.04 to about 400 |
| More Preferred | from about 1 to about 100 | from about 4 to about 400 |
| Most Preferred | from about 3 to about 30 | from about 12 to about 120 |

For example, if the preferred amount range for prednisone is 1-50 mg/day, this corresponds to a range of 2.79 μmol to 139.5 μmol per day. The starting amount range for a hybrid steroid-macrolide conjugate according to the invention will be also 2.79 μmol to 139.5 μmol of conjugate per day. This dosage can be fine-tuned in light of the present specification using the ordinary skill in the art.

The efficacy of the present compounds can be assessed by any method for assessing inflammation or anti-inflammatory effect. There are many known methods for this purpose including without limitation use of contrast ultrasound in conjunction with injection of microbubbles, measurement of inflammatory cytokines (such as TNF-α, IL-1, IFN-γ) measurement of activated immune system cells (activated T cells, cytotoxic T cells specifically recognizing the inflamed or transplanted tissue) as well as by observation (reduction of oedema, reduction of erythema, reduction of pruritus or burning sensation, reduction of body temperature, improvement in function of the afflicted organ) as well as any of the methods provided below as well as any of the methods provided below.

The therapeutic effect of compounds of the present invention was determined in *in vitro* and *in vivo* experiments

such as the following.

The beneficial antiinflammatory effect of the compounds of the present invention was determined in the following *in vitro* and *in vivo* experiments:

Formulations for oral administration can be so designed to enable bioavailability of the compound at the site of inflammation in the intestines. This can be achieved by different combinations of delayed release formulations. The compound of Formula **I** can also be used in the treatment of Crohn's disease and intestinal inflammation disease if the compound is applied in the form of an enema, for which a suitable formulation can be used.

The corresponding preparations of the compounds of the present invention can be used in the prophylaxis (including without limitation the prevention, delay or inhibition of recurrence of one or more of the clinical or subclinical symptoms discussed and defined in connection with the definitions of "treatment" above. as well as in the therapeutic treatment of several diseases and and pathological inflammatory conditions including: chronic obstructive pulmonary disorder (COPD) asthma, inflammatory nasal diseases such as allergic rhinitis, nasal polyps, intestinal diseases such as Crohn's disease, colitis, intestinal inflammation, ulcerative colitis, dermatological inflammations such as eczema, psoriasis, allergic dermatitis, neurodermatitis, pruritis, conjunctivitis and rheumatoid arthritis.

The biological effect of the compounds of the present invention was determined in the following *in vitro and in vivo* experiments:

## Assay of Binding to Human Glucocorticoid Receptor

The gene for the alpha isoform of human glucocorticoid receptor was cloned by reverse polymerase chain reaction. The total RNA was isolated from human peripheral blood lymphocytes according to the instructions of the manufacturer (Qiagen, Milano, Italy), transcripted into cDNA with AMV reverse transcriptase (Roche, Basel, Switzerland) and the gene was multiplied by specific primers 1) 5'ATATGGATCCCTGATGGACTCCAAAGAATCATTAACTCC3' and 2) 5'ATAT-CTCGAGGGCAGTCACTTTTGATGAAACAGAAG3'. The reaction product obtained was cloned into the XhoI/BamHI site of Bluescript KS plasmid (Stratagene, La Jolla, CA USA), subjected to sequencing by the dideoxy fluorescent method with M13 and M13rev primers (Microsynth, Balgach, Switzerland) and then it was cloned into the XhoI/BamHI site of pcDNA3.1 Hygro(+)plazmid (Invitrogen). $1x10^5$ COS-1 cells were seeded onto a 12-well plate (Falcon) in DMEM medium (Life Technologies, Carlsbad, CA USA) with 10% FBS (Biowhitaker) and cultivated to a 70% confluence at 37 °C in an atmosphere with 5% $CO_2$.

The medium was removed and 1 μg of DNA, 7 μl of PLUS reagent and 2 μl of Lipofectamin (Life Technologies) in 500 μl of DMEM were added per well. The cells were incubated at 37 °C in an atmosphere with 5% $CO_2$ and after 5 hours the same volume of 20% FBS/DMEM was added. After 24 hours, the medium was completely changed. 48 hours after transfection, the test compounds in different concentrations and 24 nM [$^3$H]dexamethazone (Pharmacia, Piscataway, NJ USA) in DMEM medium were added. The cells were incubated for 90 minutes at 37 °C in an atmosphere with 5% $CO_2$, washed three times with PBS buffer (Sigma, St. Louis, MO USA) cooled to 4 °C (pH=7,4), and then lysed in Tris buffer (pH=8,0) (Sigma, St. Louis, MO USA) with 0.2% of SDS (Sigma, St. Louis, MO USA). After the addition of UltimaGold XR (Packard BioScience, Groningen, The Netherlands) scintillation liquid, the residual radioactivity was read in a Tricarb (Packard) β-scintillation counter.

Compounds 7 and 10 have affinity for the glucocorticoid receptor since in the assay they displace radioactive dexamethasone from the glucocorticoid receptor. Other compounds of the invention will demonstrate similar results when tested in this assay.

## Assay of Inhibition of Mouse T-cell Hybridoma 13 Proliferation as a Result of Apoptosis Induction

Triplicates of test steroid dilution in RPMI medium (Institute of Immunology, Zagreb) with 10% FBS were added to a 96 well plate. To solutions containing compounds at various concentrations, 20000 cells per well were added and incubated overnight at 37 °C in an atmosphere with 5% $CO_2$. Then 1 μCi of [$^3$H]thymidine (Pharmacia, Piscataway, NJ USA) was added and the mixture was incubated for an additional 3 hours. The cells were harvested by applying a vacuum over GF/C filter (Packard). Onto each well, 30 μl of Microscynt O scintillation liquid (Packard) was added and the incorporated radioactivity was measured on a β-scintillation counter (Packard). The specificity of apoptosis induction by glucocorticoids was demonstrated by antagonizing the proliferation inhibition with mifepristone (Sigma, St. Louis, MO USA) a potent glucocorticoid receptor antagonist.

Compounds **7, 9** and **10** exhibit inhibition of T-cell hybridoma 13 proliferation in the concentrations from 1 μM to 1 nM. Other compounds of the invention will demonstrate antiproliferativeactivity where tested in this assay.

**Table 1a** $IC_{50}$ Values in T-cell Hybridoma 13 Assay

| Compound | $IC_{50}$ M |
|---|---|
| **7** | $4{,}64x10^{-7}$ |
| **9** | $3{,}03x10^{-7}$ |
| **10** | $4{,}17x10^{-8}$ |
| **13** | $2{,}87x10^{-7}$ |
| **14** | $4{,}55x10^{-7}$ |
| **16** | $1{,}12x10^{-7}$ |
| **17** | $4{,}16x10^{-8}$ |
| **18** | $4{,}74x10^{-7}$ |

$IC_{50}$ values were calculated using GraphPad Prism software.
All compounds having $IC_{50}$ values below 2 μM are considered active.

**Measurement of the inhibition of interleukin 4, interleukin 5 and interferon production by γ concanavalin-A induced murine splenocytes**

Splenocytes were isolated from the spleen of Balb/C mice sacrificed by thiopental injection (Pliva, Zagreb, Croatia). Spleens were chopped and mononuclear cells separated on Histopaque 1083 (Sigma Diagnostics, St. Louis, MO USA Cat. No 1083-1). Into a 96-well plate, compounds diluted in RPMI medium (Institute of Immunology, Zagreb, Croatia) were pipetted with 10% foetal bovine serum (Biowhittaker) and cells (200000 per well) in the same medium, and concanavalin-A stimulator (Sigma 2002-2003 cat. No C5275) at a final concentration of 5 μg/ml were added. The positive control, in place of the dilution of compounds, consisted of RPMI medium with 10% foetal bovine serum and concanavalin-A in the same concentration of. Cells were incubated for 72 hours at 37 °C, 95% humidity and in an atmosphere with 5% $CO_2$. Until determination of cytokines, the cells were frozen at -70 °C.
Cytokines interleukin 4, interleukin 5 and interferon γ were determined by the specific ELISA method, according to manufacturer's recommendations (R&D).
Inhibition (as percentage) was calculated using the following formula:

$$\%inh = (1 - [\text{concentration of cytokines in sample}]/[\text{concentration of cytokines in positive control}]) * 100$$

Compound **10** inhibits the production of cytokines in concentrations from 1 μM to 1 nM.

**Model of Lung Eosinophilia in Mice**

Male Balb/C mice with a body weight of 20-25 g were randomly divided into groups, and sensitised by an i.p. injection of ovalbumin (OVA, Sigma, St. Louis, MO USA) on day zero and day fourteen. On the twentieth day, the mice were subjected to a challenge test by i.n. (intranasal) application of OVA (positive control or test groups) or PBS (negative control). 48 hours after i.n. application of OVA, the animals were anaesthetized and the lungs were rinsed with 1 mL of PBS. The cells were separated on Cytospin 3 cytocentrifuge (Shandon). The cells were stained in Diff-Quick (Dade) and the percentage of eosinophils was determined by differential counting of at least 100 cells.
Beclomethasone (Pliva d.d.) were used as a standard substances, with positive and negative control. The compounds were administered daily i.n. or i.p. in different doses 2 days before the challenge test and up to the completion of the test. Corticosterone levels were determined in plasma from each animal using a kit for determination of corticosterone (R&D systems). Compound **10** (2 mg/kg intranasaly) had no effect on corticosterone levels, while beclomethasone (1 mg/kg intranasaly) used as standard significantly suppressed corticosterone levels.
Compound **10** also statistically significantly reduced (t-test, p<0.05) the number of eosinophils in the lung rinse with respect to positive control. It is anticipated that similar results will be observed for other compounds of the invention.

**Croton oil induced ear edema in male Sprague-Dawley rats**

Test and reference substances, as well as vehicle (acetone), were administered topically to the inner and the outer surface of the right ear of each animal with an automatic pipette, in a volume of 60μL/ear (30μL/surface), thirty minutes before the croton oil challenge. Test substances were administered in the doses of 2 or 5 mg/ear/60μL of acetone. Dexamethasone was administered in the dose of 1 mg/ear/60μL of acetone. Thirty minutes later, 20% croton oil emulsion in acetone was applied topically to the inner and the outer surface of the right ear of each animal with an automatic pipette, in a volume of 60μL/ear (30μL/surface). Five hours after the challenge, animals were euthanized by asphyxiation in 100% $CO_2$ atmosphere. For assessing the auricular edema, 8mm discs were cut out of left and right auricular pinna and weighed. The degree of edema was calculated by subtracting the weight of 8 mm disc of the untreated ear from that of the treated contralateral ear. The inhibition of edema in the treated animals was presented as a percentage of that in the control rats (0%).

Dexamethasone, a reference substance, applied topically once (1 mg/ear), significantly reduced ear edema by 85.77% ($p<0.05$, Non-parametric ANOVA, n = 8). Compound 10, a test substance, applied topically once as a single dose of 2 mg/ear reduced the ear edema by 51.58% ($p>0.05$ by Non-parametric ANOVA, p=0.0285 by Unpaired t-test, n = 8) and applied topically once, in a dose of 5mg/ear, significantly reduced ear edema by 85.53% ($p<0.05$, Non-parametric ANOVA, n = 8).
In another experiment compound 17 applied topically once in a dose of 5 mg/ear, significantly reduced ear edema by 76.59% (ANOVA with Tukey-Kramer Multiple Comparisons Test, $p<0.01$, n = 8).

**Subcutaneous sponge implantation model**

In this model, local inflammation was followed by determination of newly formed granulation tissue together with determination of compound effects on plasma corticosterone concentration and thymus weight in Sprague-Dawley rats. Sprague - Dawley rats were purchased from Iffa Credo, Lyon, France and 10 weeks old male rats were used. PVA sponge material was purchased as 5 mm thick sheets (Oriolik, Croatia). The sponge sheets were cut into 14 mm disks using appropriate cork bore. The disks were pre-washed first with running tap water, followed by demineralized water and finally soaked in Pursept solution (Merz Hygiene, Germany) for 1 hour. Afterwards, they were rinsed well with demineralized water, dryed and heated at 80°C for 2 hours and stored under sterile conditions. All substances were dissolved in ethanol, applied onto sterile sponges and allowed to dry in the laminar flow cabinet prior to the sponge implantation. Compound 10 was administered in a single dose of 10mg/0.3mL/sponge while beclomethasone dipropionate was applied at 2 or 10 mg/0.3mL/sponge.
On day 0, animals were anaesthetized by inhalation of Isoflurane (Abbott Laboratories Ltd., USA) and 5% oxygen, delivered in an anesthesia induction chamber (Stoelting Co., USA). Gas scavenging was provided using the Fluovac 240V system (International Market Supply, England). Anesthesia was maintained using a gas anesthesia mask (Stoelting Co., USA) and pedal reflex response where checked at intervals. The implantation area was shaved and subsequently disinfected using Pursept solution (MERZ Hygiene, Germany). Using strict aseptic procedure, two 1 cm long skin incisions were made just below the left and right *regio scapularis.* Small subcutaneous pockets were made on the left side of the left wound and the right side of the right wound using blunt forceps. In all animals, sponges with vehicle or dissolved substance applied were inserted on the left side and non-treated ones on the right side. Skin was closed with sutures and disinfected with Pursept solution. On day 7 rats were anaesthetized by intraperitoneal administration of Thiopental (PLIVA; 0,5mL/100g body weight) and exanguinated by puncturing *A. carotis com.* Blood was collected into Vacutainer tubes (Becton Dickinson, USA) for plasma corticosterone concentration analysis. Corticosterone levels in plasma were measured using R&D Systems kit for quantitative determination of corticosterone. Competitive ELISA was performed with alkaline phosphatase conjugated corticosterone as competitor. Samples were incubated for 2 hours, and after successive washing, substrate was added. After 1h absorbance was measured on 405 nm. OD values are inversely correlated with increasing corticosterone concentrations. Corticosterone concentrations were calculated using calibration curve generated with corticosterone standard concentration dilutions.
For assessing the thymus weight, thymuses were extirpated and weighed using the analytical balance. The reduction of thymus size in treated animals was presented as a percentage of that in control rats (0%).
For assessing the newly formed granulation tissue, sponges were carefully excised and each sponge perforated with sterile surgical sewing suture to keep it on the top of the Falcon tube during centrifugation. Tubes were centrifuged (1000 rpm per 10 minutes). Sponges were put into sterilizer and heated at 60°C to dry. 24 hours later, the sponges were weighed using the analytical balance for measurement of newly formed granulation tissue. The weight of treated sponge was compared to the weight of contra-lateral non-treated sponge of the same animal.
For thymus and sponge weight comparison, one-way Analyses of Variance (ANOVA) with Tukey-Kramer Multiple Comparisons Test was used. For corticosterone concentration comparison non-parametric ANOVA - Kruskal-Wallis Test with Dunn's Multiple

Comparisons test was performed. Level of significance was set at $p<0.05$.
Seven days after sponge implantation, compound 10, administered into the sponge at single dose of 10 mg/sponge, significantly reduced granulation tissue formation, in comparison to non-treated and vehicle sponges. In comparison to vehicle beclomethasone significantly decreased thymus weight seven days after sponge implantation, at both doses: 2 and 10 mg/sponge while compound 10 administered at dose of 10 mg/sponge had no effect on thymus weight. In comparison to vehicle control beclomethasone at dose of 10 mg/sponge significantly decreased plasma corticosterone concentration while compound 10 had no significant effect on plasma corticosterone levels.

**Lung neutrophilia induced by bacterial lipopolysaccharide**

Male Balb/cJ mice (Iffa Credo, Lyon, France) weighing 25 g were randomly grouped in three groups: test, positive and negative control (10 animals per each group). Vehicle, beclomethasone dipropionate or test substance were applied intranasally (i.n.) to Balb/cJ mice. Thirty minutes later, 60 $\mu$l of bacterial lipopolysaccharide (LPS), dissolved in PBS at the concentration of 167 $\mu$g/ml, was given i.n. to all groups except the negative control group, which received the same volume of PBS. Animals were sacrificed after 24 hours and bronchoalveolar lavage fluid (BALF) collected for determination of neutrophils and IL-6 and TNF-$\alpha$ concentration. Cytokines were measured using sandwich ELISA (R&D Systems). Statistical analysis was performed using GraphPad prism software, one-way ANOVA Turkey Kramer Multiple comparison test. Beclomethasone dipropionate (2 mg/kg) non-significantly decreased all tested parameters. Compared to positive control group, compound **10** in the form of phosphate salt (4 mg/kg) statistically significantly decreased neutrophils and concentrations of TNF-$\alpha$ and IL-6 in BALF.

**SYNTHETIC METHODS AND EXAMPLES**

PRECURSORS

In the following examples of methods of preparation, which in no way limit the uniqueness of the invention, the synthesis of the compound of Formula I from macrolide precursors **M1-M8** and steroid precursors **S1-S24** and nonsteroidal precursors **D10, D11** and D12 is described.

Macrolide subunits

Macrolide subunits **M1-M8** are compounds represented by the following general structure:

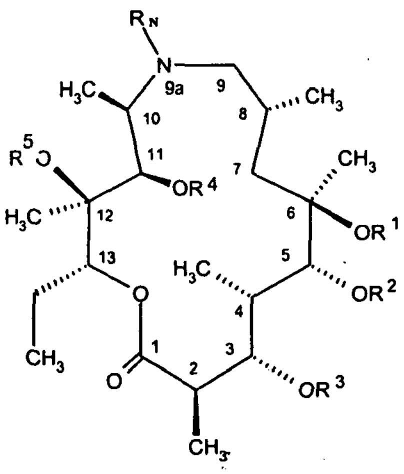

Table 1

| | $R_N$ | $R^4$ | $R^5$ | Molecular MH formula + | MH + |
|---|---|---|---|---|---|
| **M1** | H | H | H | $C_{21}H_{41}NO_7$ | 420, 2 |
| **M2** | $CH_2CH_2CN$ | H | H | $C_{24}H_{44}N_2O_7$ | 473, 3 |

(continued)

| | $R_N$ | $R^4$ | $R^5$ | Molecular MH formula + | MH + |
|---|---|---|---|---|---|
| **M3** | $CH_2-(CH_2)_2-NH_2$ | H | H | $C_{24}F_{48}N_2O_7$ | 477, 4 |
| **M4** | $CH_3$ | H | H | $C_{22}H_{43}NO_7$ | 434, 7 |
| **M5** | $CH_3$ | >C=O | | $C_{23}H_{41}NO_8$ | 460, 4 |
| **M6** | $CH_3$ | $C-(O)-NH-(CH_2)_4-NH_2$ | H | $C_{27}H_{53}N_3O_8$ | 548, 4 |
| **M7** | H | >C=O | | $C_{22}H_{39}NO_8$ | 446, 5 |
| **M8** | H | $C-(O)-NH-(CH_2)_4-NH_2$ | H | $C_{26}H_{51}N_3O_8$ | 534, 4 |
| $R^1=R^2=R^3=H$ | | | | | |

Method A

a) Compound **M1** (480 mg; 1.1 mmol) was dissolved in 10 mL of acrylonitrile and the reaction mixture was heated at 95 °C for 24 hours. Subsequently, the solvent was evaporated under reduced pressure. 500 mg of the compound **M2** was obtained, which was used for further synthesis without previous purification.

b) Compound **M2** (500 mg) was dissolved in 20 mL of absolute ethanol and hydrated with the catalyst $PtO_2$ (60 mg) for two days at the pressure of 40 atm. The mixture was purified was purified on a silica gel column, eluent

$CHCl_3$:MeOH:$NH_4OH$-6:1:0.1. 193 mg of compound **M3** was obtained. The properties of compounds **M1, M2** and **M3** are given in Table 1.

Method B

a) Azithromycin (11g) was dissolved in 40 mL $CHCl_3$ and 80 mL 6M HCl and the reaction mixture was heated at 60 °C for 20 hours. Organic and aqueous layers were separated and then the pH of the aqueous phase was adjusted to 9.5 and the aqueous layer was then extracted with dichloromethane. The organic layer was dried over anhydrous $Na_2SO_4$ and evaporated. 6 g of compound **M4** was isolated. MS (MH+) = 434.7

b) Compound **M4** (5.95 g, 13.7 mmol), ethylene carbonate (7.3 g, 82.5 mmol) and pottasium carbonate (2,28 g, 16,5 mmol) were mixed in 100 mL ethyl acetate and heated at 75°C under reflux for 72 h. Organic and aqueous layers were separated and the organic layer was dried over anhydrous $Na_2SO_4$ and evaporated. The mixture was purified on a silica gel column in the solvent system $CHCl_3$ : MeOH : $NH_4OH$ = 6:1:0.1. 3.5 mg of compound **M5** was isolated. MS (m/z): 460.4 $[MH]^+$.

c) In 1.3 mL (12.85 mmol) of 1,4-diaminobutane, 118 mg ( 0.26 mmole) of compound **M5** was dissolved. Then, 30 mg (0.26 mmole) of pyridine hydrochloride was added to the solution. The reaction mixture was stirred at room temperature for 20 hours. The product was extracted by dichloromethane and washed with water and the organic layer was subsequently dried over $Na_2SO_4$ and the solvent evaporated under reduced pressure. After purification of the mixture on a silica gel column in the solvent system $CH_2Cl_2$: MeOH: $NH_4OH$= 30: 50: 2, 50 mg of the amine **M6** was obtained. MS $(MH^+)$ = 548.4

d) Compound **M1** (5.05 g, 12.04 mmol) was mixed with ethylencarbonate (6.5 g, 7.38 mmol) and potassium-carbonate (1.7 g, 1.23 mmol). To the reaction mixture, ethyl acetate (90 ml) was added. The solution was heated to 75 °C under stirring for 24 hours. The mixture was washed with water (2x50 ml). Organic layer was then diluted with water (50 ml), adjusted to pH 7 with 2 M HCl and separated. Organic layer was again diluted with water (50 ml), adjusted to pH 7 with 2 M HCl and separated. The organic layers were combined, dried over anhydrous sodium sulphate, filtered and concentrated under vacuum. 1.7 g of the compound **M7** was obtained. MS(ES) m/z: $[MH]^+$ 446.31

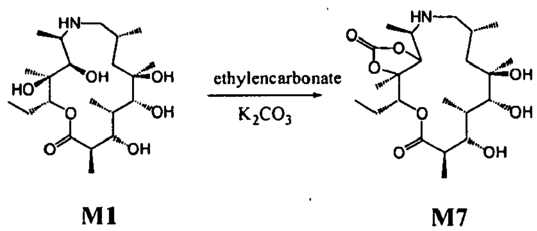


**M1** **M7**

e) In 2,25 ml (22.39 mmol) of 1,4-diaminobutane, 200 mg (0.45 mmole) of compound **M7** was dissolved. Then, 52 mg (0.45 mmol) of pyridine hydrochloride was added to the solution. The reaction mixture was stirred at room temperature for 3 days. The product was extracted by dichloromethane and washed with water and the organic layer was subsequently dried over $Na_2SO_4$ and the solvent evaporated under reduced pressure. After purification of the mixture on a silica gel column in the solvent system $CH_2Cl_2$: MeOH: $NH_4OH$= 30: 50: 2, 60 mg of the amine **M8** was obtained. MS ($MH^+$) = 534.4

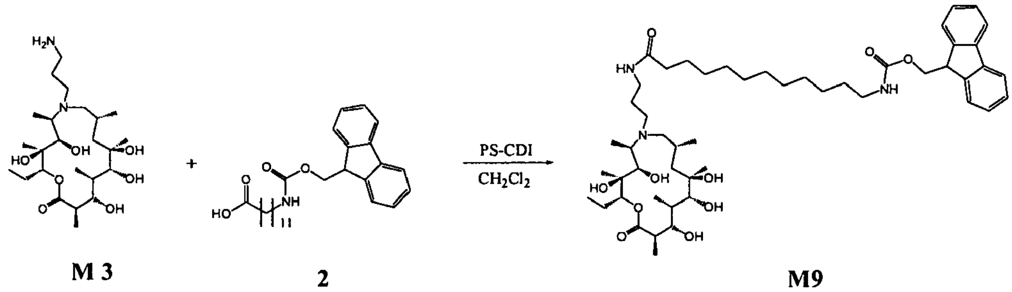


**M 3** **2** **M9**

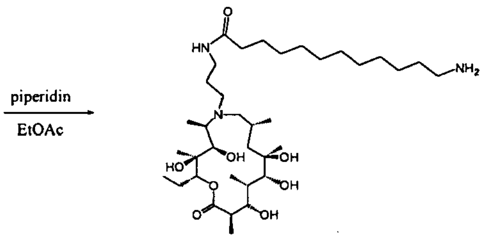


**M10**

**Compound M9.** PS-Carbodiimide resin (1.15 mg; 1.38 mmol) was added to a dry reaction vessel. 12-(Fmoc-amino)dodecanoic acid (Fluka, Lot 440842/1 50903094) (**2**) (200 mg; 0.46 mmol) in $CH_2Cl_2$ (7.5 ml) was added to the dry resin and the mixture stirred at room temperature. After 45 minutes, compound **M3** (109 mg; 0.23 mmol) in $CH_2Cl_2$ (3.5 ml) was added and the reaction stirred at 50 °C for 20 hours to afford the amide product. The mixture was filtered and solvent was concentrated under vacuum. Crude product was purified on silica gel column in the solvent system $CH_2Cl_2$:MeOH:$NH_4OH$ 6:1:0.1 the compound **M9** (160 mg) was obtained.

LC/MS (area %): 93.4 %.

HPLC-MS: MS(ES) m/z: $[MH]^+$ 896.66 (calcd. : 896.59)

**Compound M10.** Compound **M9** (160 mg, 0.18 mmol) was dissolved in piperidine (1 ml) and $CH_2Cl_2$ (5 ml). The reaction mixture was stirred at room temperature for 2 hours. The solvents were evaporated and remaining traces of piperidine were removed by addition and removal under vacuum of $CH_2Cl_2$ (several portions). Crude product was purified on silica gel column in the solvent system $CH_2Cl_2$:MeOH:$NH_4OH$ 6:1:0.1 the compound **M10** (132 mg) was obtained.

LC/MS (area %) : 95.7 %.

HPLC-MS: MS(ES) m/z: $[MH]^+$ 674.62 (calcd.: 674.52)

IR (KBr) $cm^{-1}$ : 3307, 3093, 2927, 2854, 1722, 1715, 1667, 1660, 1651, 1645, 1557, 1539, 1505, 1463, 1456, 1373, 1353, 1265, 1165, 1091, 1053, 958, 811, 736.

Steroid subunits

Steroid subunits **S1-S24** are compounds represented by the following general structure:

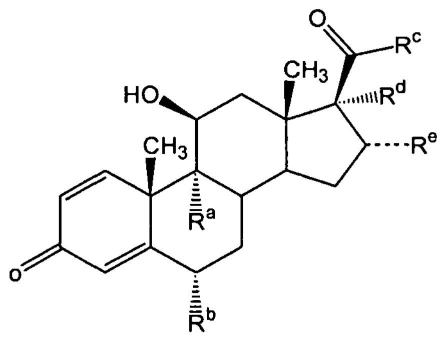

Table 2

| | Ra | Rb | Rc | Rd | Re | Molecular formula |
|---|---|---|---|---|---|---|
| **S1** | F | H | OH | OH | $CH_3$ | $C_{21}H_{27}FO_5$ |
| **S2** | F | F | OH | OH | $CH_3$ | $C_{21}H_{26}F_2O_5$ |
| **S3** | H | H | $OCH_3$ | OH | H | $C_{21}H_{28}O_5$ |
| **S4** | F | H | OH | OH | H | $C_{20}H_{25}FO_5$ |
| **S5** | H | F | OH | OH | $CH_3$ | $C_{21}H_{27}FO_5$ |
| **S6** | H | $CH_3$ | OH | OH | H | $C_{21}H_{28}O_5$ |
| **S7** | F | H | $NH(CH_2)_2NHB$ oc | H | $CH_3$ | $C_{28}H_{41}FN_2O_5$ |
| **S8** | F | F | OH | DDO | | $C_{23}H_{28}F_2O_6$ |
| **S9** | F | H | OH | OC(O)C=C | $CH_3$ | $C_{24}H_{29}FO_6$ |
| **S10** | F | H | $OCH_3$ | OC(O)C=C | $CH_3$ | $C_{25}H_{31}FO_6$ |
| **S11** | F | F | OH | OC(O)C=C | $CH_3$ | $C_{24}H_{28}F_2O_6$ |
| **S12** | F | F | $OCH_3$ | OC(O)C=C | $CH_3$ | $C_{25}H_{30}F_2O_6$ |
| **S13** | F | H | $NH(CH_2)_2NH_2$ | H | $CH_3$ | $C_{23}H_{33}FN_2O_3$ |
| **S14** | F | H | $OCH_3$ | $O(C)O(CH_2)_2NH(CH_2)_2N$ HBoc | $CH_3$ | $C_{32}H_{47}FN_2O_8$ |
| **S15** | H | F | OH | OC(O)C=C | $CH_3$ | $C_{24}H_{29}FO_6$ |
| **S16** | H | H | OH | OH | H | $C_{21}H_{27}FO_5$ |
| **S17** | H | H | OH | OC(O)C=C | H | $C_{24}H_{30}O_6$ |
| **S18** | H | F | $OCH_3$ | OC(O)C=C | $CH_3$ | $C_{25}H_{31}FO_6$ |
| **S19** | H | H | $OCH_3$ | OC(O)C=C | H | $C_{24}H_{30}O_6$ |
| **S20** | F | H | $S(O)N(CH_3)_2$ | OC(O)C=C | H | $C_{27}H_{34}FNO_6S$ |
| **S21** | F | H | $O(O)N(CH_3)_2$ | OC(O)C=C | H | $C_{27}H_{34}FNO_7$ |
| **S22** | F | H | $OCH_3$ | $O(C)O(CH_2)_2NH(CH_2)_2N\ H_2$ | $CH_3$ | $C_{27}H_{39}FN_2O_6$ |
| **S23** | H | $CH_3$ | OH | OC(O)C=C | H | $C_{24}H_{30}O_6$ |

(continued)

| | $R^a$ | $R^b$ | $R^c$ | $R^d$ | $R^e$ | **Molecular formula** |
|---|---|---|---|---|---|---|
| **S24** | H | $CH_3$ | $OCH_3$ | OC(O)C=C | H | $C_{25}H_{32}O_6$ |
| DDO = 2,2-dimethyl-1,3-dioxazolone | | | | | | |

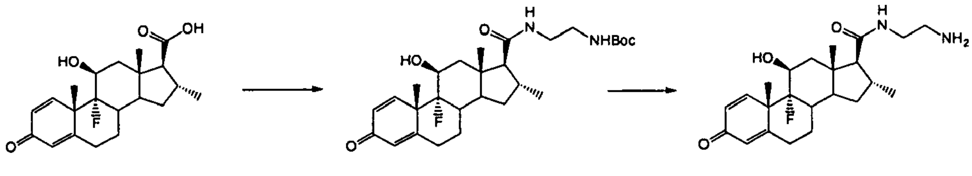

S4 S7 S13

Scheme 1

Preparation of S7 (Scheme 1)

To a solution of compound **S4** (500 mg, 1.38 mmol) in dry $CH_2Cl_2$ (10 ml) under argon, were added triethylamine (1.5 mL, 10.76 mmol), 1-hydroxybenzotriazole (373 mg, 2.76 mmol), $NH_2CH_2CH_2NHBoc$ (218 µl, 1.38 mmol) and 1-(3-dimethylaminopropyl)-3-ethylcarbodiimide hydrochloride (1.06 g, 5.52 mmol). The reaction mixture was stirred for 24 hours at room temperature in a flow of argon and concentrated under reduced pressure. Purification on a silica gel column (eluent: $CH_2Cl_2$:MeOH:$NH_4OH$=6:1:0.1) gave 646 mg of the compound **S7**.
MS(ES) m/z: $[MH]^+$ 505.46

Preparation of **S13** (Scheme 1)

A solution of compound S7 (630 mg, 1.25 mmol) in TFA (3 ml) and $CH_2Cl_2$ (3 ml) was stirred at room temperature for 1,5 hours. TFA and $CH_2Cl_2$ was removed under vacuum, and remaining traces of TFA were removed by addition and removal under vacuum of $CH_2Cl_2$ (several portions) to give 1.32 g of the compound **S13.**
MS(ES) m/z: $[MH]^+$ 405.30

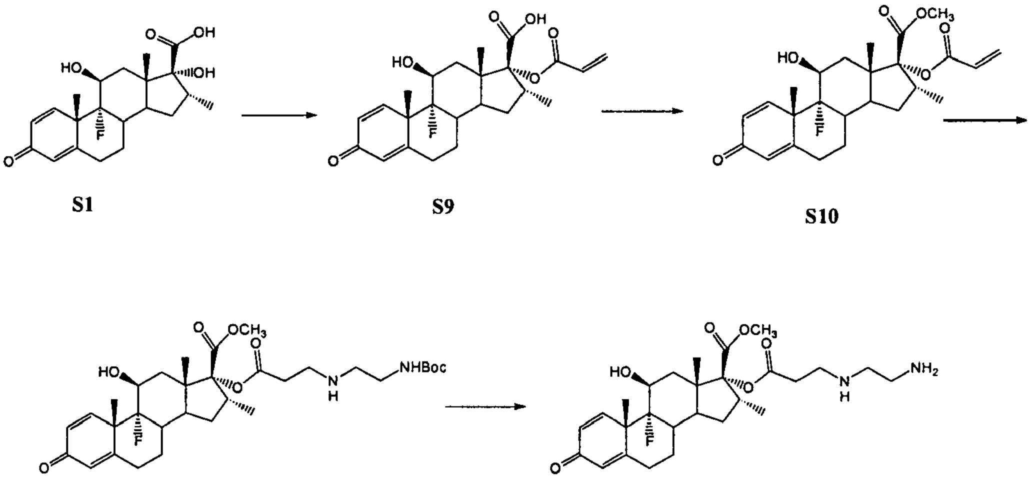

S1 S9 S10

S14 S22

Scheme 2

Preparation of **S9** (Scheme 2)

A solution of steroid **S1** (1.5 g, 3.96 mmol) and triethylamine (1.1 ml, 7.93 mmol) in $CH_2Cl_2$ (40 ml) at 0 °C was treated with acryloyl chloride (644 μl, 7.93 mmol). After 30 min the reaction mixture was washed with sat. $NaHCO_3$ and then $H_2O$, dried over anhydrous $Na_2SO_4$ and evaporated under reduced pressure to give the solid intermediate. This was stirred in acetone (30 mL) with diethylamine (2.07 mL, 19.8 mmol) for 2 hours. Solution was concentrated, diluted with water and washed with ethyl acetate. The aqueous phase was acidified to pH 2 with 2 M HCl and filtered to provide a solid. 1.28 g of the compound **S9** was obtained. MS(ES) m/z: $[MH]^+$ 433.28; IR (KBr) $cm^{-1}$ : 3477, 2940, 2879, 2601, 1731, 1655, 1596, 1452, 1401, 1376, 1276, 1401, 1376, 1276, 1221, 1195, 1146, 1118, 1064, 1038, 1011, 975, 951, 931, 904, 874, 832, 812, 767, 703, 657, 635.

Preparation of **S10** (Scheme 2)

To a solution of compound **S9** (1.1 g, 2.54 mmol) in dry THF (8 ml) under argon, was added $LiOHxH_2O$ (106 mg, 2.54 mmol). Reaction mixture was stirred at room temperature for 30 min. $Me_2SO_4$ (235 μl, 2.54 mmol) was then added and the resulting mixture was stirred at 65 °C for 3 hours. The reaction mixture was diluted with ethyl acetate (30 ml) and washed with sat. $NaHCO_3$ and then with water. Organic layer was dried over $Na_2SO_4$ and the solvent evaporated under reduced pressure. 925 mg of the compound S10 was obtained. MS(ES) m/z: $[MH]^+$ 447.32; IR (KBr) $cm^{-1}$ : 3333, 3111, 2944, 2878, 1753, 1728, 1659, 1604, 1450, 1434, 1409, 1285, 1262, 1239, 1192, 1148, 1117, 1101, 1065, 1043, 1013, 977, 928, 893, 879, 809, 707, 686, 662.

Preparation of **S14** (Scheme 2)

In a solution of compound **S10** (800 mg, 1.8 mmol) in MeOH (20 ml) and $CH_3CN$ (10 ml) $NH_2CH_2CH_2NHBoc$ (570 μl, 3.6 mmol) was added. Reaction mixture was stirred at 55 °C for 24 hours. After evaporation of the solvent under reduced pressure mixture was purified on a silica gel column in the solvent system $CH_2Cl_2$:MeOH:$NH_4OH$=90:9:1.5. 925 mg of the compound **S14** was obtained. MS(ES) m/z: $[MH]^+$ 607.38 ; IR (KBr) $cm^{-1}$ : 3404, 2975, 2941, 2878, 1745, 1665, 1619, 1509, 1459, 1392, 1366, 1268, 1243, 1175, 1116, 1064, 1047, 1032, 1015, 977, 929, 889, 814, 795, 735, 706, 687, 656..

Preparation of **S22** (Scheme 2)

A solution of compound **S14** (697 mg, 1.15 mmol) in TFA (2 ml) and $CH_2Cl_2$ (2 ml) was stirred at room temperature for 2 hours. TFA and $CH_2Cl_2$ was removed under vacuum, and remaining traces of TFA were removed by addition and removal under vacuum of $CH_2Cl_2$ (several portions). Crude product was diluted in $CH_2Cl_2$ (20 ml) and extracted with water.

Aqueous layer was neutralised with NaOH and extracted with ethyl acetate (2x20 ml). The organic layers were combined, dried over Na2SO4, filtered and concentrated under vacuum. 523 mg of the compound **S22** was obtained. MS(ES) m/z: $[MH]^+$ 507.44; IR (KBr) $cm^{-1}$ : 3416, 2940, 2878, 1741, 1664, 1619, 1452, 1392, 1375, 1268, 1240, 1201, 1178, 1117, 1064, 1047, 1032, 1014, 977, 928, 889, 799, 721, 686.

Preparation of **S15**

A solution of steroid **S5** ( 500 mg, 1.32 mmol) and triethylamine (0.37 mL, 2.64 mmol) in $CH_2Cl_2$ ( 30 ml) at 0°C was treated with acryloyl chloride (0.22 mL, 2.64 mmol). After 30 min the reaction mixture was diluted with $CH_2Cl_2$, washed with aqueous $NaHCO_3$ and then $H_2O$, dried and evaporated to give the solid intermediate. This was stirred in acetone (25 mL) with diethylamine (0.69 mL, 6.61 mmmol) for 2 hours. Solution was concentrated, diluted with water and washed with EtOAc.The aqueous phase was acidified to pH 2 with 2 N HCl and filtered to provide a solid. 259 mg of compound S15 was obtained. MS (m/z): 433.36 $[MH]^+$; IR($cm^{-1}$)/KBr: 3438, 3246, 2937, 2878, 2656, 1731, 1717, 1663, 1628, 1609, 1458, 1453, 1409, 1365, 1318, 1300, 1278,1260, 1193, 1180, 1118, 1080, 1061, 1038, 988, 971, 928,900,835,807,780,719,706.

Preparation of **S11**

A solution of steroid **S2** (0.5 g, 1.26 mmol) and triethylamine (0.35 mL, 2.52 mmol) in $CH_2Cl_2$ (30 ml) at 0°C was treated with acryloyl chloride (0.21 mL, 2.52 mmol). After 30 min the reaction mixture was diluted with $CH_2Cl_2$, washed with aqueous $NaHCO_3$ and then $H_2O$, dried and evaporated to give the solid intermediate. This was stirred in acetone (25 ml) with diethylamine (0.66 mL, 6.31 mmol) for 2 hours. Solution was concentrated, diluted with water and washed with EtOAc.The aqueous phase was acidified to pH 2 with 2 N HCl and filtered to provide a solid. 212 mg of

compound **S11** was obtained. MS (*m/z*): 451.00 $[MH]^+$; IR($cm^{-1}$)/KBr: 3423, 2941, 2880, 2624, 1726, 1665, 1619, 1609, 1458, 1407, 1377, 1301, 1261, 1234, 1199, 1150, 1120, 1071, 1041, 1028, 993, 978, 937, 899, 851, 808, 777, 710,659.

Preparation of **S12**

1, 8-Diazabicyclo[5.4.0]undec-7-ene (DBU) (1 equiv, 0.11 mmol) was added to a 10% solution of acid **S11** (0.11 mmol, 50 mg) in dimethylcarbonate and the resulting mixture was heated (100°C) for 10 minutes in microwave reactor. Reaction mixture was cooled to room temperature and diluted with $CH_2Cl_2$ and water. The organic layer was dried over $Na_2SO_4$, evaporated and purified on a silica gel column in the solvent system $CH_2Cl_2$:MeOH = 12:1. 39 mg of the compound **S12** was obtained. MS (m/z): 464.96 $[MH]^+$; IR($cm^{-1}$)/KBr: 3339, 2978, 2943, 2881, 1736, 1663, 1619, 1606, 1452, 1452, 1432, 1406, 1375, 1285, 1255, 1243, 1214, 1194, 1116, 1103, 1072, 1042, 1006, 990, 978, 933, 896, 851, 812, 735, 712, 685.

Preparation of **S17**

A solution of steroid **S16** (0.5 g, 1.44 mmol) and triethylamine (0,40 mL, 2.89 mmol) in $CH_2Cl_2$ (30 ml) at 0°C was treated with acryloyl chloride (0.24 mL, 2.89 mmol). After 30 min the reaction mixture was diluted with $CH_2Cl_2$, washed with aqueous $NaHCO_3$ and then $H_2O$, dried and evaporated to give the solid intermediate. This was stirred in acetone (25 mL) with diethylamine (0.75 mL, 7.22 mmol) for 2 hours. Solution was concentrated, diluted with water and washed with EtOAc.The aqueous phase was acidified to pH 2 with 2 N HCl and filtered to provide a solid. 260 mg of compound **S17** was obtained. MS (m/z): 401.25 $[MH]^+$; IR($cm^{-1}$)/KBr: 3567, 3448, 2938, 2914, 2851, 2643, 2602, 1732, 1713, 1653, 1606, 1596, 1452, 1406, 1394, 1346, 1297,1258, 1212, 1184, 1126, 1084, 1040, 988, 972, 941, 930, 908,888,828,812,769,719,656.

Preparation of **S18**

1, 8-Diazabicyclo[5.4.0]undec-7-ene (DBU) (1 equiv, 0.23 mmol) was added to a 10% solution of acid **S15** (0.23 mmol, 100 mg) in dimethylcarbonate and the resulting mixture was heated to reflux (90°C). After completion, the reaction mixture was cooled to room temperature and diluted with EtOAc and water. The organic layer was dried over $Na_2SO_4$, evaporated and purified on a silica gel column in the solvent system $CH_2Cl_2$:MeOH:$NH_4OH$ = 90:8:1. 40 mg of the compound **S18** was obtained. MS (*m/z*): 447.39 $[MH]^+$; IR($cm^{-1}$)/KBr: 3423, 3368, 2973, 2944, 2875, 1737, 1726, 1657, 1619, 1602, 1459, 1450, 1406, 1389, 1367, 1307, 1284, 1242, 1196, 1116, 1083, 1065, 1040, 987, 976, 941, 928, 895, 828, 812, 712, 671.

Preparation of **S19**

1, 8-Diazabicyclo[5.4.0]undec-7-ene (DBU) (1 equiv, 0.25 mmol) was added to a 10% solution of acid **S17** (0.25 mmol, 100 mg) in dimethylcarbonate and the resulting mixture was heated to reflux (90°C). After completion, the reaction mixture was cooled to room temperature and diluted with EtOAc and water. The organic layer was dried over $Na_2SO_4$, evaporated and purified on a silica gel column in the solvent system $CH_2Cl_2$:MeOH:$NH_4OH$ = 90:8:1. 42 mg of the compound **S19** was obtained.
MS (m/z): 415.32 $[MH]^+$

Preparation of **S23**

A solution of steroid **S6** (700 mg, 1.942 mmol) and triethylamine (0.54 mL, 3.884 mmol) in $CH_2Cl_2$ (50 ml) at 0°C was treated with acryloyl chloride (0.315 mL, 3.884 mmol). After 30 min the reaction mixture was diluted with $CH_2Cl_2$, washed with aqueous $NaHCO_3$ and then $H_2O$, dried and evaporated to give the solid intermediate. This was stirred in acetone (40 mL) with diethylamine (1.015 mL, 9.71 mmmol) for 2 hours. Solution was concentrated, diluted with water and washed with EtOAc.The aqueous phase was acidified to pH 2 with 2 N HCl and filtered to provide a solid. 111 mg of compound **S23** was obtained. MS (m/z): 415.48 $[MH]^+$

Preparation of **S24**

In a solution of compound **S23** (100 mg, 0.24 mmol) in dry THF (1.5 mL) 10,1 mg (0.24 mmol) of $LiOHxH_2O$ was added. Reaction mixture was stirred at room temperature for 30 min. In the reaction mixture 22.3 μL of $Me_2SO_4$ (0.24 mmol) was added and the mixture was stirred for 2 hours at 65°C. After completion, the reaction mixture was cooled to room temperature, diluted with 20 mL EtOAc and then treated sequentially with 20 mL of saturated $NaHCO_3$

and 20 mL of water. The organic layer was dried over $Na_2SO_4$, and evaporated. 121 mg of compound **S24** was obtained. MS (m/z): 429.48 $[MH]^+$

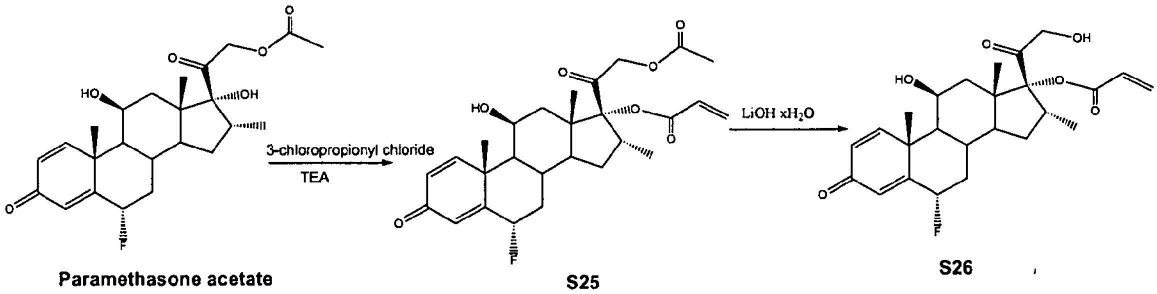


**Paramethasone acetate** **S25** **S26**

### Preparation of compound S25

To the solution of 1.0 g (2.30 mmol) paramethasone acetate in 20 mL toluene, 960 μL (6.90 mmol, 3eq) TEA was added, followed by 219 μL (2.30 mmol, 1 eq) 3-chloropropionyl chloride. The solution was stirred at room temperature for 24 hours, the evaporated and the crude product purified on the silicagel column, using solvent system ethyl-acetate: hexane 5:3. 417 mg of pure product was isolated.
MS (*m*/*z*):489.38 $[MH]^+$ MS(teor.)=488.55
Purity (HPLC-MS): 96.42 %
IR (KBr): 3386, 3112, 3037, 2960, 2924, 2876, 1751, 1724, 1679, 1619, 1602, 1501, 1452, 1410, 1388, 1373, 1342, 1318, 1267, 1234, 1198, 1178, 1139, 1120, 1092, 1050, 1028, 1011, 986, 916, 894, 871, 845, 816, 789, 742, 721, 695, 657.

### Preparation of compound S26

To the solution of 100 mg (0.205 mmol) compound **S25** in u 5 mL THF, 17 mg (0.410 mmol) $LiOHxH_2O$ and 5 mL water was added and the reaction mixture stirred at room temperature for 15 min. pH was then adjusted to 8 using 1M NaOH, and the product extracted with DCM. The organic layer was dried over anhydrous $Na_2SO_4$ and evaporated giving 28 mg of pure product.
MS (*m*/*z*):447.10 $[MH]^+$ MS(teor.)=446.51
Purity (HPLC-MS): 98.37 %

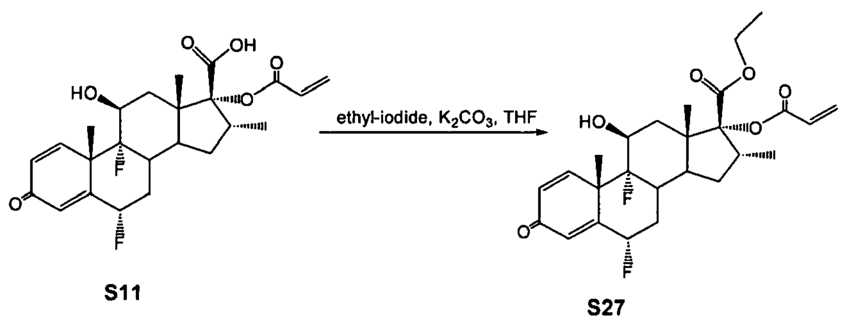


**S11** **S27**

### Compound S27

A solution of compound **S12** in 10 mL of dry THF was treated with 30.7 mg (1.0 mmol) $K_2CO_3$ and 0,0197mL (1.1 mmol) ethyl-iodide. The reaction mixture was stirred at room temp. for 24 h but no product was obtained. Heating the micture to 55°C resulted with product in 2 hours. The micture was poured into a mixture of 20 mL DCM and 20 mL water and extracted. The organic layer was washed with water, dried over anhydrous $Na_2SO_4$ and evaporated. 45 mg of crude product was isolated.
MS (*m*/*z*):479.09 $[MH]^+$ MS(teor.)=478.53

Purity (HPLC-MS): 76.77 %

Nonsteroidal subunits

Precursors for the synthesis are nonsteroidal anti-inflammatory drugs (NSAID), such as aceclofenac, acemetacin, acetaminophen, acetaminosalol, acetyl-salicylic acid, acetyl-salicylic-2-amino-4-picoline-acid, 5-aminoacetylsalicylic acid, alclofenac, amino-profen, amfenac, anileridine, bendazac, benoxaprofen, bermoprofen, $\alpha$-bisabolol, bromfenac, 5-bromosalicylic acid acetate, bromosaligenin, bucloxic acid, butibufen, carprofen, chromoglycate, cinmetacin, clindanac, clopirac, sodium diclofenac, diflunisal, ditazol, enfenamic acid, etodolac, etofenamate, felbinac, fenbufen, fenclozic acid, fendosal, fenoprofen, fentiazac, fepradinol, flufenamic acid, flunixin, flunoxaprofen, flurbiprofen, glutametacin, glycol salicylate, ibufenac, ibuprofen, ibuproxam, indomethacin, indoprofen, isofezolac, isoxepac, isoxicam, ketoprofen, ketorolac, lornoxicam, loxoprofen, meclofenamic acid, mefenamic acid, meloxicam, mesalamine, metiazinic acid, mofezolac, montelukast, naproxen, niflumic acid, olsalazine, oxaceprol, oxaprozin, oxyphenbutazone, parsalmide, perisoxal, phenyl-acethyl-salicylate, phenylbutazone, phenylsalicylate, pyrazolac, piroxicam, pirprofen, pranoprofen, protizinic acid, salacetamide, salicylamide-O-acetyl acid, salicylsulphuric acid, salicin, salicylamide, salsalate, sulindac, suprofen, suxibutazone, tenoxicam, tiaprofenic acid, tiaramide, tinoridine, tolfenamic acid, tolmetin, tropesin, xenbucin, ximoprofen, zaltoprofen, zomepirac, tomoxiprol, zafirlukast, and some example of precursors are flunixin **(D10),** flufenamic acid **(D11)** and celecoxib **(D12)** :

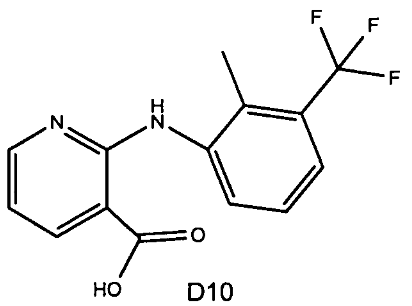

D10

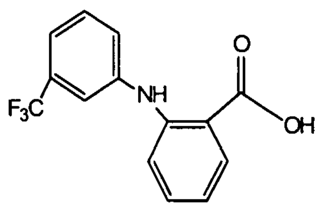

D11

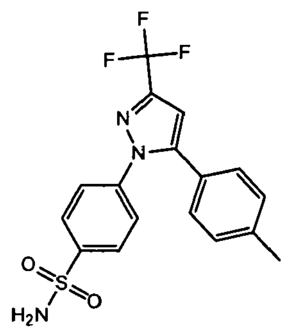

D12

Preparation of **D13** (Scheme 3)

A mixture of celecoxib **(D12)** (4 g, 10.5 mmol), DMAP (640 mg, 5.24 mmol), di-t-butyl dicarbonate (7.52 mL, 31.5 mmol) and triethylamine (1.75 mL, 12.57 mmol) in anhydrous THF (20 mL) was stirred at room temperature for 1 hour. Methyl bromoacetate (2.63 mL, 26.24 mmol) and $K_2CO_3$ (2.9 g, 21 mmol) was then added and the resulting mixture was stirred at room temperature for 22 hours. The reaction mixture was poured into sat. $NaHCO_3$ and extracted with ethyl acetate (2x50 mL). The organic layers were combined, washed with sat. NaCl (50 mL), dried over $MgSO_4$, filtered and concentrated under vacuum. The resulting glass was purified by chromatography (silica gel, 90:9:1.5 $CH_2Cl_2$:MeOH: $NH_4OH$) to afford 4.53 g of the product **D13** as a white powder. MS(ES) m/z: $[MH]^+$ 554.33 ; IR (KBr) $cm^{-1}$ : 3449, 3136,

3108, 2983, 1919, 1759, 1738, 1618, 1598, 1501, 1473, 1450, 1411, 1372, 1314, 1273, 1239, 1165, 1146, 1095, 1016, 995, 976, 939, 846, 808, 763, 744, 718, 653.

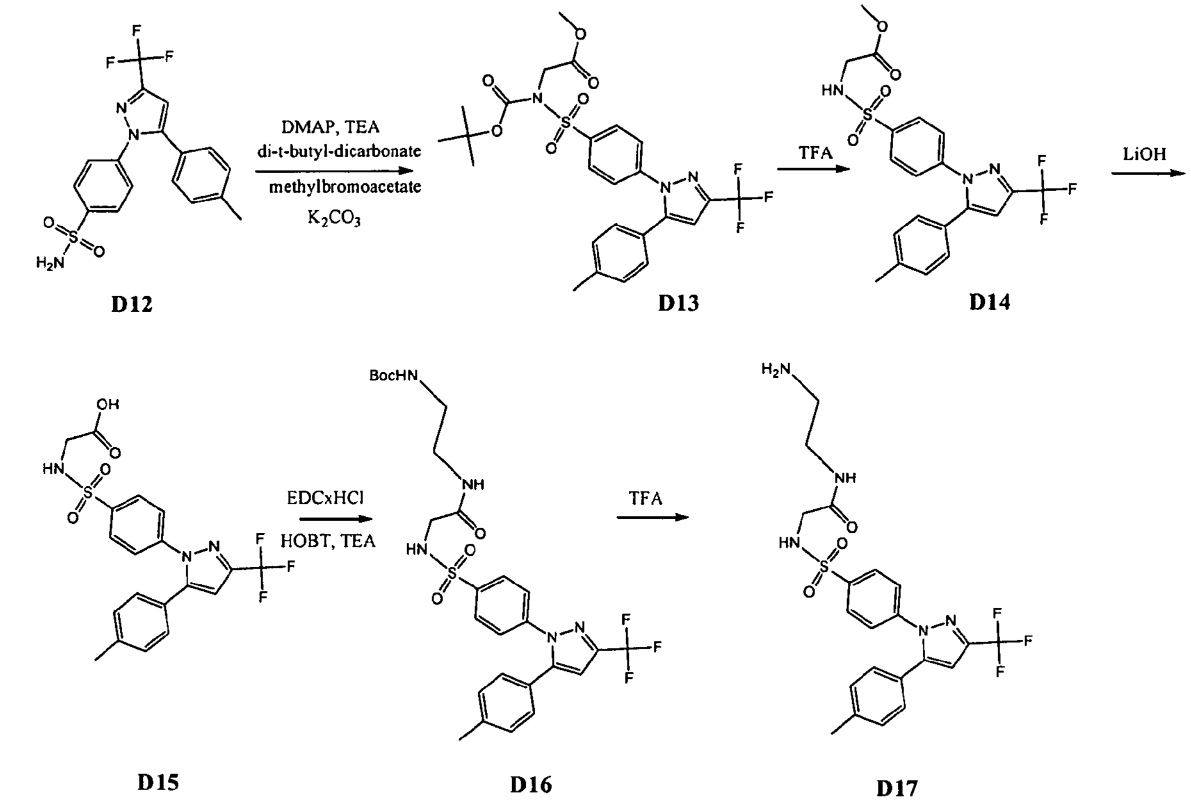


D12 D13 D14

D15 D16 D17

Scheme 3

Preparation of **D14** (Scheme 3)

A solution of compound **D13** (4.53 g, 8.18 mmol) in TFA (5 mL) and $CH_2Cl_2$ (5 mL) was stirred at room temperature for 2 hours. TFA and $CH_2Cl_2$ was removed under vacuum, and remaining traces of TFA were removed by addition and removal under vacuum of $CH_2Cl_2$ (several portions) to give 4.43 g oil product **D14**. MS(ES) m/z: $[MH]^+$ 454.27; IR (KBr) $cm^{-1}$ : 3281, 2954, 2925, 1747, 1595, 1555, 1499, 1476, 1438, 1411, 1376, 1354, 1324, 1279, 1238, 1216, 1162, 1133, 1100, 1016, 978, 949, 874, 849, 803, 762, 744, 722, 700, 633.

Preparation of **D15** (Scheme 3)

A solution of compound **D14** (4.43 g, 9.77 mmol) in THF (15 mL) was treated with a solution of LiOH (820 mg, 19.54 mmol) in water (15 mL) and stirred for 30 min. THF was removed under vacuum and resulting mixture was adjusted to pH 2 with 0.1 M HCl. Resulting solid was isolated by filtration to give 3.84 g of the compound **D15**. MS(ES) m/z: $[MH]^+$ 440.25; IR (KBr) $cm^{-1}$: 3396, 1606, 1574, 1501, 1472, 1415, 1373, 1326, 1274, 1238, 1169, 1156, 1129, 1098, 1022, 974, 930, 847, 813, 758, 628.

Preparation of **D16** (Scheme 3)

To a solution of compound **D15** (500 mg, 1.14 mmol) in dry $CH_2Cl_2$ (10 mL) under argon, were added triethylamine (1.4 mL, 10 mmol), 1-hydroxybenzotriazole (308 mg, 2.28 mmol), $NH_2CH_2CH_2NHBoc$ (180 μl, 1.14 mmol) and 1-(3-dimethylaminopropyl)-3-ethylcarbodiimide hydrochloride (874mg, 4.56 mmol). The reaction mixture was stirred for 24 hours at room temperature in a flow of argon and concentrated under reduced pressure. Purification on a silica gel column (eluent: $CH_2Cl_2$:MeOH:$NH_4OH$=6:1:0.1) gave 390 mg of the compound **D16**. MS(ES) m/z: $[MH]^+$ 582.33; IR

(KBr) $cm^{-1}$ : 3378, 2979, 2933, 2878, 1686, 1598, 1528, 1499, 1473, 1450, 1409, 1369, 1341, 1273, 1238, 1163, 1135, 1097, 1006, 976, 843, 827, 808, 761, 744, 719, 694, 627.

Preparation of **D17** (Scheme 3)

A solution of compound **D16** (300 mg, 0.52 mmol) in TFA (3 mL) and $CH_2Cl_2$ (5 mL) was stirred at room temperature for 2 hours. TFA and $CH_2Cl_2$ was removed under vacuum, and remaining traces of TFA were removed by addition and removal under vacuum of $CH_2Cl_2$ (several portions) to give 250 mg of the product **D17**. MS(ES) m/z: $[MH]^+$ 482.19.

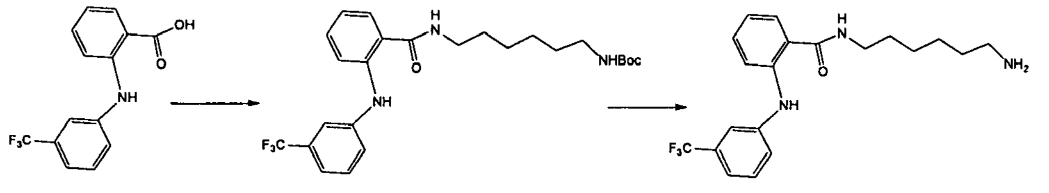

D11 D18 D19

Scheme 4

Preparation of **D18** (Scheme 4)

To a solution of flufenamic acid **D11** (245 mg, 0.87 mmol) in dry $CH_2Cl_2$ (20 ml) under argon, were added triethylamine (1.2 ml, 8.73 mmol), 1-hydroxybenzotriazole (240 mg, 1.78 mmol), $NH_2(CH_2)_6NHFmoc$ (300 mg, 0.9 mmol) and 1-(3-dimethylaminopropyl)-3-ethylcarbodiimide hydrochloride (700 mg, 3.65 mmol). The reaction mixture was stirred for 24 hours at room temperature in a flow of argon and concentrated under reduced pressure. Purification on a silica gel column (eluent: $CH_2Cl_2$:MeOH:$NH_4OH$=6:1:0.1) gave 430 mg of the compound **D18.**

Preparation of **D19** (Scheme 4)

A solution of compound **D18** (400 mg, 0.66 mmol) in ethyl acetate (5 mL) and piperidine (2 mL) was stirred at room temperature for 1 hours. Ethyl acetate and piperidine was removed under vacuum. 370 mg of the compound **D19** was obtained.
MS(ES) m/z: $[MH]^+$ 380.22

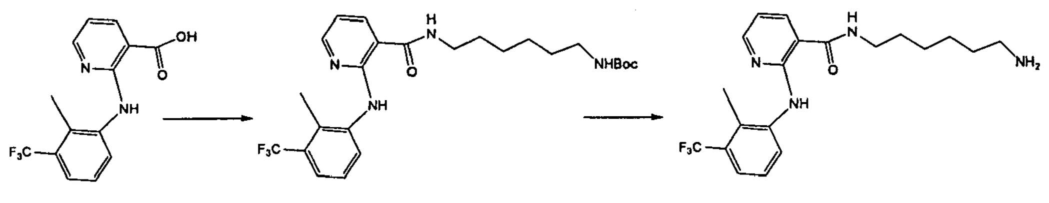

D10 D20 D21

Scheme 5

Preparation of **D20** (Scheme 5)

To a solution of flunixin **D10** (340 mg, 1.15 mmol) in dry $CH_2Cl_2$ (10 mL) under argon, were added triethylamine (1.6 mL, 11.5 mmol), 1-hydroxybenzotriazole (312 mg, 2.3 mmol), $NH_2(CH_2)_6NHFmoc$ (390 mg, 1.15 mmol) and 1-(3-dimethylaminopropyl)-3-ethylcarbodiimide hydrochloride (880 mg, 4.6 mmol). The reaction mixture was stirred for 24 hours at room temperature in a flow of argon and concentrated under reduced pressure. Purification on a silica gel column (eluent: $CH_2Cl_2$:MeOH:$NH_4OH$=6:1:0.1) gave 548 mg of the compound **D20**.
MS(ES) m/z: $[MH]^+$ 617.66

Preparation of **D21** (Scheme 5)

A solution of compound **D20** (548 mg, 0.89 mmol) in ethyl acetate (5 mL) and piperidine (2 mL) was stirred at room temperature for 1 hours. Ethyl acetate and piperidine was removed under vacuum. 732 mg of the product **D21** was obtained.
MS(ES) m/z: $[MH]^+$ 395.45

**Examples**

**Compound 1**

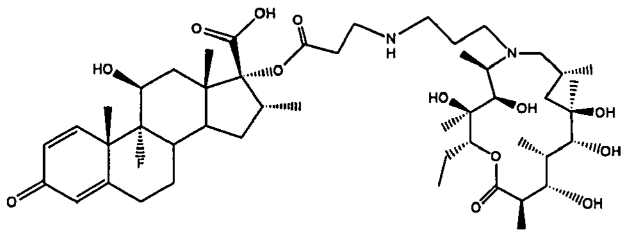

In a solution of compound **S9** ( 250 mg, 0.58 mmol) in methanol ( 20 mL) 915mg ( 1.16 mmol) of the macrolide **M3** was added. Reaction mixture was stirred at 55°C for 24 hours. After evaporation of the solvent, mixture was purified on a silica gel column in the solvent system $CH_2Cl_2$:MeOH:$NH_4OH$ = 30:50:2. 540 mg of the compound **1** was obtained. MS (m/z): 909,42 $[MH]^+$.

**Compound 2**

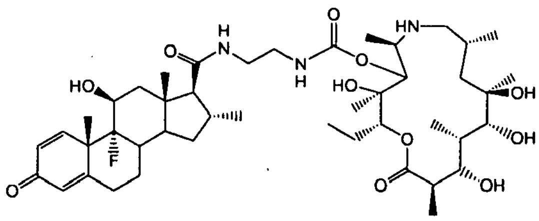

Compound **S13** (1.3 g, 3.2 mmol) and compound **M7** (130 mg, 0.3 mmol) was dissolved in pyridine (5 mL). Then, pyridine hydrochloride (35 mg, 0.3 mmol) and 1,8-diazabicyclo [5.4.0]-undec-7-ene (1 ml) were added to the solution. The reaction mixture was stirred at room temperature for 7 days. The product was extracted by $CH_2Cl_2$ and washed with water and the organic layer was subsequently dried over $Na_2SO_4$ and the solvent evaporated under reduced pressure. After purification of the mixture on a silica gel column in the solvent system $CH_2Cl_2$:MeOH:$NH_4OH$=6:1:0.1 80 mg of the product **2** was obtained. MS(ES) m/z: $[MH]^+$ 850.70; IR (KBr) $cm^{-1}$: 3409, 2939, 2877, 1664, 1535, 1495, 1381, 1296, 1248, 1163, 1091, 1070, 975, 928, 889, 829, 757, 702.

**Compound 3**

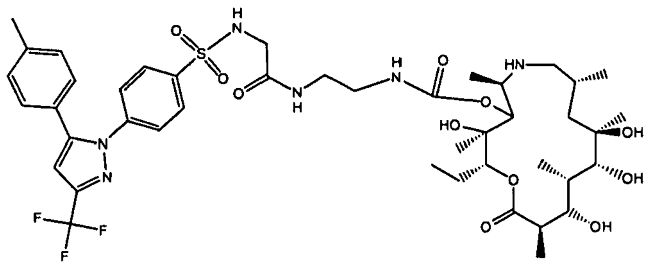

Compound **D17** (220 mg, 0.5 mmol) and compound **M7** (222 mg, 0.5 mmol) was dissolved in pyridine (5 mL). Then, pyridine hydrochloride (58 mg, 0.5 mmol) and 1,8-diazabycyclo [5.4.0]-undec-7-ene (1 mL) were added to the solution. The reaction mixture was stirred at room temperature for 6 days. The product was extracted by $CH_2Cl_2$ and washed with water and the organic layer was subsequently dried over $Na_2SO_4$ and the solvent evaporated under reduced pressure. After purification of the mixture on a silica gel column in the solvent system $CH_2Cl_2$:MeOH:$NH_4OH$=90:9:1.5 24 mg of the product **3** was obtained. MS(ES) m/z: $[MH]^+$ 927.68; IR (KBr) $cm^{-1}$ : 3416, 2973, 2934, 2880, 1660, 1599, 1546, 1498, 1471, 1376, 1339, 1272, 1238, 1163, 1134, 1097, 974, 843, 808, 761, 740, 703, 615.

**Compound 4**

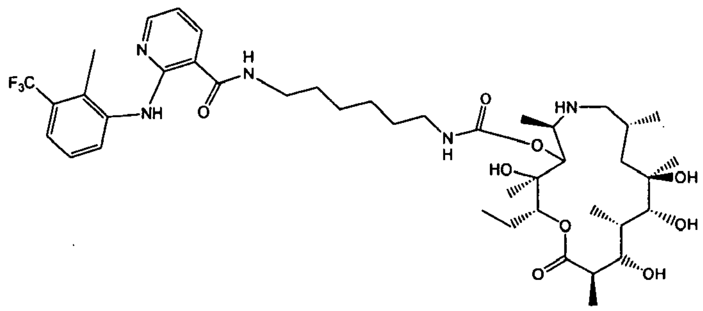

Compound **D21** (732 mg, 1.86 mmol) and compound **M7** (220 mg, 0.5 mmol) was dissolved in pyridine (7 mL). Then, pyridine hydrochloride (60 mg, 0.5 mmol) and 1,8-diazabycyclo [5.4.0]-undec-7-ene (1 mL) were added to the solution. The reaction mixture was stirred at room temperature for 6 days. The product was extracted by $CH_2Cl_2$ and washed with water and the organic layer was subsequently dried over $Na_2SO_4$ and the solvent evaporated under reduced pressure. After purification of the mixture on a silica gel column in the solvent system $CH_2Cl_2$:MeOH:$NH_4OH$=90:9:1.5 70 mg of the product **4** was obtained. MS(ES) m/z: $[MH]^+$ 840.43; IR (KBr) $cm^{-1}$ : 3339, 2971, 2935, 2878, 1645, 1593, 1524, 1462, 1380, 1320, 1254, 1186, 1168, 1122, 1088, 1023, 975, 927, 795, 772, 720, 665, 614

**Compound 5**

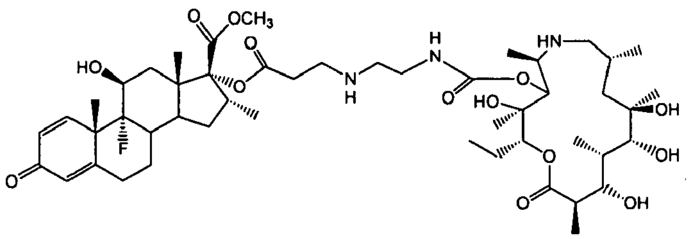

Compound **S22** (220 mg, 0.5 mmol) and compound **M7** (223 mg, 0.5 mmol) was dissolved in pyridine (5 mL).

Then, pyridine hydrochloride (58 mg, 0.5 mmol) and 1,8-diazabycyclo [5.4.0]-undec-7-ene (1 mL) was added to the solution. The reaction mixture was stirred at room temperature for 6 days. The product was extracted by $CH_2Cl_2$ and washed with water and the organic layer was subsequently dried over $Na_2SO_4$ and the solvent evaporated under reduced pressure. After purification of the mixture on a silica gel column in the solvent system $CH_2Cl_2$:MeOH:$NH_4OH$=90:9:1.5 80 mg of the product **5** was obtained. MS(ES) m/z: $[MH]^+$ 953.80

**Compound 6**

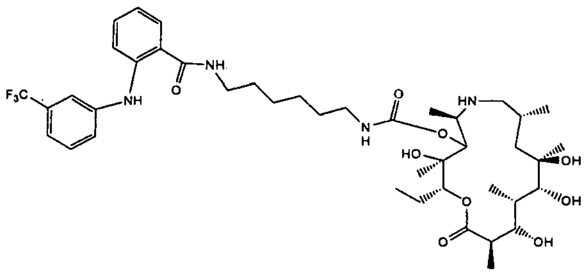

Compound **D19** (380 mg, 0.5 mmol) and compound **M7** (222 mg, 0.5 mmol) was dissolved in pyridine (5 mL). Then, pyridine hydrochloride (58 mg, 0.5 mmol) and 1,8-diazabycyclo [5.4.0]-undec-7-ene (1 mL) were added to the solution. The reaction mixture was stirred at room temperature for 6 days. The product was extracted by $CH_2Cl_2$ and washed with water and the organic layer was subsequently dried over $Na_2SO_4$ and the solvent evaporated under reduced pressure. After purification of the mixture on a silica gel column in the solvent system $CH_2Cl_2$:MeOH:$NH_4OH$=90:9:1.5 18 mg of the product **6** was obtained. MS(ES) m/z: $[MH]^+$ 825.47; IR (KBr) $cm^{-1}$ : 3369, 2970, 2935, 2878, 1632, 1595, 1524, 1452, 1426, 1377, 1336, 1283, 1248,1165, 1124, 1097, 1070, 975, 928, 793, 750, 699, 663.

**Compound 7**

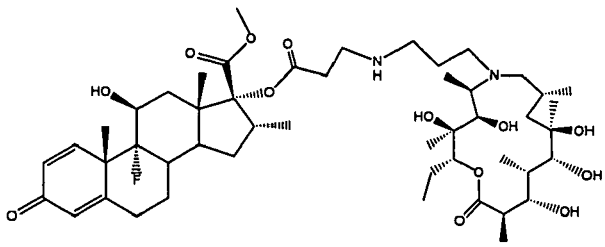

In a solution of compound **S10** ( 50 mg, 0.11 mmol) in methanol ( 10 mL) 107 mg ( 0.22 mmol) of the macrolide **M3** was added. Reaction mixture was stirred at 55°C for 24 hours. After evaporation of the solvent, mixture was purified on a silica gel column in the solvent system $CH_2Cl_2$:MeOH:$NH_4OH$ = 6:1:0.1. 23 mg of the compound 7 was obtained. MS (m/z): 923.47 $[MH]^+$ ; IR($cm^{-1}$)/KBr: 3449, 2938, 2878, 1738, 1665, 1621, 1562, 1544, 1525, 1521, 1460, 1377, 1353, 1266, 1242, 1178, 1099, 1050, 1015, 977, 957, 891, 810, 705, 673.

**Compound 8**

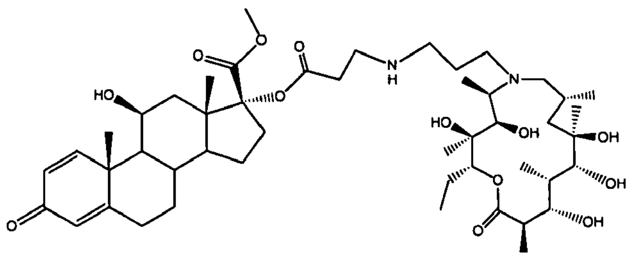

In a solution of compound **S19** ( 41 mg, 0.099 mmol) in methanol ( 6 mL) 47 mg ( 0.009 mmol) of the macrolide **M3** was added. Reaction mixture was stirred at 55°C for 24 hours. After evaporation of the solvent, mixture was purified on a silica gel column in the solvent system $CH_2Cl_2$:MeOH:$NH_4OH$ = 90:8:1. 19 mg of the compound **8** was obtained.. MS (m/z): 891.58 $[MH]^+$; IR($cm^{-1}$)/KBr: 3449, 2974, 2935, 2876, 1871,1846, 1735, 1658, 1618, 1545, 1509, 1459, 1375, 1352, 1283, 1177, 1127, 1087, 1036, 995, 958, 939, 888, 819, 708.

**Compound 9**

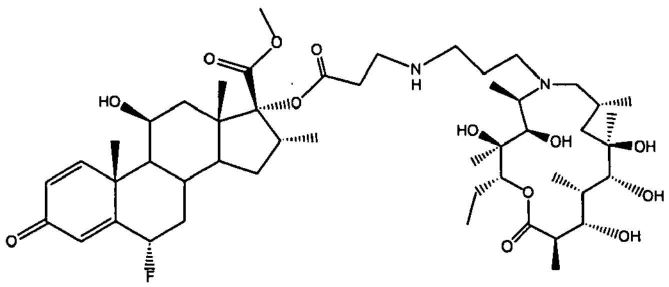

Reaction mixture of compound **S18** ( 77 mg, 0.17 mmol) in methanol ( 8 mL) and 165 mg ( 0.35 mmol) of the macrolide **M3** was stirred at 55°C for 24 hours. After evaporation of the solvent, mixture was purified on a silica gel column in the solvent system $CH_2Cl_2$:MeOH:$NH_4OH$ = 90:8:1 and 36 mg of the compound 9 was obtained. MS (*m*/*z*): 923.61 $[MH]^+$; IR($cm^{-1}$)/KBr: 3449, 2974, 2951, 2935, 2878, 1736, 1665, 1626, 1605, 1561, 1509, 1459, 1375, 1319, 1289, 1254, 1175, 1080, 1038, 958, 928, 900, 822, 757, 719, 666.

**Compound 10**

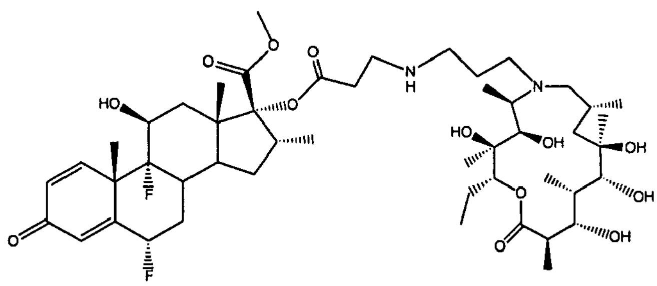

Reaction mixture of compound **S12** ( 37 mg, 0.08 mmol) in methanol:acetonitrile ( 2:5) and 76 mg ( 0.16 mmol) of the macrolide **M3** was stirred at 55°C for 24 hours. After evaporation of the solvent, mixture was purified on a silica gel column in the solvent system $CH_2Cl_2$:MeOH:$NH_4OH$ = 90:8:1 and 55 mg of the compound **10** was obtained. MS (m/z): 941.97 $[MH]^+$; IR($cm^{-1}$)/KBr: 3449, 2964, 2936, 2878, 1736, 1670, 1630, 1561, 1509, 1459, 1376, 1288, 1260, 1236, 1178, 1106, 1074, 1035, 994, 956, 940, 899, 849, 817, 755, 709, 669.

**Compound 11**

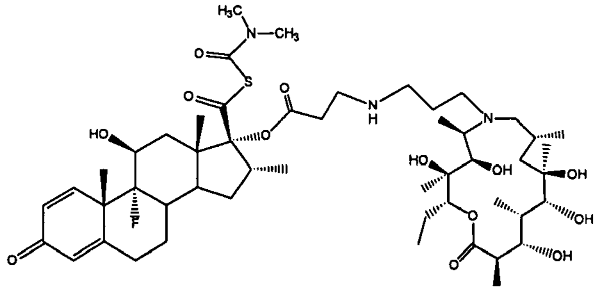

A solution of compound **1** (130 mg, 0.14 mmol) and dimethyltiocarbamoylchloride (35.32 mg, 0.286 mmol) in 2-butanone ( 10 mL) at room temperature was treated sequentially with triethylamine (0.044 ml, 0,31 mmol), sodium iodide (21 mg, 0,143 mmol), and water (0.013 mL) and stirred for 3 days. Reaction mixture was then treated sequentially with dimethyacetamide (0.52 mL) and water (3.23 mL); cooled to 0°C, stirred for 2 hours and extracted with EtOAc. Organic layer was dried over $Na_2SO_4$ and evaporated under reduced pressure. Mixture was purified on a silica gel column in the solvent system $CH_2Cl_2$:MeOH:$NH_4OH$ = 90:9:0.5. 32 mg of the compound **11** was obtained. MS (m/z): 996.48$[MH]^+$; IR($cm^{-1}$)/KBr: 3449, 2938, 2878, 1735, 1665, 1624, 1458, 1375, 1250, 1174, 1103, 1056, 1034, 1013, 981, 956, 929, 894, 781, 703, 672.

**Compound 12**

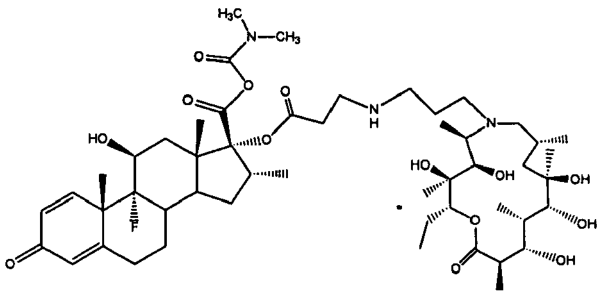

A solution of compound 1 (130 mg, 0.14 mmol) and dimethylcarbamoylchloride (35.32 mg, 0.286 mmol) in 2-butanone ( 10 mL) at room temperature was treated sequentially with triethylamine (0.044 ml, 0.31 mmol), sodium iodide (21 mg, 0.143 mmol), and water (0.013 mL) and stirred for 3 days. Reaction mixture was then treated sequentially with dimethyacetamide (0.52 mL) and water (3.23 mL); cooled to 0°C, stirred for 2 hours and extracted with EtOAc. Organic layer was dried over $Na_2SO_4$ and evaporated under reduced pressure. Mixture was purified on a silica gel column in the solvent system $CH_2Cl_2$:MeOH:$NH_4OH$ = 90:9:0.5. 30 mg of the compound **12** was obtained. MS (*m*/*z*): 980.5 $[MH]^+$

**Compound 13**

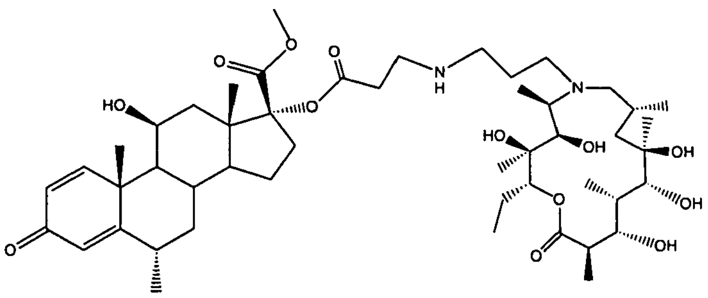

In a solution of compound **S24** ( 70 mg, 0,163 mmol) in 10 mL of methanol and 5 mL of acetonitrile 156 mg ( 0.327 mmol) of the macrolide **M3** was added. Reaction mixture was stirred at 55°C for 24 hours. After evaporation of the solvent, mixture was purified on a silica gel column in the solvent system $CH_2Cl_2$:MeOH:$NH_4OH$ = 90:9:1,5. 50 mg of the compound **13** was obtained. MS (*m*/*z*): 906.00 $[MH]^+$.

**Compound 14**

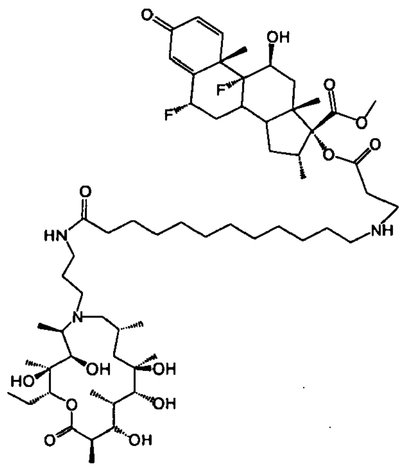

Compound **M10** (60 mg; 0.09 mmol) and compound **S12** (42 mg; 0.09 mmol) were dissolved in MeOH (10 ml) and $CH_3CN$ (5 ml) and the resulting reaction mixture was stirred at 50 °C over night. After concentrating solvents under vacuum the crude product was purified two times on silica gel column in the solvent system $CH_2Cl_2$:MeOH:$NH_4OH$ 90: 8:1 giving 31 mg of compound **14**.
LC/MS (area %): 94 %.
HPLC-MS: MS(ES) m/z: $[MH]^+$ 1138.80 (calcd. : 1138.73)
IR (KBr) $cm^{-1}$ : 3417, 2932, 2855, 1742, 1670, 1633, 1547, 1457, 1376, 1288, 1260, 1181, 1091, 1074, 1051, 994, 957, 940, 899, 849, 818, 711.

**Compound 15**

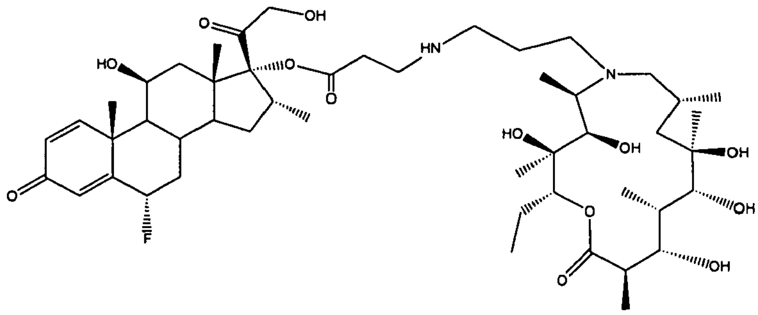

In 10 mL MeOH, 25 mg of compound **S26** was disolved followed by addition 34.68 mg of amine M3. The reaction mixture was stirred for 24 h at 55°C. After evaporation of the solvent, product was purified on on a silica gel column using solvent system $CHCl_3$:MeOH:$NH_4OH$ 6:1:0.1 giving 12 mg of compound **15**.
MS (*m/z*):923.31 $[MH]^+$ MS(teor.)=923.16
Purity (HPLC-MS): 93.50 %

**Compound 16**

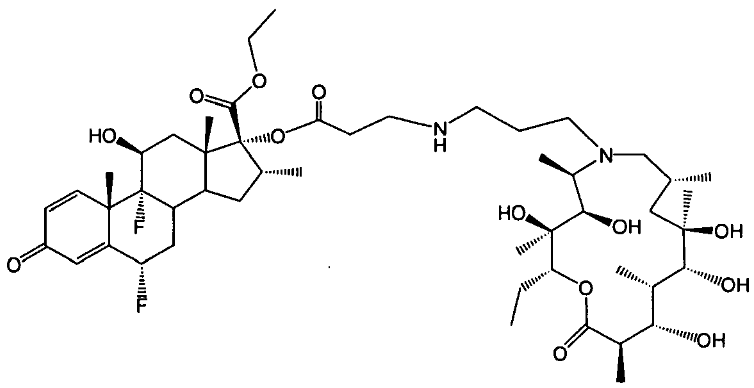

To a solution of 45 mg (0.094 mmol) of compound **S27** in 10 mL MeOH, 89.6 mg (0.188 mmol) of amine **M3** was added. The reaction mixture was stirred for 24 h at 55°C. After evaporation of the solvent, product was purified on on a silica gel column using solvent system $CHCl_3$:MeOH:$NH_4OH$ 6:1:0.1 giving15 mg of compound **16**.
MS (*m/z*):955.41 $[MH]^+$ MS(teor.)=955.17
Purity (HPLC-MS): 88.26 %

**Compound 17**

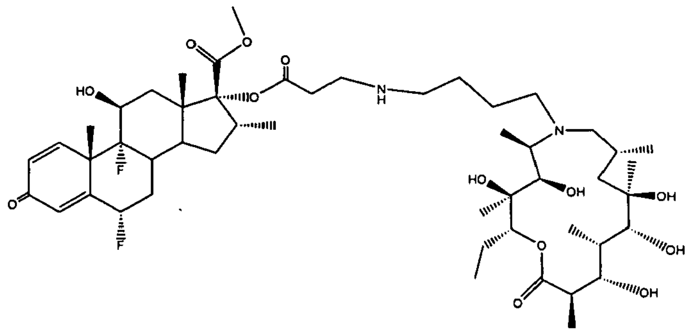

To a solution of 100 mg (0.215 mmol) of compound **S12** in 10 mL MeOH, 137.3 mg (0.280 mmol) amine **M11** (prepared as described in international publication WO2004/094449, example 16) was added. The reaction mixture was stirred for 24 h at 55°C. After evaporation of the solvent, product was purified on a silica gel column using solvent system $CHCl_3$:MeOH:$NH_4OH$ 6:1:0.1 giving139 mg of compound **17**.
MS (*m*/*z*):955.35 $[MH]^+$ MS(teor.)=954.56
Purity (HPLC-MS): 97.65 %
IR (KBr): 3450, 2939, 2879, 1738, 1671, 1628, 1562, 1545, 1525, 1459, 1377, 1288, 1259, 1236, 1179, 1072, 1053, 1036, 994, 957, 941, 900, 850, 817, 756, 709, 667.

**Compound 18**

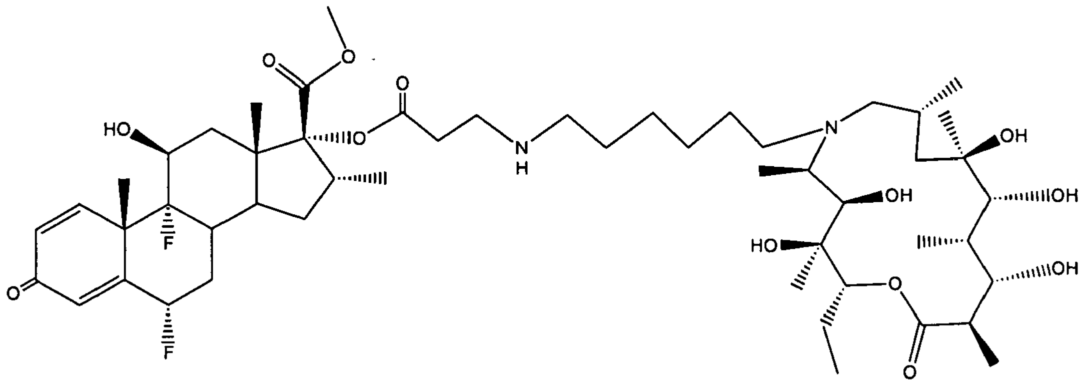

To a solution of 100 mg (0.215 mmol) of compound **S12** in 10 mL MeOH, 137.3 mg (0.280 mmol) of amine **M12** (prepared as described in international publication WO2004/094449, example 17) was added. The reaction mixture was stirred for 24 h at 55°C. After evaporation of the solvent, product was purified on on a silica gel column using solvent system $CHCl_3$:MeOH:$NH_4OH$ 6:1:0.1 giving 162 mg of compound **18**.
MS (*m*/*z*):983.37 $[MH]^+$ MS(teor.)=982.59
Purity (HPLC-MS): 98.68 %
IR (KBr): 3448, 2937, 2878, 1736, 1671, 1631, 1458, 1376, 1288, 1259, 1236, 1178, 1105, 1073, 1053, 1036, 994, 975, 957, 941, 899,849, 817, 755, 709, 664.

**Compound 19**

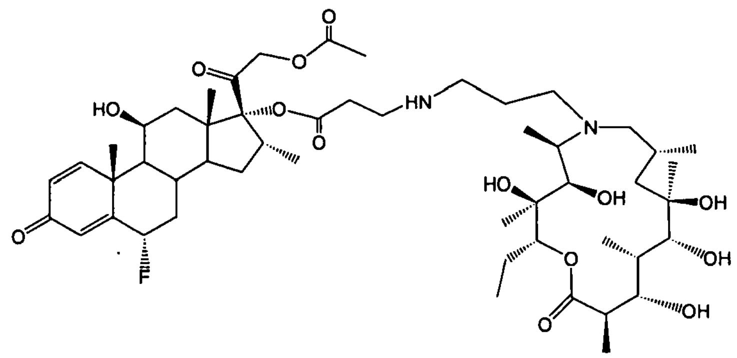

To a solution of 150 mg (0.307 mmol) of compound **S25** in 10 mL MeOH, 292.6 mg (0.615 mmol) of amine **M3** was added. The reaction mixture was stirred for 24 h at 55°C. After evaporation of the solvent, product was purified on on a silica gel column using solvent system $CHCl_3$:MeOH:$NH_4OH$ 6:1:0.1 giving 43 mg of compound **19**.
MS (*m*/*z*):983.37 $[MH]^+$ MS(teor.)=965.19
Purity (HPLC-MS): 98.15%

**Compound 20**

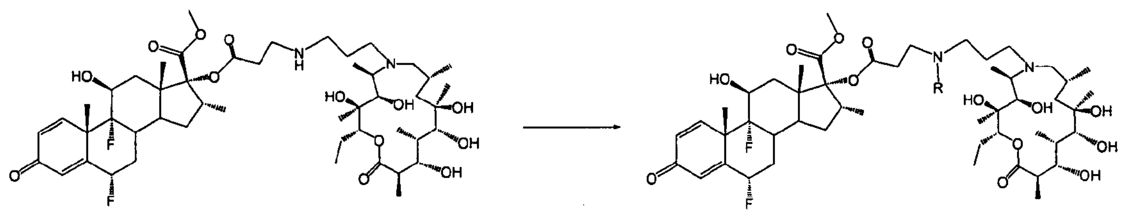

**Compound 10**

**20** R=$COCH_3$
**21** R=$CH_2CH_3$
**22** R=$CH(CH_3)_2$
**23** R=$CH_2COOCH_3$

Compound **10** (97 mg; 0,1 mmol) was dissolved in MeOH (10 ml), and cooled to 2°C. At this temperature, acetanhydride (2 µl; 0,2 mmol) was added drop wise to the reaction mixture.

After stirring for three hours at this temperature, the solvent was evaporated and a white, oily product was obtained which was subsequently purified on a silica gel column, eluent $CHCl_3$:MeOH:$NH_4OH$ 90:8:1. 88 mg of the compound **20** was obtained.
HPLC-MS: MS(ES) m/z: $[MH]^+$ 983,5
IR (KBr) $cm^{-1}$ : 3444, 2953, 2879, 1744, 1714, 1671, 1633, 1455, 1377, 1289, 1259, 1180, 1074, 1049, 1035, 994, 957, 940, 899, 849, 818, 709

**Compound 21**

Compound **10** (100 mg; 0,1 mmol) was dissolved in MeOH (10 ml). In the solution N,N-diisopropylethylamine (177 µl; 1 mmol) and iodoethane (52 µl; 0,65 mmol) were added. The reaction mixture was stirred at 50 °C for 24 hours. The solvent was evaporated under vacuum and crude product was purified on silica gel column in the solvent system $CH_2Cl_2$:MeOH:$NH_4OH$ 90:8:1. 22 mg of the compound **21** was obtained.
MS(ES) m/z: $[MH]^+$ 969,4

**Compound 22**

Compound **10** (200 mg; 0,2 mmol) was dissolved in $CH_3CN$ (10 ml). In the solution N,N-diisopropylethylamine (442 µl; 2,6 mmol) and 2-iodopropane (520 µl; 5,2 mmol) were added. The reaction mixture was stirred at 50 °C for 24 hours. The solvent was evaporated under vacuum and crude product was purified on silica gel column in the solvent

system $CH_2Cl_2$:MeOH:$NH_4OH$ 90:8:1. 70 mg of the compound **22** was obtained.
MS(ES) m/z: $[MH]^+$ 983,5

**Compound 23**

Compound **10** (200 mg; 0,2 mmol) was dissolved in THF (10 ml). In the solution methylbromoacetate (46 µl; 0,5 mmol) and potassium carbonate (55 mg; 0,4 mmol) were added. The reaction mixture was stirred at room temperature for 20 hours. The solvent was evaporated under vacuum and crude product was purified on silica gel column in the solvent system $CH_2Cl_2$:MeOH:$NH_4OH$ 90:8:1. 148 mg of the compound **23** was obtained.
MS(ES) m/z: $[MH]^+$ 1013,5

## Claims

1. A compound according to Formula I:

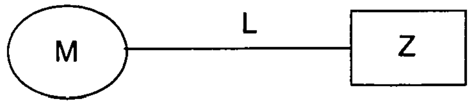


**I**

wherein
M represents the macrolide subunit of the substructure VIII:

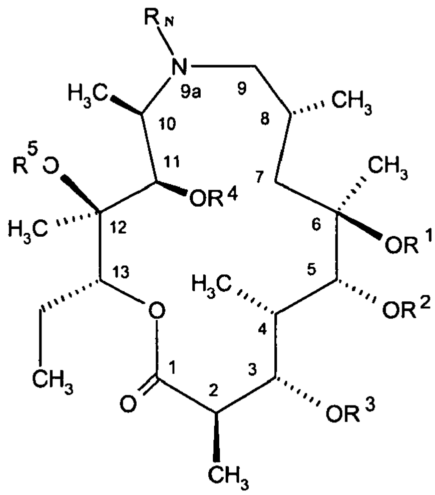

**VIII**

wherein
$R^1$, $R^2$, $R^3$ and $R^5$ are, chosen independently of each other, from the group consisting of hydrogen $C_1$-$C_4$ alkyl, alkanoyl, alkoxycarbonyl, arylmethoxycarbonyl, aroyl, arylalkyl, alkylsilyl, alkylsilylalkoxyalkyl or a covalent bond with $\mathbf{X^1}$ of chain **L** of formula **IX;**
$R^4$ is chosen from the group consisting of a covalent link with $\mathbf{X^1}$ of chain L of formula **IX,** hydrogen $C_1$-$C_4$ alkyl, alkanoyl, alkoxycarbonyl, arylmethoxycarbonyl, aroyl, arylalkyl, alkylsilyl and alkylsilylalkoxyalkyl; or $R^4$ is a group that can combine with $R^5$ to form a cyclic carbonate or carbamate, or with $>NR_N$ forms a cyclic carbamate;
$R_N$ represents hydrogen $C_1$-$C_4$ alkyl or a covalent bond with $X^1$ of the chain L of formula **IX;**
L is a linking group
**Z** represents a non steroidal anti-inflammatory subunit or a steroid subunit and pharmaceutically acceptable salts of the foregoing and pharmaceutically acceptable compositions containing the foregoing.

**2.** The compound according to claim 1 wherein L represents the chain of the substructure **IX** or **XIII:**

$$-X^1-(CH_2)_m-Q-(CH_2)_n-X^2- \qquad \mathbf{IX}$$

$$-X^1-(CH_2)_m-V-(CH_2)_p-Q-(CH_2)_n-X^2- \qquad \mathbf{XIII}$$

wherein
$X^1$ is selected from: $-CH_2$, $-CH_2-NH-$, $-C(O)-$, $-OC(O)-$, $=N-O-$, $-C(O)NH-$ or $-OC(O)NH-$;
$X^2$ is selected from: $-NH-$, $-CH_2-$;$-NHC(O)-$, $-C(=O)$ , $-OC(O)-$, $-C(=O)O-$, or$-C(O)NH-$;
Q is $-NH-$ or $-CH_2-$;
wherein each $-CH_2-$ or $-NH-$ group are optionally substituted by $C_1$-$C_7$-alkyl, $C_2$-$C_7$-alkenyl, $C_2$-$C_7$-alkynyl, $C(O)R^x$, $C(O)OR^x$, $C(O)NHR^x$, $CH_2C(O)OR^x$,
wherein $R^x$ may be $C_1$-$C_7$-alkyl, aryl or heteroaryl;
V is $-NH-$ or $-NH-C(O)-$;
the symbols m, n and p are independently a zero or whole number from 0 to 12 with the proviso that if Q=NH; n cannot be zero.

**3.** The compound according to claims 1 or 2 wherein Z is a steroid of the substructure X:

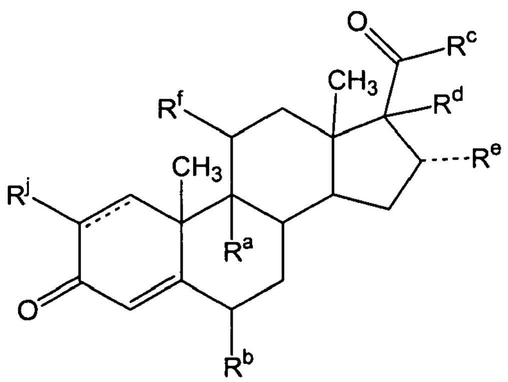

**X**

wherein
$R^a$, $R^b$, are chosen independently of each other from the group conisting of hydrogen, methyl and halogen;
$R^f$ is chosen from the group consisting of hydrogen, hydroxyl group, halogen or forms (C=O) a carbonyl group with the carbon atom to which it is linked;
$R^c$ is hydroxy, $C_1$-$C_4$alkyl; $C_1$-$C_4$ alkoxy; $C_1$-$C_4$alkylhydroxy; NH-$C_1$-$C_4$alkyl; $CH_2OC(O)C_1$-$C_4$alkyl or $XC(O)N(R^1R^2)$ wherein X is S or O, $R^1$ and $R^2$ are independently $C_1$-$C_6$ alkyl or $R^1$ and $R^2$ together are $C_1$-$C_6$ alkylene; or $R^c$ is $SCH_2$ or $CH_2Y$ wherein Y is halogen; or $R^c$ is a covalent bond with $X^2$ of the chain **L** provided that chain L is linked to R4 of macrolide subunit of formula VIII;
$R^d$ is the covalent link with $X^2$ of chain L, hydrogen, hydroxy, methyl, $C_1$-$C_4$ alkoxy or together with $R^e$ and the carbon atoms to which they are attached form 1,3-dioxolane ring which can be additionally alkyl or alkenyl mono or di-substituted;
$R^e$ is hydrogen, hydroxy, methyl, and $C_1$-$C_4$ alkoxy; and
$R^j$ is chosen from the group consisting of hydrogen and chlorine.

**4.** A compound according to claim 1 wherein said **Z** is a nonsteroidal anti-inflammatory (NSAID) subunit.

**5.** A compound according to claim 4 wherein the NSAID subunit is selected from the group consisting of subunits of aceclofenac, acemetacin, acetaminophen, acetaminosalol, acetyl-salicylic acid, acetyl-salicylic-2-amino-4-picoline-acid, 5-aminoacetylsalicylic acid, alclofenac, aminoprofen, amfenac, ampyrone, ampiroxicam, anileridine, bendazac, benoxaprofen, bermoprofen, α-bisabolol, bromfenac, 5-bromosalicylic acid acetate, bromosaligenin, bucloxic acid, butibufen, carprofen, celecoxib, chromoglycate, cinmetacin, clindanac, clopirac, sodium diclofenac, diflunisal, ditazol,

droxicam, enfenamic acid, etodolac, etofenamate, felbinac, fenbufen, fenclozic acid, fendosal, fenoprofen, fentiazac, fepradinol, flufenac, flufenamic acid, flunixin, flunoxaprofen, flurbiprofen, glutametacin, glycol salicylate, ibufenac, ibuprofen, ibuproxam, indomethacin, indoprofen, isofezolac, isoxepac, isoxicam, ketoprofen, ketorolac, lornoxicam, loxoprofen, meclofenamic acid, mefenamic acid, meloxicam, mesalamine, metiazinic acid, mofezolac, montelukast, mycophenolic acid, nabumetone, naproxen, niflumic acid, nimesulide, olsalazine, oxaceprol, oxaprozin, oxyphenbutazone, paracetamol, parsalmide, perisoxal, phenyl-acethyl-salicylate, phenylbutazone, phenylsalicylate, pyrazolac, piroxicam, pirprofen, pranoprofen, protizinic acid, reserveratol, salacetamide, salicylamide, salicylamide-O-acetyl acid, salicylsulphuric acid, salicin, salicylamide, salsalate, sulindac, suprofen, suxibutazone, tamoxifen, tenoxicam, theophylline, tiaprofenic acid, tiaramide, ticlopridine, tinoridine, tolfenamic acid, tolmetin, tropesin, xenbucin, ximoprofen, zaltoprofen, zomepirac, tomoxiprol, zafirlukast and cyclosporine.

**6.** A compound according to claim 5 wherein the NSAID subunit is not acetyl salicylic acid or mycophenolic acid.

**7.** A compound according to claim 5 wherein the NSAID subunit is chosen from the group consisting of flufenamic acid, flunixin and celecoxib.

**8.** The compound according to claims 1-7 wherein $R^1$, $R^2$, $R^3$, $R^4$ and $R^5$ are each independently chosen from the group consisting of hydrogen and $C_1$-$C_4$ alkyl and $R_N$ represents a covalent bond with $\mathbf{X^1}$ of the chain **L**.

**9.** The compound according to claim 8 wherein $R^1$, $R^2$, $R^3$, $R^4$ and $R^5$ are chosen independently from the group consisting of hydrogen and methyl.

**10.** The compound according to claims 1-7 wherein $R^4$ represents a covalent bond with $X^1$ of the chain L and $R^1$, $R^2$, $R^3$, $R^4$ and $R^5$ are each independently chosen from the group consisting of hydrogen and $C_1$-$C_4$ alkyl.

**11.** The compound according to claim 10 wherein $R^1$, $R^2$, $R^3$, $R^4$ and $R^5$ are chosen independently from the group consisting of hydrogen and methyl.

**12.** The compound according to claim 1 wherein $R_N$ represents a covalent bond with $X^1$ of the chain L.

**13.** The compound according to claim 1 wherein $X^1$ is $-CH_2-$ and $X_2$ is -C(O)O-.

**14.** The compound according to claim 1 wherein $X^1$ is -C(O)NH- and $X_2$ is -NH-.

**15.** The compound according to claim 1 wherein $X^1$ is -C(O)NH- and $X_2$ is -NHC(O)-.

**16.** The compound according to claim 3 wherein $X^1$ is - C(O)NH- and $X^2$ is -NH-.

**17.** The compound according to claim 3 wherein $X^1$ is $-CH_2-$ and $X^2$ is -C(O)O-.

**18.** The compound according to claim 3 wherein $R^d$ is covalent link with $X^2$ of the chain **L**.

**19.** The compound according to claim 3 wherein substructure **X** is chosen from the group consisting of

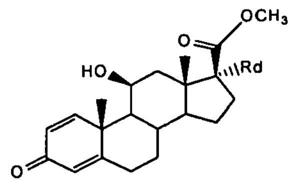

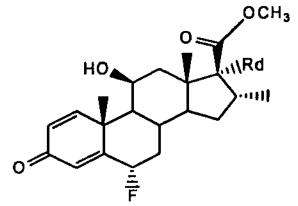

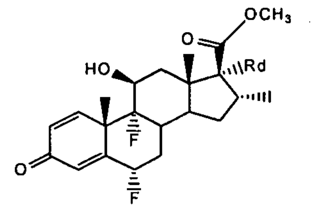

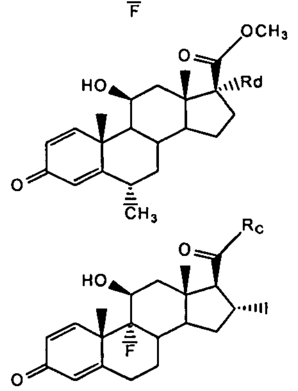

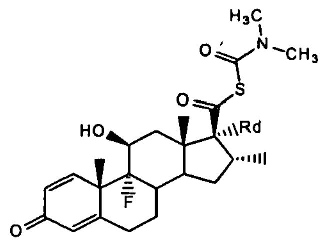

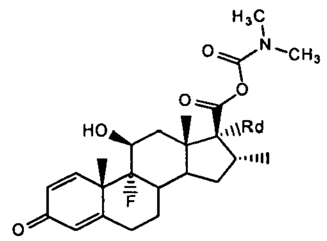

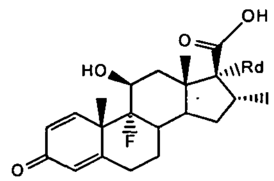

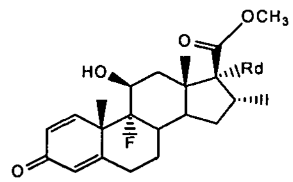

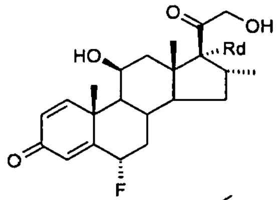

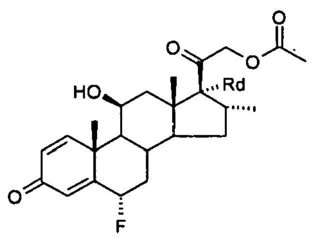

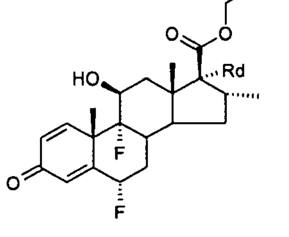

**20.** The compound according to claim 8 wherein substructure X is

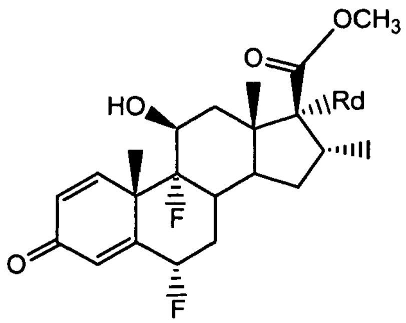

**21.** A compound according to claim 1 having the structure

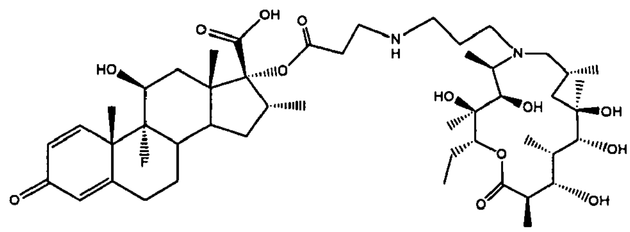

and pharmaceutically acceptable salts and solvates thereof.

**22.** A compound according to claim 1 having the structure

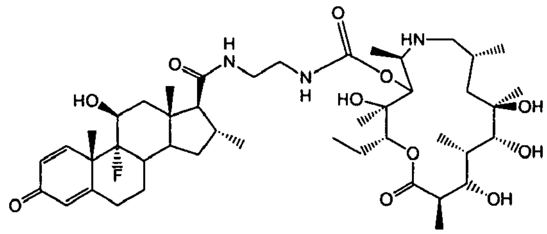

and pharmaceutically acceptable salts and solvates thereof.

**23.** A compound according to claim 1 having the structure

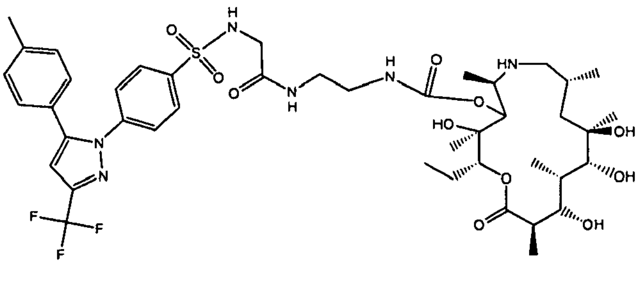

and pharmaceutically acceptable salts and solvates thereof.

**24.** A compound according to claim 1 having the structure

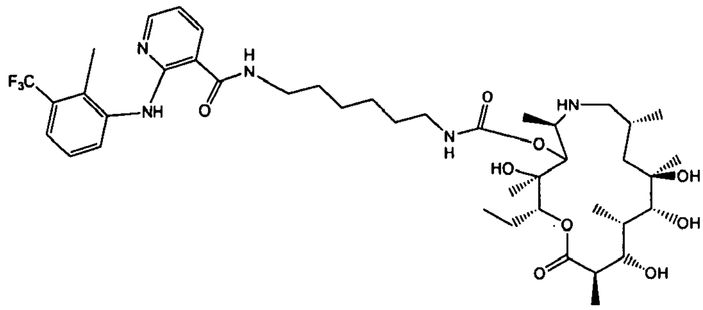

and pharmaceutically acceptable salts and solvates thereof.

**25.** A compound according to claim 1 having the structure

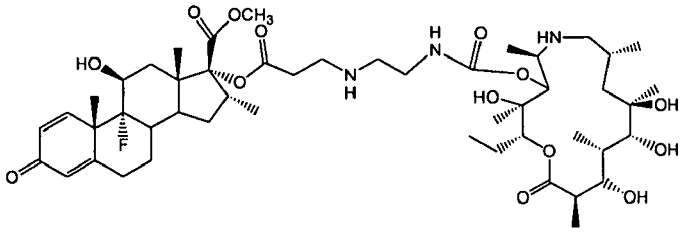

and pharmaceutically acceptable salts and solvates thereof.

**26.** A compound according to claim 1 having the structure

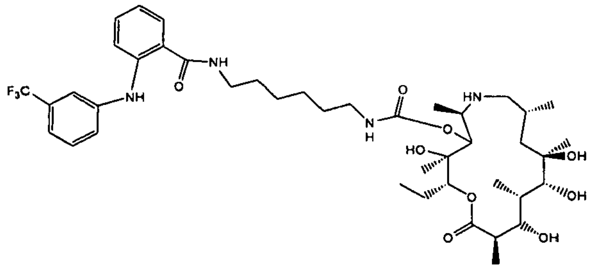

and pharmaceutically acceptable salts and solvates thereof.

**27.** A compound according to claim 1 having the structure

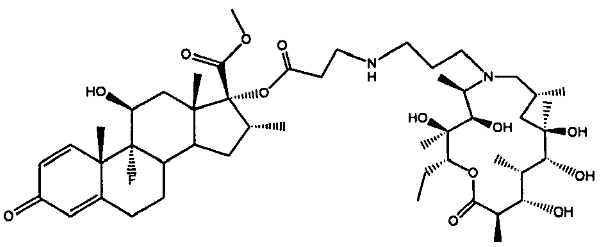

and pharmaceutically acceptable salts and solvates thereof.

**28.** A compound according to claim 1 having the structure

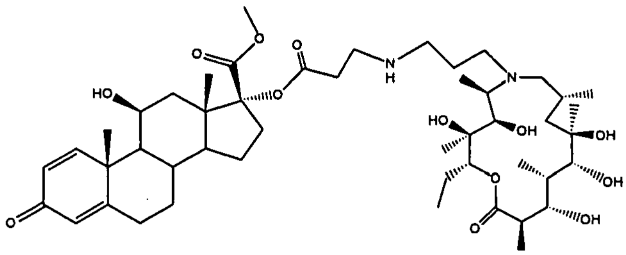

and pharmaceutically acceptable salts and solvates thereof.

**29.** A compound according to claim 1 having the structure

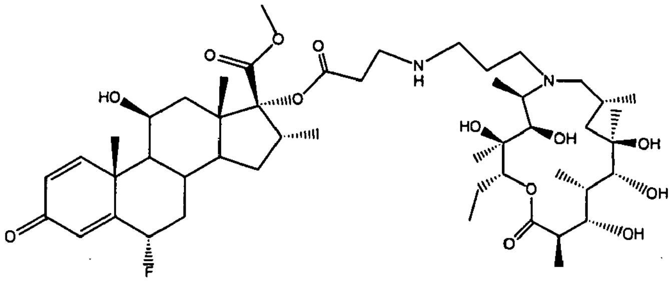

and pharmaceutically acceptable salts and solvates thereof.

**30.** A compound according to claim 1 having the structure

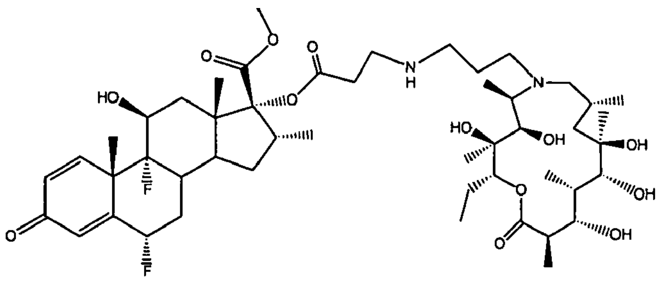

and pharmaceutically acceptable salts and solvates thereof.

**31.** A compound according to claim 1 having the structure

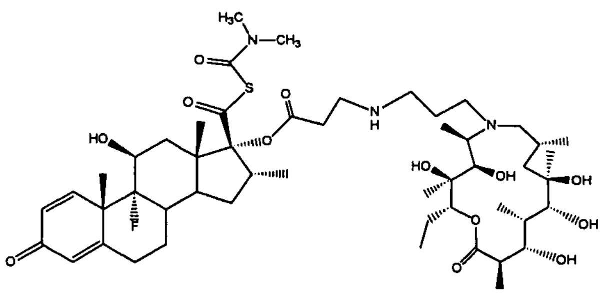

and pharmaceutically acceptable salts and solvates thereof.

**32.** A compound according to claim I having the structure

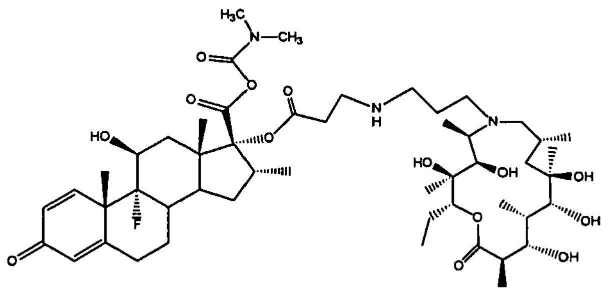

and pharmaceutically acceptable salts and solvates thereof.

**33.** A compound according to claim 1 having the structure

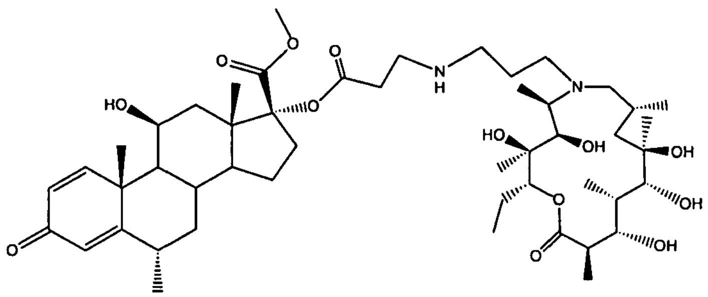

and pharmaceutically acceptable salts and solvates thereof.

**34.** A compound according to claim 1 having the structure

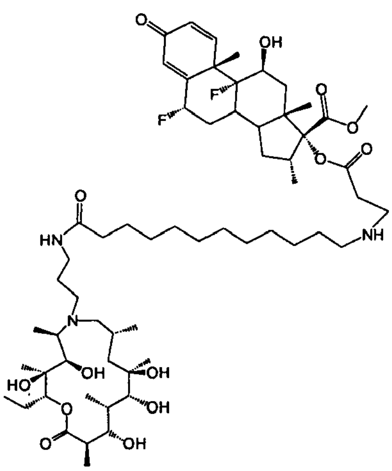

and pharmaceutically acceptable salts and solvates thereof.

**35.** A compound according to claim 1 having the structure

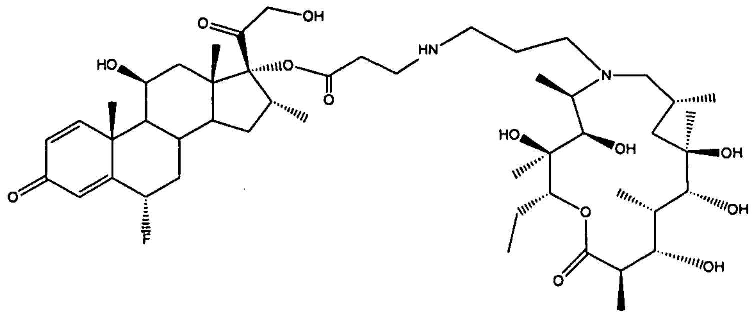

and pharmaceutically acceptable salts and solvates thereof.

**36.** A compound according to claim 1 having the structure

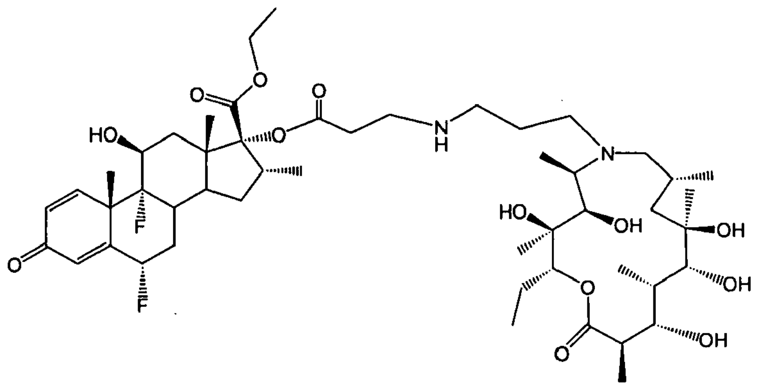

and pharmaceutically acceptable salts and solvates thereof.

**37.** A compound according to claim 1 having the structure

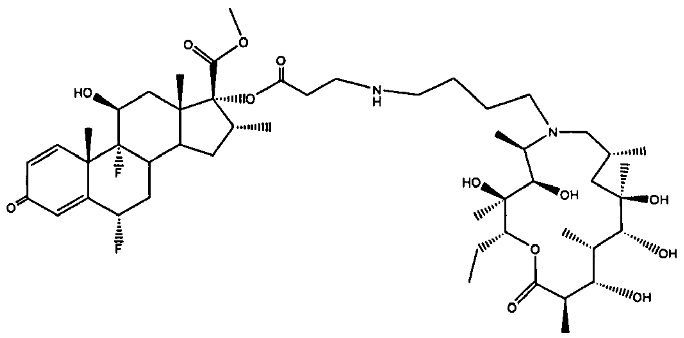

and pharmaceutically acceptable salts and solvates thereof.

**38.** A compound according to claim 1 having the structure

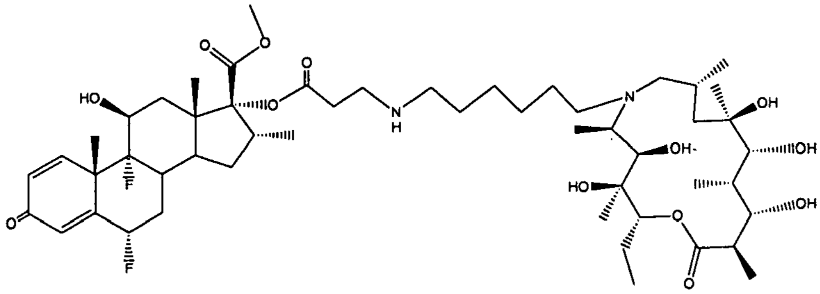

and pharmaceutically acceptable salts and solvates thereof.

**39.** A compound according to claim 1 having the structure

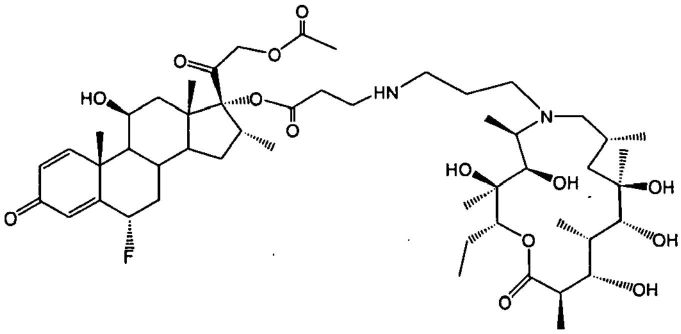

and pharmaceutically acceptable salts and solvates thereof.

**40.** A compound according to claim 1 having the structure

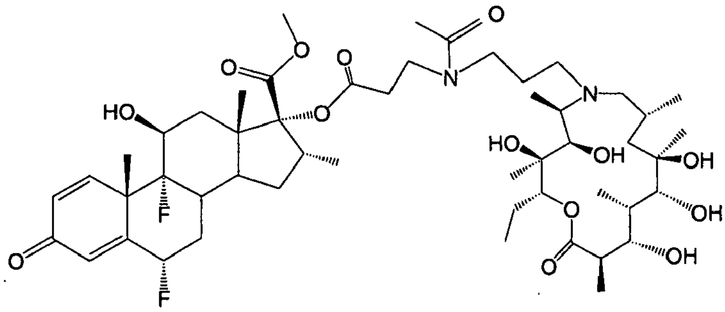

and pharmaceutically acceptable salts and solvates thereof.

**41.** A compound according to claim 1 having the structure

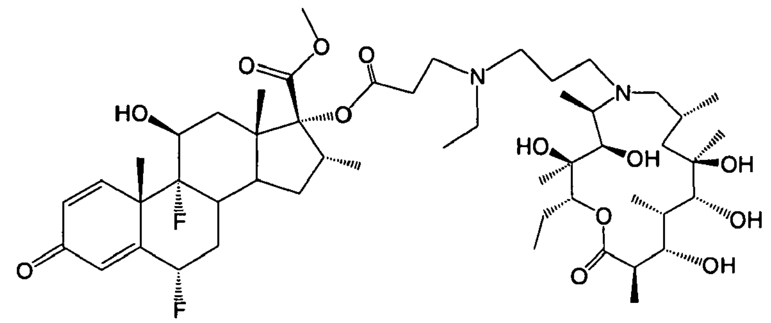

and pharmaceutically acceptable salts and solvates thereof.

**42.** A compound according to claim 1 having the structure

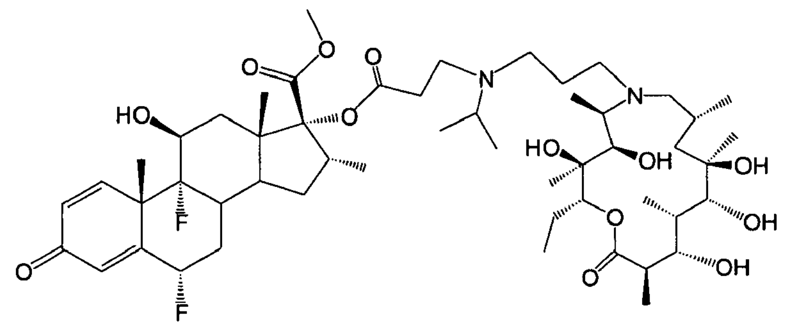

and pharmaceutically acceptable salts and solvates thereof.

**43.** A compound according to claim 1 having the structure

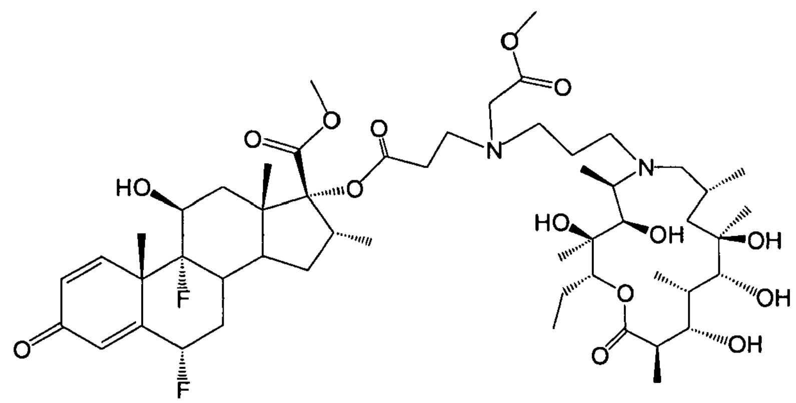

and pharmaceutically acceptable salts and solvates thereof.

**44.** A pharmaceutical composition comprising a compound according to claims 1 to 43 and pharmaceutically acceptable salt or solvate thereof as well as pharmaceutically acceptable diluent or carrier.

**45.** A method of treatment of inflammatory diseases, disorders and conditions **characterized by** or associated with an undesirable inflammatory immune response, and all diseases and conditions induced by or associated with an excessive secretion of TNF-α and IL-1 which comprises administering to a subject a therapeutically effective amount of a compound according to claims 1 to 43.

**46.** A method of treating inflammatory conditions and immune or anaphylactic disorders associated with infiltration of leukocytes into inflamed tissue in a subject in need thereof which comprises administering to said subject a therapeutically effective amount of the compound of claim 1 to 43.

**47.** The method according to claim 45, wherein inflammatory conditions and immune disorders are selected from the group consisting of asthma, adult respiratory distress syndrome, chronic obstructive pulmonary disease, inflammatory bowel conditions, Crohn's disease, bronchitis, and cystic fibrosis.

**48.** The method according to claim 45, wherein said inflammatory conditions and immune disorders are selected from the group consisting of inflammatory conditions or immune disorders of the lungs, joints, eyes, bowel, skin, and heart.

**49.** A method according to claim 45, wherein said inflammatory conditions and immune disorders are selected from the group consisting of asthma, adult respiratory distress syndrome, bronchitis, cystic fibrosis, rheumatoid arthritis, rheumatoid spondylitis, osteoarthritis, gouty arthritis, uveitis, conjunctivitis, inflammatory bowel conditions, Crohn's disease, ulcerative colitis, distal proctitis, psoriasis, eczema, dermatitis, coronary infarct damage, chronic inflammation, endotoxin shock, and smooth muscle proliferation disorders.

**50.** A method of treatment of inflammatory diseases, disorders and conditions **characterized by** or associated by excessive unregulated production of cytokines or inflammatory mediators which comprises administering to a subject a therapeutically effective amount of a compound according to claims 1 to 43.

Europäisches Patentamt
European Patent Office
Office européen des brevets

# EUROPEAN SEARCH REPORT

Application Number
EP 09 07 5144

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 03/070174 A (SYMPORE GMBH; BURNET, MICHAEL; GUSE, JAN-HINRICH; GUTKE, HANS-JURGEN;) 28 August 2003 (2003-08-28)<br>* page 87; compound 59 *<br>* page 98; compound 71 *<br>----- | 1-15 | INV.<br>C07J43/00<br>C07D413/12<br>C07D267/00<br>A61K31/58<br>A61P29/00 |
| X,D | WO 02/055531 A (PLIVA D.D; MERCEP, MLANDEN; MESIC, MILAN; TOMASKOVIC, LINDA; KOMAC, MA) 18 July 2002 (2002-07-18)<br>* page 43; compounds 25-27 *<br>* pages 39-42; compounds 1-20 *<br>----- | 1-15 | |
| X,P | WO 2004/094449 A (PLIVA PHARMACEUTICAL INDUSTRY, INC; MERCEP, MLADEN; MESIC, MILAN; TOMA) 4 November 2004 (2004-11-04)<br>* the whole document *<br>----- | 1-15 | |
| E | WO 2006/018698 A2 (PLIVA ISTRAZIVACKI INST D O O [HR]; MERCEP MLADEN [HR]; MESIC MILAN [H) 23 February 2006 (2006-02-23)<br>* page 19; compound 1 *<br>* page 25; compound 12 *<br>----- | 1-9,12 | TECHNICAL FIELDS SEARCHED (IPC)<br>C07J<br>C07D<br>A61K<br>A61P |

The present search report has been drawn up for all claims

1

| Place of search | Date of completion of the search | Examiner |
|---|---|---|
| The Hague | 21 April 2010 | Duval, Eric |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................
& : member of the same patent family, corresponding document

EPO FORM 1503 03.82 (P04C01)

Europäisches Patentamt
European Patent Office
Office européen des brevets

Application Number
EP 09 07 5144

## CLAIMS INCURRING FEES

The present European patent application comprised at the time of filing claims for which payment was due.

- [ ] Only part of the claims have been paid within the prescribed time limit. The present European search report has been drawn up for those claims for which no payment was due and for those claims for which claims fees have been paid, namely claim(s):

- [X] No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

- [ ] All further search fees have been paid within the fixed time limit. The present European search report has been drawn up for all claims.

- [ ] As all searchable claims could be searched without effort justifying an additional fee, the Search Division did not invite payment of any additional fee.

- [ ] Only part of the further search fees have been paid within the fixed time limit. The present European search report has been drawn up for those parts of the European patent application which relate to the inventions in respect of which search fees have been paid, namely claims:

- [ ] None of the further search fees have been paid within the fixed time limit. The present European search report has been drawn up for those parts of the European patent application which relate to the invention first mentioned in the claims, namely claims:

- [ ] The present supplementary European search report has been drawn up for those parts of the European patent application which relate to the invention first mentioned in the claims (Rule 164 (1) EPC).

## ANNEX TO THE EUROPEAN SEARCH REPORT ON EUROPEAN PATENT APPLICATION NO. EP 09 07 5144

This annex lists the patent family members relating to the patent documents cited in the above-mentioned European search report.
The members are as contained in the European Patent Office EDP file on
The European Patent Office is in no way liable for these particulars which are merely given for the purpose of information.

21-04-2010

| Patent document cited in search report | Publication date | Patent family member(s) | Publication date |
|---|---|---|---|
| WO 03070174 A | 28-08-2003 | AU 2003219770 A1 | 09-09-2003 |
| | | CA 2476423 A1 | 28-08-2003 |
| | | EP 1483277 A2 | 08-12-2004 |
| | | HR 20040850 A2 | 31-08-2005 |
| | | NZ 535354 A | 31-01-2008 |
| WO 02055531 A | 18-07-2002 | AT 462713 T | 15-04-2010 |
| | | BG 108073 A | 31-05-2005 |
| | | BR 0206347 A | 10-02-2004 |
| | | CA 2434009 A1 | 18-07-2002 |
| | | CN 1491230 A | 21-04-2004 |
| | | CZ 20031825 A3 | 12-11-2003 |
| | | EE 200300293 A | 15-10-2003 |
| | | EP 1351973 A1 | 15-10-2003 |
| | | HK 1065320 A1 | 04-04-2007 |
| | | HR 20010018 A2 | 31-12-2002 |
| | | JP 2004518670 T | 24-06-2004 |
| | | PL 363478 A1 | 15-11-2004 |
| | | SK 9792003 A3 | 08-01-2004 |
| | | UA 77166 C2 | 15-10-2003 |
| | | US 2009105163 A1 | 23-04-2009 |
| | | US 2004198677 A1 | 07-10-2004 |
| | | YU 55203 A | 03-03-2006 |
| WO 2004094449 A | 04-11-2004 | AU 2004232546 A1 | 04-11-2004 |
| | | BR PI0410490 A | 13-06-2006 |
| | | CA 2523081 A1 | 04-11-2004 |
| | | CL 8722004 A1 | 18-03-2005 |
| | | CN 1777619 A | 24-05-2006 |
| | | DK 1633771 T3 | 16-11-2009 |
| | | EP 1633771 A1 | 15-03-2006 |
| | | EP 2070940 A1 | 17-06-2009 |
| | | ES 2330109 T3 | 04-12-2009 |
| | | HK 1086842 A1 | 30-04-2009 |
| | | HR 20030324 A2 | 28-02-2005 |
| | | HR 20090551 T1 | 31-01-2010 |
| | | IS 8141 A | 22-11-2005 |
| | | JP 2006524221 T | 26-10-2006 |
| | | KR 20060024770 A | 17-03-2006 |
| | | MX PA05011359 A | 12-05-2006 |
| | | PT 1633771 E | 20-10-2009 |
| | | RU 2355699 C2 | 20-05-2009 |
| | | SI 1633771 T1 | 31-12-2009 |
| | | US 2005080003 A1 | 14-04-2005 |
| | | ZA 200508388 A | 26-07-2006 |

EPO FORM P0459

For more details about this annex : see Official Journal of the European Patent Office, No. 12/82

# ANNEX TO THE EUROPEAN SEARCH REPORT ON EUROPEAN PATENT APPLICATION NO. EP 09 07 5144

This annex lists the patent family members relating to the patent documents cited in the above-mentioned European search report.
The members are as contained in the European Patent Office EDP file on
The European Patent Office is in no way liable for these particulars which are merely given for the purpose of information.

21-04-2010

| Patent document cited in search report | Publication date | Patent family member(s) | Publication date |
|---|---|---|---|
| WO 2006018698 A2 | 23-02-2006 | AU 2005273592 A1 | 23-02-2006 |
| | | BR PI0514254 A | 03-06-2008 |
| | | CA 2576291 A1 | 23-02-2006 |
| | | EP 1778292 A2 | 02-05-2007 |
| | | JP 2008509899 T | 03-04-2008 |
| | | KR 20070046917 A | 03-05-2007 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Patent documents cited in the description

- WO 9413690 A
- WO 9414834 A
- WO 9213872 A
- WO 9213873 A
- WO 0042055 A
- EP 0283055 A
- EP 0775489 A
- EP 771564 A
- WO 02055531 A1
- WO 0205531 A
- US 20040014685 A
- WO 04005310 A2
- US 20040077612 A
- US 20040097434 A
- WO 04005309 A
- US 20050080003 A
- US 20040087517 A
- WO 2003070174 A
- US 6297260 B
- WO 0187890 A
- HR 20010018
- WO 02055531 A
- WO 04005310 A
- US 6402733 B
- US 6273086 B
- US 6228346 B
- WO 2004094449 A

### Non-patent literature cited in the description

- **Gladue, R. P. et al.** *Antimicrob. Agents Chemother.,* 1989, vol. 33, 277-282
- **Olsen, K. M. et al.** *Antimicrob. Agents Chemother.,* 1996, vol. 40, 2582-2585
- *J. Antimicrob. Chemother.,* 1998, vol. 41, 37-46
- *J. Antimicrob. Chemother.,* 1992, vol. 30, 339-348
- *J. Immunol.,* 1997, vol. 159, 3395-4005
- *Am. J. Respir. Crit. Care. Med.,* 1997, vol. 156, 266-271
- **Berge S. M. et al.** Pharmaceutical Salts. *J. of Pharma. Sci.,* 1977, vol. 66, 1
- **E.W. Martin.** Remington's Pharmaceutical Sciences
- **Huang C.M. et al.** *Chem.&Biol.,* 2000, vol. 7, 453-461
- **Hess S. et al.** *Bioorg.&Med. Chem.,* 2001, vol. 9, 1279-1291
- **Pandori M. W. et al.** *Chem.&Biol.,* 2002, vol. 9, 567-573
- **T.W. Greene ; P.G.M Wuts.** Protective Groups in Organic Synthesis. John Wiley & Son, Inc, 1991
- **P.J. Kocienski.** Protecting Groups. Georg Thieme Verlag, 1994
- **Newman et al.** *Thorax,* 1985, vol. 40, 61-676
- **Berenberg, M.** *J. Asthma USA,* 1985, vol. 22, 87-92